# EUROPEAN PATENT SPECIFICATION

(11) **EP 2 139 557 B1**
(45) Date of publication and mention of the grant of the patent: **13.05.2015**
(21) Application number: 08734777.9
(22) Date of filing: 26.03.2008
(51) Int. Cl.: A61N 1/40, A61N 5/02

(54) **ELECTRONIC SYSTEM FOR INFLUENCING CELLULAR FUNCTIONS IN A WARM-BLOODED MAMMALIAN SUBJECT**
ELEKTRONISCHES SYSTEM ZUR BEEINFLUSSUNG VON ZELLULÄREN FUNKTIONEN IN EINEM WARMBLÜTIGEN SÄUGETIER
SYSTÈME ÉLECTRONIQUE PERMETTANT D'INFLUENCER LES FONCTIONS CELLULAIRES CHEZ UN MAMMIFÈRE À SANG CHAUD

(30) Priority: 27.03.2007 EP 07006320
(43) Date of publication of application: 06.01.2010
(73) Proprietor: Therabionic LLC, Winston-Salem, NC 27106-3567 (US)
(72) Inventor: Pasche, Boris, Chicago, IL 60611 (US); Barbault, Alexandre, 68000 Colmar (FR)
(74) Representative: Zimmermann & Partner Patentanwälte mbB
(86) International application number: PCT/EP2008/002379
(87) International publication number: WO 2008/116640

(56) References cited:
- EP-A2- 0 592 851
- EP-A2- 1 070 518
- US-A- 5 690 692
- US-A- 6 167 304
- US-A1- 2005 090 732
- CORDESSES L: "Direct digital synthesis: a tool for periodic wave generation (part 1)" IEEE SIGNAL PROCESSING MAGAZINE, IEEE SERVICE CENTER, PISCATAWAY, NJ, US, vol. 21, no. 4, July 2004 (2004-07), pages 50-54, XP011115196 ISSN: 1053-5888

## Description

### FIELD OF THE INVENTION

This invention relates to an electronic system for influencing cellular functions in a warm-blooded mammalian subject. More particularly, the invention concerns research findings related to how earlier electronic systems may be modified and programmed to achieve both improved and additional therapeutic effects.

### BACKGROUND OF THE INVENTION

Reference is made to European Patent EP 0 592 851 B1 and corresponding Patents and Patent Applications and to the various publications referred to therein. Since the time of the priority Application filed in the USA on 25 September 1992 (US Serial No 951563 now USP 5,441,528), a number of further publications related to effects of very low energy electromagnetic fields on patients suffering from insomnia and/or anxiety disorders have taken place:
Koziol JA, Erman M, Pasche B, Hajdukovic R, Mitler MM (1993) Assessing a changepoint in a sequence of repeated measurements with application to a low-energy emission therapy sleep study. J Applied Statistics 20: 393-400
Amato D, Pasche B (1993) An evaluation of the safety of low energy emission therapy. Compr Ther 19: 242-247
Higgs L, Reite M, Barbault A, Lebet JP, Rossel C, Amato D, Dafni U, Pasche B (1994) Subjective and Objective Relaxation Effects of Low Energy Emission Therapy. Stress Medicine 10: 5-13
Reite M, Higgs L, Lebet JP, Barbault A, Rossel C, Kuster N, Dafni U, Amato D, Pasche B (1994) Sleep Inducing Effect of Low Energy Emission Therapy. Bio-electromagnetics 15: 67-75
Lebet JP, Barbault A, Rossel C, Tomic Z, Reite M, Higgs L, Dafni U, Amato D, Pasche B (1996) Electroencephalographic changes following low energy emission therapy. Ann Biomed Eng 24: 424-429
Pasche B, Erman M, Hayduk R, Mitler M, Reite M, Higgs L, Dafni U, Amato D, Rossel C, Kuster N, Barbault A, Lebet J-P (1996) Effects of Low Energy Emission Therapy in chronic psychophysiological insomnia. Sleep 19: 327-336
Kelly TL, Kripke DF, Hayduk R, Ryman D, Pasche B, Barbault A (1997) Bright light and LEET effects on circadian rhythms, sleep and cognitive performance. Stress Medicine 13: 251-258
Pasche B, Barbault A (2003) Low-Energy Emission Therapy: Current Status and Future Directions. In Bioelectromagnetic Medicine, Rosch PJ, Markov MS (eds) pp 321-327. Marcel Dekker, Inc.: New York, New York.

The above publications are related to an earlier device, system and use thereof described in said EP 0 592 851 B1. The improved electronic system and programmed control thereof in accordance with the present invention, however, has been determined to find therapeutic application not only for influencing cellular functions (or malfunctions) leading to central nervous system (CNS) disorders, but more particularly for influencing other cellular functions (or malfunctions) including directly or indirectly influencing cancerous cell growth or proliferation thereof in warm-blooded mammalian subjects. The direct or indirect influence on cancerous cell growth may involve but is not necessarily limited to any of prophylactic avoidance of cancerous cell formation, influencing of cell functions such as for exampleinfluencing leukocyte cell functions which can lead to inhibition of cancerous cell growth or proliferation thereof, and/or killing of cancerous cells harboured by a warm-blooded mammalian subject.

Electromagnetic energy generating devices and use of electromagnetic energies for treating living mammalian subjects harbouring cancerous cells described in the literature include: USP 5,908,441 issued June 1, 1999 to Bare; James E. and the references cited therein and so-called "NovoCure technology" involving in vivo implantation of electrodes to either side of tumorous growths. This literature however does not contemplate very low energy emissions of electromagnetic energy involving amplitude-modulated high frequency carrier signals as required in terms of the present invention.

US Patent 5,690,692 issued on November 25, 1997 entitled "Bio-Active Frequency Generator and Method" describes a programmable control which instructs a frequency synthesizer to enable generation of an electrical current at a specific precise frequency signal or at a series of specific precise frequencies signals having a square wave form to within an accuracy of 0.001 Hz. This Patent contemplates amplifying the voltage of the generated signals and applying the signals to a subject at the specific precise frequency or sequentially at the series of specific precise frequencies by means of electrodes held by or otherwise connected to the subject (which may be a mammal or a food). Once again, this Patent does not contemplate very low energy emissions involving amplitude-modulated high frequency carrier signals as required in terms of the present invention.

### SUMMARY OF THE INVENTION

In one aspect of the invention, an electronic system is provided which is activatable by electrical power. The system is employed to influence cellular functions or malfunctions in a warm-blooded mammalian subject. The system comprises one or more controllable low energy electromagnetic energy generator circuits for generating one or more high frequency radio frequency RF carrier signals. One or more microprocessors or integrated circuits comprising or communicating with the one or more generator circuits are provided which are also for receiving control information from a source of programmed control information. The one or more generator circuits include one or more amplitude modulation control signal generators for controlling amplitude modulated variations of the one or more high frequency carrier signals. The one or more generator circuits furthermore include one or more programmable amplitude modulation frequency control signal generators for controlling the frequency at which the amplitude modulations are generated. The one or more amplitude modulation frequency control generators are, in terms of an important improvement of the present invention, adapted to accurately control the frequency of the amplitude modulations to within an accuracy of at least 1000 ppm relative to one or more determined or predetermined reference amplitude modulation frequencies selected from within a range of 0.01 Hz to 150 kHz. The system furthermore comprises a connection or coupling position for connection or coupling to or being connected or coupled to an electrically conductive applicator for applying to the warm-blooded mammalian subject the one or more amplitude-modulated low energy emissions at said accurately controlled modulation frequencies.

As used herein, the term, "accurately controlled" means that the modulated low energy electromagnetic emissions should be modulated to within a resolution of at most about 1 Hz of intended higher frequencies (greater than about 1000 Hz) determined or predetermined modulation frequencies. For example, if one of the one or more determined or predetermined modulation frequencies to be applied to the warm-blooded mammalian subject is about 2000 Hz, the accurate control should lead to such modulated low energy emission being generated at a frequency of between about 1999 and about 2001 Hz. However, and in terms of what has been determined from experiences in treating human subjects harbouring cancerous cells with the aim of arresting proliferation or killing of such cells, it is preferable that the accurate control should lead to a resolution of about 0.5, more preferably about 0.1, yet more preferably about 0.01 and indeed most preferably about 0.001 Hz of the intended determined or predetermined modulation frequency.

Of importance is the requirement for emissions to be at a very low and safe energy level and result in low levels of absorption, the reason believed to be that physiological exchanges or flow of electrical impulses within warm-blooded animals (which are to be affected by application of the emissions of the present invention) are similarly at very low energy levels. In any event, in the region (at or near to the position of contact or close-by induction of the electrically conductive applicator with a subject receiving treatment), the specific absorption rate (SAR) should be and is most preferably substantially less than 1.6 milliW/g weight of living tissue.

Furthermore of importance to achieve the the intended biological therapeutic effect is that the stability of the emissions be maintained during emission, and that such stability should preferably be of the order of 10⁻⁵, more preferably 10⁻⁶, and most preferably 10⁻⁷, stability being determined as the relative deviation of frequency divided by the desired frequency, e.g. 0,01 Hz (deviation) / 1'000 Hz (desired freq.) = 10⁻⁵.

As already described in said EP 0 592 851 B1, the system includes a microprocessor (which may more recently be replaced by an integrated circuit) into which control information is loaded from an application storage device. The microprocessor (or now alternatively integrated circuit) then controls the function of the system to produce the desired therapeutic emissions. Also described is the provision in the system of an impedance transformer connected intermediate the emitter of low energy electromagnetic emissions and a probe (here more broadly described as an electrically conductive applicator) for applying the emissions to the patient. The impedance transformer substantially matches the impedance of the patient seen from the emitter circuit with the impedance of the output of the emitter circuit.

### BRIEF DESCRIPTION OF THE DRAWINGS

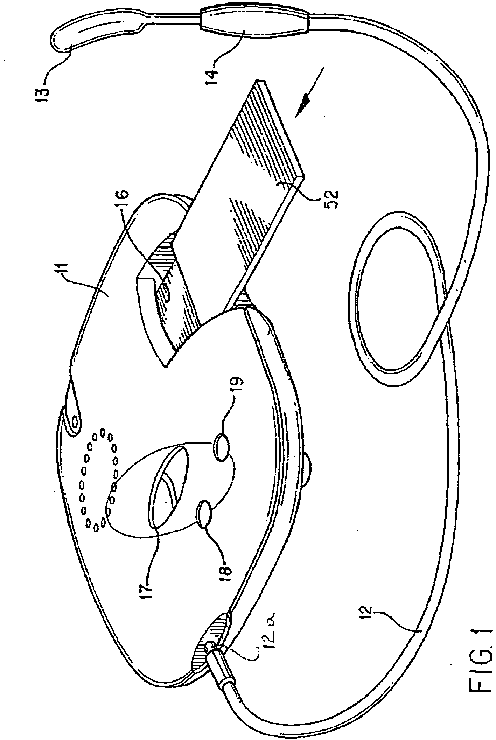

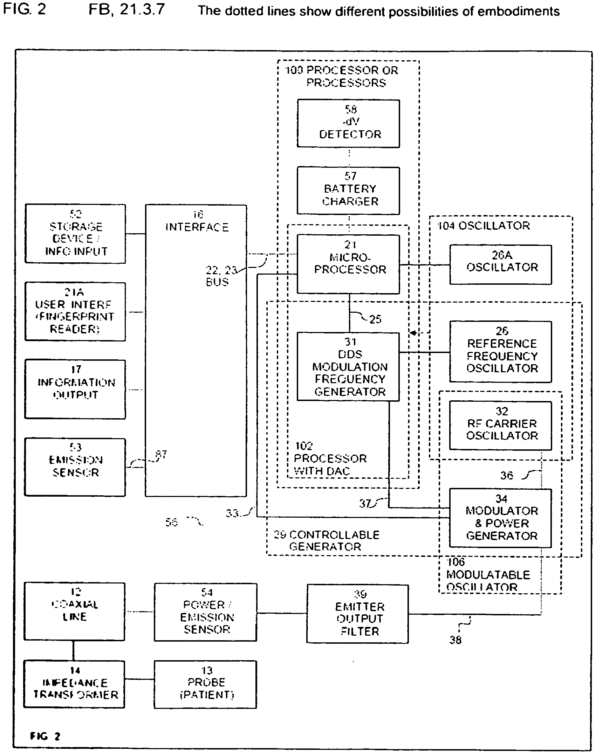

FIG. 1 shows an exemplary casing structure for the electronic circuit shown in Figure 2, an applicator 13 (exemplified as a probe suitable for being placed in the mouth of a patient) and an interface 16 (which may be replaced by a receiver) for receiving information from a source of information 52 such as may be comprised in an information storage device, e.g. of the nature described and illustrated in Figures 12 to 17 of EP 0 592 851 B1.
FIG. 2 is a block diagram of exemplary circuitry which may be comprised in the exemplary casing structure of FIG. 1. This Figure 2 differs essentially from Figure 2 of EP 0 592 851 B1 by comprising a highly accurate modulation frequency generator 31 (named a Digital Direct Synthesizer or DDS), which enables accurate control of modulatable oscillator represented by dotted line block 106.

### DETAILED DESCRIPTION

Referring to FIG. 1, presented is a modulated low energy electromagnetic emission application system 11, in accordance with the present invention. As described in prior U.S. Pat. Nos. 4,649,935 and 4,765,322, such a system has proven to be useful in the practice of *Low Energy Emission Therapy* (LEET, a trademark of Sym-tonic S.A. or a successor of this Company), which involves application of emissions of low energy radio frequency (RF) electromagnetic waves to a warm-blooded mammalian subject. The application has proven to be an effective mode of treating a warm-blooded mammalian subject suffering from central nervous system (CNS) disorders such as, for example, generalized anxiety disorders, panic disorders, sleep disorders including insomnia, psychiatric disorders such as depression, obsessive compulsive disorders, disorders resulting from substance abuse, sociopathy, post traumatic stress disorders or other disorders of the central nervous system and combinations thereof.

The system includes an electrically conductive applicator 12, 13 for applying one or more electromagnetic emissions to the warm-blooded mammalian subject. One form of applicator may consist of an electrically conductive probe or mouthpiece 13 which is inserted into the mouth of a subject undergoing treatment. Probe 13 is connected to an electromagnetic energy emitter (see also FIG. 2), through coaxial cable 12 and impedance matching transformer 14.

It has previously been considered that an efficient connection of an electrically conductive applicator to a subject could only be achieved by means of a probe which is adapted to be applied to any mucosa of the subject, such as by being located within oral, nasal, optical, urethral, anal, and/or vaginal cavities or surfaces. It has however now been determined that in fact satisfactory application of emissions to a patient can be achieved by simpler physical contact of the electrically conductive applicator with the skin of the patient. Emissions to the patient may, for example be achieved by a conductive, inductive, capacitive or radiated coupling to the patient. An example of a coupling found to be effective involving indirect physical contact with the skin of a patient, is an insulated applicator to be placed over or within an ear of the patient. The emissions thus passed to the patient may be either by capacitive or radiated means or by a combination of both. An important advantage of a device which does not need to be placed in the mouth of a patient is that the patient is able to speak clearly during a time of treatment and can receive treatment during activities of daily living. The treatment is accordingly more user-friendly, can be administered for longer periods of time and can lead to enhanced patient compliance.

Electronic system 11 also includes a connector or coupler for connection to a programmable device such as a computer or an interface or receiver 16 which is adapted to receive an application storage device 52 such as, for example, magnetic media, semiconductor media, optical media or mechanically encoded media, or programmed emissions programmed with control information employed to control the operation of system 11 so that the desired type of *low energy emission therapy* is applied to the patient.

Application storage device 52 can be provided with a microprocessor which, when applied to interface 16, operates to control the function of system 11 to apply the desired *low energy emission therapy.* Alternatively, application storage device 52 can be provided with a microprocessor which is used in combination with microprocessor 21 within system 11. In such case, the microprocessor within device 52 could assist in the interfacing of storage device 52 with system 11, or could provide security checking functions.

System 11 may also include a display 17 which can display various indications of the operation of system 11. In addition, system 11 may include on and off power buttons 18 and 19, optionally replaced by user interface 21A (refer to Figure 2).

Referring to FIG. 2, presented is a block diagram of exemplary electronic circuitry of system 11, in accordance with the present invention. A data processor, such as for example, microprocessor or integrated circuit 21, operates as the controller for electronic system 11, and is connected to control the various components of the system 11, for example, through address bus 22, data bus 23 and input/output lines 25. The block diagram of FIG. 2 is modified as compared to FIG.2 of EP 0 592 851 B1 by including what is known as a digital direct synthesizer (DDS) 31 which operates as a accurate and stable modulation frequency generator within the system 11. An exemplary DDS device is available from Analog Devices of Norwood, MA 02062-9106, USA, Part No AD9835. The device is a numerically controlled oscillator and modulation capabilities are provided for phase modulation and frequency modulation. As represented by dotted line block 102, entitled "PROCESSOR WITH DAC", the functionality of the DDS may also be combined with microprocessor 21 with digital to analogue converter (DAC).

Microprocessor 21 preferably includes internal storage for the operation of a coded control program, and temporary data. In addition, microprocessor 21 may include input/output ports and internal timers. Microprocessor 21 may be a microcontroller, for example microcontrollers 8048 or 8051 available from Intel Corporation of Santa Clara, CA 95054-1549, USA.

The timing for microprocessor 21 is provided by system clock oscillator 26A which may be run at any clock frequency suitable for the particular type of microprocessor used. An exemplary clock frequency is about 8.0 MHz. Oscillator 26A may be replaced by reference frequency oscillator 26 which secures the stability of the accurate modulation frequency. RF (radio frequency) oscillator 32 may also be employed for this purpose. A combination of oscillators is represented by dotted line block 104, entitled "OSCILLATOR".

In general, microprocessor 21 functions to control controllable electromagnetic energy generator circuit 29 to produce a desired form of modulated low energy electromagnetic emission for application to a subject through applicator or probe 13.

Dotted line block 29, entitled CONTROLLABLE GENERATOR, includes DDS modulation frequency generator 31 and carrier signal oscillator 32. Microprocessor 21 operates to activate or deactivate controllable generator circuit 29 through oscillator disable line 33. Controllable generator circuit 29 also includes an AM modulator and power generator 34 which operates to amplitude modulate a carrier signal produced by carrier oscillator 32 on carrier signal line 36, with a modulation signal produced by modulation signal generator circuit 31 on modulation signal line 37. The combination of the functionality of the DDS modulation frequency generator 31, with processor 21 with DAC, represented by dotted line block 102, enables output lines 33 and 37 to be combined to produce a single signal. The combination furthermore enables arbitrary or periodic wave forms of any shape to be generated.

AM modulatorand power generator 34 produces an amplitude modulated carrier signal on modulated carrier signal line 38, which is then applied to emitter output filter circuit 39. The filter circuit 39 is connected to probe or applicator 13 via power emission sensor 54, coaxial cable 12 and impedance transformer 14.

Microprocessor 21 controls DDS modulation signal generator circuit 31 of controllable generator circuit 29 via interface lines 25.

Microprocessor 21 may select a desired waveform stored in a modulation waveform storage device 43 and also controls a waveform address generator 41 to produce on waveform address bus 42 a sequence of addresses which are applied to modulation signal storage device 43 in order to retrieve the selected modulation signal.

A further embodiment possibility is a combination of PROCESSOR WITH DAC dotted line block 102 with OSCILLATOR dotted line block 104 or with a combination of oscillators 26 and 26A. With such a combination, the hardware solution can be realized internally in the processor 102 with multiple outputs 33 and 37 or a single output combining these signals.

Wave forms that have been successfully employed include square wave forms or sinusoidal wave forms. Other possible modulation signal wave forms include rectified sinusoidal, triangular, or other wave forms and combinations of all of the above.

The particular modulation control information employed by microprocessor 21 to control the operation of controllable generator circuit 29, is stored in application storage device 52. The application storage device is conveniently a computer comprising or being for receiving the information.

Interface 16 is configured as appropriate for the particular application storage device 52 in use. Interface 16 translates the control information stored in application storage device 52 into a usable form for storage within the memory of microprocessor 21 to enable microprocessor 21 to control controllable generator circuit 29 to produce the desired modulated low energy emission.

Interface 16 may directly read the information stored on application storage device 52, or it may read the information through use of various known communication links. For example, radio frequency, microwave, laser, telephone, internet or optical based communications links may be employed to transfer information between interface or receiver 16 and application storage device or computer 52.

The system 11 may comprise a user identification device, included in block 21a in Figure 2. Conveniently, such a device communicates with the one or more data processors or integrated circuits 21 via interface 16, as shown. The user identification device may be of any type, a finger print reader being an example. Such a reader is for example available from Lenovo, 70563 Stuttgart, Germany, Part No. 73P4774.

The control information stored in application storage device or computer 52 specifies various controllable parameters of the modulated low energy RF electromagnetic emission to be applied to a subject through applicator or probe 13. Such controllable parameters include, for example, but are not necessarily limited to, the frequency and amplitude of the carrier, the amplitudes and frequencies and wave forms of the modulation of the carrier, the duration of the emission, the power level of the emission, the duty cycle of the emission (i.e., the ratio of on time to off time of pulsed emissions applied during a treatment), the sequence of application of different modulation frequencies for a particular application, and the total number of treatments and duration of each treatment prescribed for a particular subject, and combinations thereof.

For example, the carrier signal and modulation signal may be selected to drive the applicator or probe 13 with an amplitude modulated signal in which the carrier signal includes spectral frequency components below about 1 GHz, and preferably between about 1 MHz and about 900 MHz, and in which the modulation signal comprises spectral frequency components between about 0.01 Hz and 150 KHz. The one or more modulation frequencies may be simultaneously emitted or sequenced to form the modulation signal.

As an additional feature, an electromagnetic emission sensor 53 may be provided to detect the presence of electromagnetic emissions at the frequency of the carrier oscillator 32. Emission sensor 53 provides microprocessor 21 with an indication of whether or not electromagnetic emissions at the desired frequency are present. Microprocessor 21 then takes appropriate action, for example, by displaying an error message on display 17, disabling controllable generator circuit 29, or the like.

A power sensor 54 is preferably included which detects the amount of power applied to the subject through applicator or probe 13 compared to the amount of power returned or reflected from the subject. This ratio is indicative of the proper use of the system during a therapeutic session. Power sensor 54 applies to microprocessor 21, through power sensor line 56, an indication of the amount of power applied to patient through applicator or probe 13 relative to the amount of power reflected from the patient.

The indication provided on power sense line 56 may be digitalized and employed by microprocessor 21, for example, to detect and control a level of applied power, and to record on application storage device 52 information related to the actual treatments applied to and received by the patient. Such information may then be used by a physician or other clinician to assess patient treatment compliance and effect. Such treatment information may include, for example: the number of treatments applied for a given time period; the actual time and date of each treatment; the number of attempted treatments; the treatment compliance (i.e., whether the applicator or probe was in place or not during the treatment session); and the cumulative dose of a particular modulation frequency.

The level of power applied is preferably controlled to cause the specific absorption rate (SAR) of energy absorbed by the patient to be from about 1 microWatt per kilogram of tissue to about50 Watts per kilogram of tissue. Preferably, the power level is controlled to cause an SAR of from about 100 microWatts per kilogram of tissue to about 10 Watts per kilogram of tissue. Most preferably, the power level is controlled to cause an SAR of from about 1 milliWatt per kilogram of tissue to about 100 milliWatts per kilogram of tissue. These SARs may be in any tissue of the patient, but are preferably in the tissue of the central nervous system or the diseased tissue.

System 11 may also include powering circuitry including battery and charger circuit 57 and battery voltage change detector 58.

The RF carrier oscillator 32 produces a RF carrier frequency of about 27 MHz. Other embodiments of the invention contemplate RF carrier frequencies of about 48 MHz, about 433 MHz or about 900 MHz. In general, the RF carrier frequency produced by carrier oscillator 32 has spectral frequency components less than about 1 GHz and preferably between about 1 MHz and about 916 MHz. Although the described embodiment contemplates that once set, the carrier oscillator frequency remains substantially constant, the carrier frequency produced by carrier oscillator 32 may be variable and controllable by microprocessor 21 by use of stored or transmitted control information.

Carrier oscillator 32 produces on carrier signal line 36 a carrier signal which is then modulated by the modulation signal carried on signal line 37.

Oscillator disable line 33 enables microprocessor 21 to disable the signal from oscillator 32 by applying an appropriate disable signal to oscillator disable line 33.

The output of the AM modulator and power generator 34 appears on signal line 38. This modulated signal is applied through emitter output filter 39 which substantially reduces or eliminates the carrier harmonics resulting from side effects of the modulator and power generator circuit 34.

The output of the AM modulator and power generator 34 and emitter output filter 39 may be designed to possess a 50 Ohm output impedance to match a 50 Ohm impedance of coaxial cable 12.

It has been determined through impedance measurements that when a probe 13 is applied within the mouth of a subject, the probe/subject combination exhibits a complex impedance of the order of about 150+j200 Ohms. Impedance transformer 14 serves to match this complex impedance with the 50 Ohm impedance of coaxial cable 12 and therefore the output impedance of the AM modulator 34 and output filter 39. This promotes power transmission, and minimizes reflections.

The arrangement described above has been optimized for a contact probe with coupling to the mucosa of the mouth. In a further example, a conductive, isolated probe has been used at a frequency around 433 MHz coupling to the outer ear channel. Due to the different probe design in such a frequency band and with this coupling method, the values of matching elements (79 and 81 described in EP 0592 851 B1) would be different or could even be omitted. Applicator or probe 13 may then be regarded as a capacitive coupler or as an antenna matched to the capacitive load.

As described in EP 0 592 851 B1, with reference to the flow charts of FIGS. 11 a-d, microprocessor 21 may operate to analyse the signal appearing on power sense line 56 to determine and control the amount of power applied to the patient, and to assess patient treatment compliance, and possibly to record indicia of the patient treatment compliance on application storage device 52 for later analysis and assessment by a physician or other clinician.

Exemplary of treatments performed on patients have included brain, bladder, colorectal, kidney, mesothelium, neuroendocrine, liver, lung, breast, ovary, pancreas, prostate and thyroid tumour types. The treatments involved applying an about 27.12 MHz RF signal, amplitude modulated at specifically defined frequencies ranging from about 0.2 to about 23,000 Hz at very high precision and stability. Further Examples of treatment modes (at specific accurately controlled AM frequencies) for specified types of tumours are described in detail below.

The following are synopses of abstracts for future publications related to uses of electronic devices of the present invention:

### Example A

### A phase I study of therapeutic amplitude-modulated electromagnetic fields (THERABIONIC) in advanced tumors

Boris Pasche¹, Alexandre Barbault ¹, Brad Bottger ², Fin Bomholt ³, Niels Kuster⁴
¹ Cabinet Medical de l'Avenue de la Gare 6, CH-1003-Lausanne, Switzerland.
² Danbury Hospital, Danbury, CT-06810.
³ SPEAG, Zurich, CH-8004-Zurich, Switzerland
⁴ IT'IS Foundation, Swiss Federal Institute of Technology, Zurich, Switzerland.

Background: *In vitro* studies suggest that low levels of amplitude-modulated electromagnetic fields may modify cell growth. Specific frequencies have been identified specific frequencies that may block cancer cell growth. A portable and programmable device capable of delivering low levels of amplitude-modulated electromagnetic fields has been developed. The device emits a 27.12 MHz radiofrequency signal, amplitude-modulated at cancer-specific frequencies ranging from 0.2 to 23,000 Hz with high precision. The device is connected to a spoon-like coupler, which is placed in the patient's mouth during treatment.

Methods: A phase I study was conducted consisting of three daily 40 min treatments. From March 2004 to September 2006, 24 patients with advanced solid tumors were enrolled. The median age was 57.0 ± 12.2 years. 16 patients were female. As of January 2007, 5 patients are still on therapy, 13 patients died of tumor progression, 2 patients are lost to follow-up and one patient withdrew consent. The most common tumor types were breast (7), ovary (5) and pancreas (3). 22 patients had received prior systemic therapy and 16 had documented tumor progression prior to study entry.

Results: The median duration of therapy was 15.7 ± 19.9 weeks (range: 0.4-72.0 weeks). There were no NCI grade 2, 3 or 4 toxicities. Three patients experienced grade 1 fatigue during and immediately after treatment. 12 patients reported severe pain prior to study entry. Two of them reported significant pain relief with the treatment. Objective response could be assessed in 13 patients, 6 of whom also had elevated tumor markers. 6 additional patients could only be assessed by tumor markers. Among patients with progressive disease at study entry, one had a partial response for > 14.4 weeks associated with > 50% decrease in CEA, CA 125 and CA 15-3 (previously untreated metastatic breast cancer); one patient had stable disease for 34.6 weeks (add info); one patient had a 50% decrease in CA 19-9 for 12.4 weeks (recurrent pancreatic cancer). Among patients with stable disease at enrollment, four patients maintained stable disease for 17.0, > 19.4, 30.4 and > 63.4 weeks.

Conclusions: The treatment is a safe and promising novel treatment modality for advanced cancer. A phase II study and molecular studies are ongoing to confirm those results.

### Example B

### A phase II study of therapeutic amplitude-modulated electromagnetic fields (THERABIONIC) in the treatment of advanced hepatocellular carcinoma (HCC)

Frederico P Costa ¹, Andre Cosme de Oliveira ¹, Roberto Meirelles Jr ¹, Rodrigo Surjan ¹, Tatiana Zanesco¹, Maria Cristina Chammas ¹, Alexandre Barbault ², Boris Pasche ².
¹ Hospital das Clinicas da Faculdade de Medicina da Universidade de São Paulo, São Paulo, Brazil. ² Cabinet Medical Avenue de la Gare 6, CH-1003-Lausanne, Switzerland

Background : Phase I data suggest that low levels of electromagnetic fields amplitude-modulated at specific frequencies administered intrabucally with the device of Example A are a safe and potentially effective treatment for advanced cancer. The device emits a 27.12 MHz RF signal, amplitude-modulated with cancer-specific frequencies ranging from 0.2 to 23,000 Hz with high precision. The device is connected to a spoon-like coupler placed in the patient's mouth during treatment. Patients with advanced hepatocellular carcinoma HCC and limited therapeutic options were offered treatment with a combination of HCC-specific frequencies.

Methods: From October 2005 to October 2006, 38 patients with advanced HCC were recruited in a phase II study. The patients received three daily 40 min treatments until disease progression or death. The median age was 64.0 ± 14.2 years. 32 patients were male and 29 patients had documented progression of disease (POD) prior to study entry.

Results: As of January 2007, 12 patients are still on therapy, 20 patients died of tumor progression, 2 patients are lost to follow-up and 3 patients withdrew consent. 27 patients are eligible for response. The overall objective response rate as defined by partial response (PR) or stable disease (SD) in patients with documented POD at study entry was 31.6%: 3 PR and 9 SD. The median survival was 20.7 weeks with a median duration of therapy of 17.5 weeks. 13 patients have received therapy for more than six months. The median duration of response is 12.9 weeks. 12 patients reported pain at study entry: 8 of them (66%) experienced decreased pain during treatment. There were no NCI grade 2/3/4 toxicities. One patient developed grade 1 mucositis and grade 1 fatigue.

| | | |
|---|---|---|
| Patient characteristics (n = 38) | | |
| Cirrhosis | 36 | |
| Portal vein thrombosis | 9 | |
| Elevated AFP | 25 | |
| Extra-hepatic metastases | 12 | |
| Previous intrahepatic/systemic therapy | 30 | |
| Previous hepatic resection/RFA or ethanol | 8 | |
| CLIP | 0/1: 12 | ≥ 2: 22 |
| Okuda | I: 14 | II/III: 20 |
| Child-Pugh | A: 15 | B: 19 |
| MELD | Median: 10 | |

Conclusion: In patients with advanced HCC the treatment is a safe and effective novel therapeutic option, which has antitumor effect and provides pain relief in the majority of patients.

Thus, it is seen that the electronic device of the present invention, comprising means for the accurate control over the frequencies and stability of amplitude modulations of a high frequency carrier signal, provides a safe and promising novel treatment modality for the treatment of patients suffering from various types of advanced forms of cancer.

Exemplary of above accurately controlled amplitude modulated frequencies controlling the frequency of amplitude modulations of a high frequency carrier signal are set forth below along with the type of cancer or tumour harboured by a subject to be treated.

### Example 1. AM Frequencies employed for treatment of breast cancer (188 frequencies so far included):

| | | |
|---|---|---|
| 78,76 Hz | 3434,693 Hz | 5426,323 Hz |
| 181,821 Hz | 3594,231 Hz | 5431,542 Hz |
| 414,817 Hz | 3647,619 Hz | 5521,621 Hz |
| 440,933 Hz | 3742,957 Hz | 5739,422 Hz |
| 628,431 Hz | 3753,382 Hz | 5745,218 Hz |
| 721,313 Hz | 3830,732 Hz | 5821,975 Hz |
| 813,205 Hz | 3855,823 Hz | 6037,432 Hz |
| 818,342 Hz | 3916,321 Hz | 6044,333 Hz |
| 891,901 Hz | 3935,218 Hz | 6086,256 Hz |
| 929,095 Hz | 3975,383 Hz | 6208,932 Hz |
| 929,1 Hz | 3993,437 Hz | 6212,808 Hz |
| 1021 Hz | 4153,192 Hz | 6231,031 Hz |
| 1372,207 Hz | 4194,968 Hz | 6280,321 Hz |
| 1372,934 Hz | 4241,321 Hz | 6329,391 Hz |
| 1588,721 Hz | 4243,393 Hz | 6476,896 Hz |
| 1670,699 Hz | 4253,432 Hz | 6497,319 Hz |
| 1821,729 Hz | 4314,444 Hz | 6504,983 Hz |
| 1836,219 Hz | 4318,222 Hz | 6651,276 Hz |
| 2193,937 Hz | 4375,962 Hz | 6757,901 Hz |
| 2221,323 Hz | 4393,419 Hz | 6758,321 Hz |
| 2278,312 Hz | 4417,243 Hz | 6855,286 Hz |
| 2357,832 Hz | 4481,463 Hz | 6858,121 Hz |
| 2381,443 Hz | 4482,223 Hz | 6898,489 Hz |
| 2417,323 Hz | 4495,138 Hz | 7092,219 Hz |
| 2431,334 Hz | 4549,808 Hz | 7120,218 Hz |
| 2450,332 Hz | 4558,306 Hz | 7127,311 Hz |
| 2551,313 Hz | 4779,451 Hz | 7156,489 Hz |
| 2556,221 Hz | 4838,674 Hz | 7208,821 Hz |
| 2598,853 Hz | 4871,513 Hz | 7282,169 Hz |
| 2621,322 Hz | 4895,296 Hz | 7376,329 Hz |
| 2740,191 Hz | 4962,213 Hz | 7488,742 Hz |
| 2851,347 Hz | 4969,224 Hz | 7541,319 Hz |
| 2885,322 Hz | 4979,321 Hz | 7577,421 Hz |
| 2919,273 Hz | 5027,231 Hz | 7621,085 Hz |
| 3074,333 Hz | 5059,792 Hz | 7627,207 Hz |
| 3115,188 Hz | 5118,094 Hz | 7650,939 Hz |
| 3249,529 Hz | 5176,287 Hz | 7691,212 Hz |
| 3405,182 Hz | 5365,222 Hz | 7842,184 Hz |
| 3432,274 Hz | 5376,392 Hz | 7849,231 Hz |
| 7915,423 Hz | 9012,282 Hz | 11840,323 Hz |
| 7932,482 Hz | 9012,896 Hz | 11925,089 Hz |
| 7949,196 Hz | 9060,323 Hz | 12123,281 Hz |
| 7967,311 Hz | 9072,409 Hz | 12267,281 Hz |
| 8021,229 Hz | 9131,419 Hz | 12294,283 Hz |
| 8070,181 Hz | 9199,232 Hz | 12611,288 Hz |
| 8114,032 Hz | 9245,927 Hz | 12629,222 Hz |
| 8149,922 Hz | 9270,322 Hz | 12633,372 Hz |
| 8194,19 Hz | 9279,193 Hz | 12648,221 Hz |
| 8245,801 Hz | 9393,946 Hz | 13315,335 Hz |
| 8328,322 Hz | 10227,242 Hz | 13331,358 Hz |
| 8330,534 Hz | 10340,509 Hz | 13735,241 Hz |
| 8355,987 Hz | 10363,313 Hz | 13826,325 Hz |
| 8408,121 Hz | 10449,323 Hz | 13853,232 Hz |
| 8431,184 Hz | 10456,383 Hz | 13990,123 Hz |
| 8452,119 Hz | 10468,231 Hz | 14122,942 Hz |
| 8548,324 Hz | 10470,456 Hz | 14162,332 Hz |
| 8749,383 Hz | 10472,291 Hz | 14519,232 Hz |
| 8782,421 Hz | 10689,339 Hz | 14543,128 Hz |
| 8784,424 Hz | 10832,222 Hz | 15651,323 Hz |
| 8923,1 Hz | 11525,121 Hz | 17352,085 Hz |
| 8923,361 Hz | 11541,915 Hz | 18785,463 Hz |
| 8935,752 Hz | 11812,328 Hz | 30182,932 Hz |
| 8936,1 Hz | 11812,419 Hz | |

### Example 2. AM Frequencies employed for treatment oliver cancer (162 frequencies so far included):

| | | |
|---|---|---|
| 423,321 Hz | 1975,196 Hz | 2743,995 Hz |
| 427,062 Hz | 2017,962 Hz | 2744,211 Hz |
| 470,181 Hz | 2083,419 Hz | 2831,951 Hz |
| 560,32 Hz | 2190,731 Hz | 2843,283 Hz |
| 642,932 Hz | 2221,323 Hz | 2859,891 Hz |
| 668,209 Hz | 2324,393 Hz | 2873,542 Hz |
| 677,972 Hz | 2353,478 Hz | 2886,232 Hz |
| 811,924 Hz | 2362,309 Hz | 3042,012 Hz |
| 842,311 Hz | 2419,309 Hz | 3078,983 Hz |
| 843,22 Hz | 2425,222 Hz | 3086,443 Hz |
| 1250,504 Hz | 2430,219 Hz | 3127,232 Hz |
| 1755,402 Hz | 2431,094 Hz | 3160,942 Hz |
| 1873,477 Hz | 2471,328 Hz | 3206,315 Hz |
| 1924,702 Hz | 2478,331 Hz | 3267,433 Hz |
| 3269,321 Hz | 6383,321 Hz | 9332,397 Hz |
| 3457,291 Hz | 6461,175 Hz | 9381,221 Hz |
| 3505,229 Hz | 6733,331 Hz | 9740,219 Hz |
| 3516,296 Hz | 6758,232 Hz | 9768,331 Hz |
| 3531,296 Hz | 6779,482 Hz | 9797,294 Hz |
| 3546,323 Hz | 6856,222 Hz | 10317,499 Hz |
| 3572,106 Hz | 6877,183 Hz | 10443,311 Hz |
| 3576,189 Hz | 6980,525 Hz | 10456,383 Hz |
| 3669,513 Hz | 7019,235 Hz | 10579,425 Hz |
| 3923,221 Hz | 7043,209 Hz | 10863,209 Hz |
| 4013,932 Hz | 7130,323 Hz | 10866,382 Hz |
| 4071,121 Hz | 7144,142 Hz | 11067,418 Hz |
| 4079,951 Hz | 7210,223 Hz | 11149,935 Hz |
| 4222,821 Hz | 7291,21 Hz | 11163,895 Hz |
| 4238,402 Hz | 7510,92 Hz | 11802,821 Hz |
| 4256,321 Hz | 7529,233 Hz | 11953,424 Hz |
| 4289,296 Hz | 7549,212 Hz | 12223,329 Hz |
| 4312,947 Hz | 7650,028 Hz | 12265,295 Hz |
| 4435,219 Hz | 7680,518 Hz | 12267,233 Hz |
| 4471,188 Hz | 7692,522 Hz | 12623,191 Hz |
| 4483,889 Hz | 7829,231 Hz | 12685,231 Hz |
| 4486,384 Hz | 7862,209 Hz | 12721,423 Hz |
| 4629,941 Hz | 7947,392 Hz | 12785,342 Hz |
| 4732,211 Hz | 7979,308 Hz | 14085,222 Hz |
| 4876,218 Hz | 8028,339 Hz | 14333,209 Hz |
| 5086,281 Hz | 8055,942 Hz | 14537,331 Hz |
| 5124,084 Hz | 8072,134 Hz | 14542,432 Hz |
| 5133,121 Hz | 8141,174 Hz | 14655,03 Hz |
| 5247,142 Hz | 8336,383 Hz | 14828,234 Hz |
| 5270,834 Hz | 8432,181 Hz | 15149,213 Hz |
| 5340,497 Hz | 8452,119 Hz | 15237,489 Hz |
| 5520,218 Hz | 8460,944 Hz | 16110,932 Hz |
| 5882,292 Hz | 8475,221 Hz | 16144,343 Hz |
| 5926,512 Hz | 8492,193 Hz | 18265,238 Hz |
| 6037,311 Hz | 8542,311 Hz | 18283,323 Hz |
| 6180,334 Hz | 8818,104 Hz | 18863,292 Hz |
| 6329,195 Hz | 8852,329 Hz | 18930,995 Hz |
| 6350,333 Hz | 8853,444 Hz | 19970,311 Hz |
| 6361,321 Hz | 8858,179 Hz | 20330,294 Hz |
| 6364,928 Hz | 8939,212 Hz | 20365,284 Hz |

### Example 3. AM Frequencies employed for treatment of ovarian cancer (273 frequencies so far included):

| | | |
|---|---|---|
| 78,76 Hz | 1552,123 Hz | 2973,771 Hz |
| 181,821 Hz | 1579,212 Hz | 3080,592 Hz |
| 410,245 Hz | 1624,802 Hz | 3157,483 Hz |
| 414,817 Hz | 1670,699 Hz | 3161,465 Hz |
| 436,332 Hz | 1696,403 Hz | 3223,232 Hz |
| 447,942 Hz | 1762,938 Hz | 3238,148 Hz |
| 481,191 Hz | 1771,402 Hz | 3249,529 Hz |
| 489,292 Hz | 1775,313 Hz | 3262,145 Hz |
| 559,292 Hz | 1821,729 Hz | 3314,321 Hz |
| 608,321 Hz | 2016,323 Hz | 3361,671 Hz |
| 655,435 Hz | 2034,231 Hz | 3366,311 Hz |
| 657,397 Hz | 2050,282 Hz | 3523,215 Hz |
| 657,483 Hz | 2053,396 Hz | 3527,233 Hz |
| 664,211 Hz | 2082,234 Hz | 3542,213 Hz |
| 708,8 Hz | 2089,092 Hz | 3590,376 Hz |
| 708,822 Hz | 2221,323 Hz | 3629,232 Hz |
| 734,921 Hz | 2228,832 Hz | 3632,793 Hz |
| 749,221 Hz | 2253,704 Hz | 3636,289 Hz |
| 764,232 Hz | 2254,329 Hz | 3637,085 Hz |
| 778,295 Hz | 2278,312 Hz | 3669,513 Hz |
| 779,403 Hz | 2332,949 Hz | 3770,189 Hz |
| 806,021 Hz | 2348,233 Hz | 3858,916 Hz |
| 806,389 Hz | 2381,443 Hz | 3919,232 Hz |
| 809.313 Hz | 2413,193 Hz | 3957,185 Hz |
| 824,327 Hz | 2425,222 Hz | 3975,228 Hz |
| 825,145 Hz | 2433,321 Hz | 4061,131 Hz |
| 835,129 Hz | 2439,253 Hz | 4072,322 Hz |
| 839,521 Hz | 2465,23 Hz | 4169,451 Hz |
| 841,208 Hz | 2477,919 Hz | 4174,259 Hz |
| 843,312 Hz | 2669,177 Hz | 4241,321 Hz |
| 956,984 Hz | 2715,232 Hz | 4243,393 Hz |
| 958,929 Hz | 2733,843 Hz | 4261,228 Hz |
| 985,313 Hz | 2802,339 Hz | 4279,113 Hz |
| 1024,208 Hz | 2812,321 Hz | 4309,335 Hz |
| 1102,635 Hz | 2831,386 Hz | 4314,188 Hz |
| 1121,329 Hz | 2835,332 Hz | 4318,222 Hz |
| 1159,738 Hz | 2851,347 Hz | 4328,928 Hz |
| 1372,207 Hz | 2877,192 Hz | 4380,321 Hz |
| 1396,498 Hz | 2885,322 Hz | 4394,134 Hz |
| 1502,181 Hz | 2887,385 Hz | 4412,252 Hz |
| 1518,208 Hz | 2894,972 Hz | 4424,236 Hz |
| 4439,341 Hz | 6855,286 Hz | 8779,323 Hz |
| 4442,161 Hz | 6875,232 Hz | 8792,231 Hz |
| 4447,221 Hz | 6882,949 Hz | 8819,127 Hz |
| 4458,339 Hz | 7206,403 Hz | 8831,132 Hz |
| 4556,322 Hz | 7232,214 Hz | 9028,031 Hz |
| 4566,009 Hz | 7257,489 Hz | 9173,264 Hz |
| 4682,643 Hz | 7276,209 Hz | 9184,338 Hz |
| 4718,331 Hz | 7281,219 Hz | 9186,919 Hz |
| 4749,302 Hz | 7285,693 Hz | 9393,946 Hz |
| 4765,331 Hz | 7429,212 Hz | 9482,409 Hz |
| 4917,202 Hz | 7460,932 Hz | 9737,211 Hz |
| 5011,325 Hz | 7480,228 Hz | 9746,232 Hz |
| 5149,331 Hz | 7495,763 Hz | 9922,231 Hz |
| 5228,172 Hz | 7539,432 Hz | 10032,684 Hz |
| 5237,132 Hz | 7564,185 Hz | 10446,028 Hz |
| 5313,353 Hz | 7650,028 Hz | 10478,221 Hz |
| 5745,218 Hz | 7689,728 Hz | 10545,313 Hz |
| 5757,897 Hz | 7780,294 Hz | 10639,345 Hz |
| 5762,386 Hz | 8021,921 Hz | 10743,118 Hz |
| 5812,322 Hz | 8038,961 Hz | 10813,981 Hz |
| 5869,321 Hz | 8040,322 Hz | 10832,421 Hz |
| 5882,292 Hz | 8044,233 Hz | 10838,243 Hz |
| 5921,249 Hz | 8095,313 Hz | 10862,429 Hz |
| 5991,932 Hz | 8143,491 Hz | 10865,127 Hz |
| 6069,458 Hz | 8164,332 Hz | 10917,229 Hz |
| 6071,319 Hz | 8261,121 Hz | 10977,188 Hz |
| 6083,214 Hz | 8302,285 Hz | 11120,209 Hz |
| 6161,782 Hz | 8309,752 Hz | 11177,289 Hz |
| 6169,341 Hz | 8372,532 Hz | 11177,409 Hz |
| 6275,232 Hz | 8408,121 Hz | 11321,491 Hz |
| 6294,929 Hz | 8424,229 Hz | 11359,093 Hz |
| 6350,333 Hz | 8428,313 Hz | 11673,031 Hz |
| 6406,891 Hz | 8435,451 Hz | 11793,886 Hz |
| 6407,207 Hz | 8486,421 Hz | 11895,229 Hz |
| 6450,787 Hz | 8492,797 Hz | 12074,531 Hz |
| 6477,098 Hz | 8548,324 Hz | 12216,212 Hz |
| 6477,929 Hz | 8554,361 Hz | 12253,329 Hz |
| 6478,338 Hz | 8562,965 Hz | 12260,933 Hz |
| 6543,421 Hz | 8579,323 Hz | 12262,853 Hz |
| 6552,24 Hz | 8579,333 Hz | 12292,222 Hz |
| 6663,955 Hz | 8642,181 Hz | 12357,353 Hz |
| 6753,338 Hz | 8655,818 Hz | 12527,032 Hz |
| 6851,323 Hz | 8758,341 Hz | 12755,333 Hz |
| 12947,311 Hz | 14947,184 Hz | 17970,122 Hz |
| 13717,221 Hz | 15429,139 Hz | 18337,222 Hz |
| 13825,295 Hz | 15443,309 Hz | 18378,321 Hz |
| 13829,195 Hz | 15450,183 Hz | 18921,415 Hz |
| 14410,949 Hz | 16144,343 Hz | 18926,951 Hz |
| 14436,201 Hz | 17932,432 Hz | 18931,327 Hz |
| 14537,218 Hz | 17951,395 Hz | 114508,332 Hz |

### Example 4. AM Frequencies employed for treatment of prostate cancer (183 frequencies so far included):

| | | |
|---|---|---|
| 331,3 Hz | 847,332 Hz | 3251,815 Hz |
| 331,358 Hz | 1083,309 Hz | 3264,827 Hz |
| 403,218 Hz | 1102,635 Hz | 3278,329 Hz |
| 461,233 Hz | 1102,71 Hz | 3281,432 Hz |
| 522,2 Hz | 1240,336 Hz | 3348,783 Hz |
| 522,213 Hz | 1372,934 Hz | 3519,118 Hz |
| 618,4 Hz | 1444,288 Hz | 3539,962 Hz |
| 618,407 Hz | 1486,322 Hz | 3551,318 Hz |
| 618,8 Hz | 1563,332 Hz | 3556,439 Hz |
| 656,295 Hz | 1591,322 Hz | 3572,321 Hz |
| 657,394 Hz | 1670,699 Hz | 3670,129 Hz |
| 657,397 Hz | 1697,321 Hz | 3681,341 Hz |
| 657,4 Hz | 1743,521 Hz | 3686,021 Hz |
| 657,483 Hz | 2031,448 Hz | 3753,382 Hz |
| 659,033 Hz | 2050,282 Hz | 3774,923 Hz |
| 694.4 Hz | 2076,519 Hz | 3867,692 Hz |
| 694,689 Hz | 2156,332 Hz | 3909,333 Hz |
| 694,7 Hz | 2229,515 Hz | 3916,321 Hz |
| 741,4 Hz | 2243,121 Hz | 4031,233 Hz |
| 741,421 Hz | 2381,443 Hz | 4031,933 Hz |
| 749,221 Hz | 2440,489 Hz | 4038,203 Hz |
| 752,9 Hz | 2475,912 Hz | 4081,743 Hz |
| 752,933 Hz | 2477,919 Hz | 4084,319 Hz |
| 776,194 Hz | 2628,324 Hz | 4139,322 Hz |
| 785,219 Hz | 2669,328 Hz | 4153,192 Hz |
| 786,332 Hz | 2824,832 Hz | 4223,795 Hz |
| 793,331 Hz | 2887,829 Hz | 4231,221 Hz |
| 809,205 Hz | 2891,331 Hz | 4241,321 Hz |
| 819,322 Hz | 3081,523 Hz | 4320,513 Hz |
| 844,8 Hz | 3249,529 Hz | 4329,152 Hz |
| 844,822 Hz | 3250,125 Hz | 4380,321 Hz |
| 4417,312 Hz | 6871,943 Hz | 9351,931 Hz |
| 4489,452 Hz | 6973,393 Hz | 9393,946 Hz |
| 4549,808 Hz | 7120,932 Hz | 9694,179 Hz |
| 4558,306 Hz | 7146,509 Hz | 9984,405 Hz |
| 4638,293 Hz | 7192,505 Hz | 10226,223 Hz |
| 4740,322 Hz | 7251,309 Hz | 10390,232 Hz |
| 4854,318 Hz | 7251,322 Hz | 10514,768 Hz |
| 4882,322 Hz | 7278,124 Hz | 10689,339 Hz |
| 4978,822 Hz | 7279,335 Hz | 10772,419 Hz |
| 5237,152 Hz | 7299,119 Hz | 10818,452 Hz |
| 5264,222 Hz | 7527,229 Hz | 11165,239 Hz |
| 5289,195 Hz | 7589,925 Hz | 11985,353 Hz |
| 5426,323 Hz | 7699,193 Hz | 12209,329 Hz |
| 5431,542 Hz | 7842,184 Hz | 12308,321 Hz |
| 5455,593 Hz | 8023,32 Hz | 12583,339 Hz |
| 6345,332 Hz | 8096,939 Hz | 13820,329 Hz |
| 6347,433 Hz | 8245,801 Hz | 14013,123 Hz |
| 6363,284 Hz | 8315,291 Hz | 14171,434 Hz |
| 6418,331 Hz | 8357,305 Hz | 14681,329 Hz |
| 6496,231 Hz | 8408,121 Hz | 14759,131 Hz |
| 6538,295 Hz | 8432,209 Hz | 14986,794 Hz |
| 6577,421 Hz | 8535,238 Hz | 15930,249 Hz |
| 6590,328 Hz | 8552,431 Hz | 16026,623 Hz |
| 6651,276 Hz | 8585,224 Hz | 17880,954 Hz |
| 6706,431 Hz | 8935,752 Hz | 18247,532 Hz |
| 6743,322 Hz | 9015,253 Hz | 18282,211 Hz |
| 6783,282 Hz | 9018,233 Hz | 18629,328 Hz |
| 6850,197 Hz | 9068,231 Hz | 19469,318 Hz |
| 6855,286 Hz | 9137,232 Hz | 19766,218 Hz |
| 6864,896 Hz | 9156,321 Hz | 60317,352 Hz |

### Example 5. AM Frequencies employed for treatment of kidney cancer (36 frequencies so far included):

| | | |
|---|---|---|
| 628,321 Hz | 2254,329 Hz | 7054,279 Hz |
| 631,141 Hz | 3555,209 Hz | 7074,429 Hz |
| 643,312 Hz | 3928,343 Hz | 7254,343 Hz |
| 812,512 Hz | 4420,932 Hz | 8041,289 Hz |
| 826,321 Hz | 4819,228 Hz | 8727,224 Hz |
| 1372,934 Hz | 4828,321 Hz | 8760,983 Hz |
| 2082,241 Hz | 5314,322 Hz | 8831,132 Hz |
| 2156,931 Hz | 6007,332 Hz | 8870,228 Hz |
| 10565,321 Hz | 11421,933 Hz | 12631,331 Hz |
| 10586,229 Hz | 11523,212 Hz | 12693,272 Hz |
| 10634,293 Hz | 11561,221 Hz | 14411,321 Hz |
| 10687,949 Hz | 11846,212 Hz | 20178,941 Hz |

### Example 6. AM Frequencies employed for treatment of thyroid cancer (110 frequencies so far included):

| | | |
|---|---|---|
| 493,442 Hz | 3475,216 Hz | 7534,221 Hz |
| 517,202 Hz | 3509,522 Hz | 7623,184 Hz |
| 618,927 Hz | 3533,328 Hz | 7725,339 Hz |
| 621,321 Hz | 3637,085 Hz | 7920,879 Hz |
| 648,252 Hz | 3682,489 Hz | 8013,953 Hz |
| 663,407 Hz | 4154,301 Hz | 8019,912 Hz |
| 821,202 Hz | 4243,393 Hz | 8040,231 Hz |
| 874,341 Hz | 4261,228 Hz | 8078,955 Hz |
| 914,429 Hz | 4330,289 Hz | 8082,173 Hz |
| 941,311 Hz | 4340,833 Hz | 8147,1 Hz |
| 983,429 Hz | 4358,333 Hz | 8281,259 Hz |
| 1587,811 Hz | 4366,294 Hz | 8309,752 Hz |
| 1723,389 Hz | 4426,387 Hz | 8311,371 Hz |
| 2179,231 Hz | 4458,339 Hz | 8435,094 Hz |
| 2315,888 Hz | 4479,113 Hz | 8525,789 Hz |
| 2341,312 Hz | 4744,424 Hz | 8744,527 Hz |
| 2445,123 Hz | 4865,421 Hz | 9009,329 Hz |
| 2454,232 Hz | 5323,192 Hz | 9070,809 Hz |
| 2723,302 Hz | 5324,123 Hz | 10020,521 Hz |
| 2740,384 Hz | 5548,879 Hz | 10039,109 Hz |
| 2749,323 Hz | 5711,283 Hz | 10127,279 Hz |
| 2856,253 Hz | 5754,332 Hz | 10134,161 Hz |
| 2859,495 Hz | 6455,131 Hz | 10257,324 Hz |
| 2886,232 Hz | 6620,132 Hz | 10498,339 Hz |
| 3021,122 Hz | 6666,839 Hz | 11537,292 Hz |
| 3078,275 Hz | 6714,189 Hz | 11559,292 Hz |
| 3080,592 Hz | 6745,333 Hz | 11913,222 Hz |
| 3198,323 Hz | 6766,281 Hz | 11927,934 Hz |
| 3248,321 Hz | 6884,432 Hz | 11955,949 Hz |
| 3271,329 Hz | 7036,122 Hz | 12120,049 Hz |
| 3284,192 Hz | 7230,838 Hz | 12139,222 Hz |
| 3335,332 Hz | 7323,209 Hz | 13636,082 Hz |
| 3434,911 Hz | 7355,378 Hz | 13654,272 Hz |
| 3440,212 Hz | 7432,143 Hz | 13677,211 Hz |
| 14014,941 Hz | 16048,391 Hz | 17881,709 Hz |
| 14445,214 Hz | 17323,196 Hz | 17911,323 Hz |
| 16023,119 Hz | 17577,221 Hz | |

### Example 7. AM Frequencies employed for treatment of bladder cancer (28 frequencies so far included):

| | | |
|---|---|---|
| 623,243 Hz | 3438,109 Hz | 8235,21 Hz |
| 757,084 Hz | 3692,319 Hz | 8749,232 Hz |
| 870,4 Hz | 3952,308 Hz | 9354,812 Hz |
| 2454,423 Hz | 5230,227 Hz | 12532,729 Hz |
| 2480,191 Hz | 6022,942 Hz | 13467,209 Hz |
| 2581,101 Hz | 6061,711 Hz | 13777,9 Hz |
| 2715,232 Hz | 6710,899 Hz | 14015,241 Hz |
| 3042,012 Hz | 6721,912 Hz | 18524,419 Hz |
| 3196,194 Hz | 7181,784 Hz | |
| 3265,323 Hz | 7458,209 Hz | |

### Example 8. AM Frequencies employed for treatment of colon cancer (100 frequencies so far included):

| | | |
|---|---|---|
| 78,76 Hz | 3373,892 Hz | 5386,212 Hz |
| 796,562 Hz | 3390,925 Hz | 5407,192 Hz |
| 841,541 Hz | 3409,179 Hz | 5426,323 Hz |
| 842,783 Hz | 3432,274 Hz | 5496,434 Hz |
| 914,429 Hz | 3509,522 Hz | 5555,212 Hz |
| 1162,117 Hz | 3531,422 Hz | 5572,032 Hz |
| 1372,207 Hz | 3533,328 Hz | 5634,933 Hz |
| 1372,934 Hz | 3766,296 Hz | 5724,231 Hz |
| 1718,532 Hz | 4040,839 Hz | 5758,378 Hz |
| 2243,169 Hz | 4081,022 Hz | 5787,342 Hz |
| 2278,312 Hz | 4123,953 Hz | 5948,897 Hz |
| 2286,5 Hz | 4146,274 Hz | 5967,448 Hz |
| 2286,519 Hz | 4233,822 Hz | 5976,825 Hz |
| 2334,178 Hz | 4282,332 Hz | 6182,322 Hz |
| 2423,292 Hz | 4318,222 Hz | 6292,379 Hz |
| 2454,423 Hz | 4344,082 Hz | 6324,493 Hz |
| 2464,229 Hz | 4416,221 Hz | 6341,248 Hz |
| 2598,853 Hz | 4481,242 Hz | 6471,322 Hz |
| 2623,048 Hz | 4724,263 Hz | 6477,218 Hz |
| 3131,123 Hz | 4751,319 Hz | 6558,342 Hz |
| 3161,465 Hz | 4755,323 Hz | 6855,286 Hz |
| 3175,313 Hz | 4788,485 Hz | 7129,843 Hz |
| 3249,529 Hz | 5149,331 Hz | 7140,187 Hz |
| 3363,229 Hz | 5217,402 Hz | 7162,422 Hz |
| 7368,222 Hz | 8568,033 Hz | 11220,222 Hz |
| 7645,859 Hz | 8573,122 Hz | 11283,378 Hz |
| 7829,234 Hz | 9226,222 Hz | 12256,432 Hz |
| 7866,229 Hz | 9351,9 Hz | 13749,858 Hz |
| 7877,334 Hz | 9737,211 Hz | 15231,548 Hz |
| 8013,314 Hz | 9744,193 Hz | 15248,324 Hz |
| 8374,942 Hz | 9942,321 Hz | 58191,928 Hz |
| 8384,228 Hz | 10301,371 Hz | 60317,352 Hz |
| 8408,121 Hz | 10401,515 Hz | |
| 8534,111 Hz | 10872,693 Hz | |

### Example 9. AM Frequencies employed for treatment of pancreas cancer (166 frequencies so far included):

| | | |
|---|---|---|
| 331,3 Hz | 2477,919 Hz | 4056,384 Hz |
| 331,365 Hz | 2542,221 Hz | 4085,971 Hz |
| 436,3 Hz | 2598,853 Hz | 4144,592 Hz |
| 436,332 Hz | 2647,938 Hz | 4153,192 Hz |
| 447,942 Hz | 2685,081 Hz | 4161,889 Hz |
| 476,127 Hz | 2716,095 Hz | 4243,393 Hz |
| 559,292 Hz | 2721,331 Hz | 4332,498 Hz |
| 589,187 Hz | 2732,231 Hz | 4341,423 Hz |
| 624,218 Hz | 2809,849 Hz | 4355,327 Hz |
| 727 Hz | 2823,428 Hz | 4417,885 Hz |
| 734,921 Hz | 2835,332 Hz | 4422,322 Hz |
| 809,313 Hz | 3134,313 Hz | 4451,297 Hz |
| 845,309 Hz | 3241,461 Hz | 4486,384 Hz |
| 870,4 Hz | 3255,219 Hz | 4558,306 Hz |
| 963,221 Hz | 3263,432 Hz | 4580 Hz |
| 1156,79 Hz | 3286,255 Hz | 4685,082 Hz |
| 1157 Hz | 3330,935 Hz | 4839,589 Hz |
| 1179 Hz | 3373,892 Hz | 5151,402 Hz |
| 1360,133 Hz | 3438,109 Hz | 5209,911 Hz |
| 1372,207 Hz | 3449,219 Hz | 5262,282 Hz |
| 1372,934 Hz | 3535,219 Hz | 5271,312 Hz |
| 1804,126 Hz | 3549,215 Hz | 5387,73 Hz |
| 1816,221 Hz | 3564,419 Hz | 5494,928 Hz |
| 1873,477 Hz | 3619,412 Hz | 5521,221 Hz |
| 1967,211 Hz | 3622,312 Hz | 5573,209 Hz |
| 1990,482 Hz | 3638,432 Hz | 5609,382 Hz |
| 2278,312 Hz | 3696,424 Hz | 5929,616 Hz |
| 2315,921 Hz | 3943,214 Hz | 5948,897 Hz |
| 2320,315 Hz | 3976,929 Hz | 5966,112 Hz |
| 2334,178 Hz | 4014,889 Hz | 5976,825 Hz |
| 2381,443 Hz | 4041,219 Hz | 6064,197 Hz |
| 2469 Hz | 4044,195 Hz | 6086,256 Hz |
| 6157,253 Hz | 7985,122 Hz | 10528,239 Hz |
| 6215,298 Hz | 8008,323 Hz | 10582,095 Hz |
| 6333,917 Hz | 8013,312 Hz | 10926,111 Hz |
| 6365,242 Hz | 8045,484 Hz | 10948,411 Hz |
| 6558,342 Hz | 8242,332 Hz | 10955,558 Hz |
| 6568,278 Hz | 8351,622 Hz | 11538,193 Hz |
| 6823,194 Hz | 8408,121 Hz | 11904,741 Hz |
| 6853,391 Hz | 8455,894 Hz | 12255,229 Hz |
| 6855,286 Hz | 8551,231 Hz | 12613,341 Hz |
| 7213,204 Hz | 8743,321 Hz | 12819,942 Hz |
| 7228,528 Hz | 8789,631 Hz | 13674,482 Hz |
| 7238,232 Hz | 8868,809 Hz | 13731,322 Hz |
| 7277,921 Hz | 9012,241 Hz | 14525,312 Hz |
| 7280,422 Hz | 9028,994 Hz | 14537,218 Hz |
| 7320,494 Hz | 9131,232 Hz | 14549,331 Hz |
| 7366,412 Hz | 9658,296 Hz | 14845,453 Hz |
| 7534,221 Hz | 9663,495 Hz | 14944,989 Hz |
| 7548,713 Hz | 9680,737 Hz | 15246,315 Hz |
| 7567,127 Hz | 9824,442 Hz | 18668,239 Hz |
| 7620,851 Hz | 9942,321 Hz | 19321,231 Hz |
| 7663,209 Hz | 10279,122 Hz | 19347,208 Hz |
| 7725,203 Hz | 10388,49 Hz | 30182,932 Hz |
| 7852,233 Hz | 10438,495 Hz | |
| 7920,879 Hz | 10518,311 Hz | |

### Example 10. AM Frequencies employed for treatment of lung cancer (80 frequencies so far included):

| | | |
|---|---|---|
| 304,148 Hz | 3128,822 Hz | 4378,321 Hz |
| 694,7 Hz | 3139,297 Hz | 4416,221 Hz |
| 694,727 Hz | 3193,212 Hz | 4481,242 Hz |
| 708,8 Hz | 3348,783 Hz | 4777,521 Hz |
| 708,841 Hz | 3360,971 Hz | 4798,422 Hz |
| 1587,811 Hz | 3366,311 Hz | 4837,241 Hz |
| 1759,318 Hz | 3373,892 Hz | 4959,842 Hz |
| 1873,477 Hz | 3440,212 Hz | 5013,321 Hz |
| 2253,704 Hz | 3461,322 Hz | 5047,523 Hz |
| 2391,312 Hz | 3682,489 Hz | 5068,322 Hz |
| 2454,232 Hz | 3727,231 Hz | 5371,922 Hz |
| 2729,929 Hz | 3749,882 Hz | 5538,432 Hz |
| 2741,261 Hz | 3769,942 Hz | 5548,879 Hz |
| 2761,312 Hz | 4131,235 Hz | 5679,309 Hz |
| 2784,491 Hz | 4158,393 Hz | 5734,143 Hz |
| 2812,443 Hz | 4243,393 Hz | 5787,342 Hz |
| 2855,218 Hz | 4347,733 Hz | 6445,309 Hz |
| 2859,495 Hz | 4373,411 Hz | 6838,434 Hz |
| 6870,955 Hz | 8442,473 Hz | 10424,908 Hz |
| 6879,216 Hz | 8773,916 Hz | 10452,913 Hz |
| 7079,411 Hz | 8935,752 Hz | 10824,609 Hz |
| 7216,288 Hz | 9121,223 Hz | 11656,329 Hz |
| 7376,089 Hz | 9181,434 Hz | 12748,919 Hz |
| 7761,289 Hz | 9317,913 Hz | 15774,291 Hz |
| 8082,173 Hz | 9363,896 Hz | 15798,333 Hz |
| 8281,259 Hz | 9736,919 Hz | 16510,321 Hz |
| 8352,189 Hz | 9753,321 Hz | |

### Example 11. AM Frequencies employed for treatment of leiomyosarcoma (36 frequencies so far included):

| | | |
|---|---|---|
| 836,923 Hz | 4241,321 Hz | 6651,276 Hz |
| 843,181 Hz | 4266,591 Hz | 7168,892 Hz |
| 1411,241 Hz | 4337,322 Hz | 7406,309 Hz |
| 2073,721 Hz | 4424,112 Hz | 7452,528 Hz |
| 2381,443 Hz | 4436,111 Hz | 7649,209 Hz |
| 2711,019 Hz | 4485,22 Hz | 7808,352 Hz |
| 2911,329 Hz | 5545,521 Hz | 9040,313 Hz |
| 3232,185 Hz | 5577,841 Hz | 9074,294 Hz |
| 3518,321 Hz | 5631,422 Hz | 9189,092 Hz |
| 3544,209 Hz | 5696,184 Hz | 9484,512 Hz |
| 3569,219 Hz | 6472,098 Hz | 9943,972 Hz |
| 4233,822 Hz | 6558,342 Hz | 12086,394 Hz |

### Example 12. AM Frequencies employed for treatment of mesothelioma (16 frequencies so far included):

| | | |
|---|---|---|
| 958,929 Hz | 3319,945 Hz | 6516,793 Hz |
| 1713,913 Hz | 3449,219 Hz | 7224,197 Hz |
| 1736,782 Hz | 3622,312 Hz | 9471,152 Hz |
| 2334,178 Hz | 5151,402 Hz | 14617,393 Hz |
| 2607,193 Hz | 5887,022 Hz | |
| 3112,974 Hz | 5965,922 Hz | |

### Example 13. AM Frequencies employed for treatment of neuro-endocrine (30 frequencies so far included):

| | | |
|---|---|---|
| 1766,335 Hz | 2741,261 Hz | 3296,431 Hz |
| 2408,225 Hz | 3020,212 Hz | 3348,783 Hz |
| 2441,502 Hz | 3128,822 Hz | 3360,971 Hz |
| 2647,938 Hz | 3238,742 Hz | 3440,212 Hz |
| 3533,328 Hz | 5548,879 Hz | 7482,245 Hz |
| 3666,283 Hz | 5739,422 Hz | 7575,393 Hz |
| 4079,282 Hz | 5849,241 Hz | 8359,932 Hz |
| 4243,393 Hz | 6291,631 Hz | 9073,418 Hz |
| 4426,387 Hz | 6406,891 Hz | |
| 5245,818 Hz | 6780,679 Hz | |
| 5536,242 Hz | 7151,264 Hz | |

### Example 14. AM Frequencies employed for treatment of leukemia and chronic lymphoid cancer (17 frequencies so far included):

| | | |
|---|---|---|
| 814,413 Hz | 3361,671 Hz | 7629,318 Hz |
| 825,145 Hz | 5245,452 Hz | 8172,405 Hz |
| 2415,243 Hz | 5557,333 Hz | 8272,338 Hz |
| 2436,316 Hz | 6850,197 Hz | 8438,453 Hz |
| 2874,432 Hz | 6919,322 Hz | 12950,331 Hz |
| 2891,029 Hz | 7587,224 Hz | |

### Example 15. AM Frequencies employed for treatment of myeloma, multiple cancer (20 frequencies so far included):

| | | |
|---|---|---|
| 765,196 Hz | 2883,618 Hz | 5249,331 Hz |
| 2336,238 Hz | 2919,273 Hz | 7967,311 Hz |
| 2372,122 Hz | 3265,323 Hz | 7973,125 Hz |
| 2381,443 Hz | 3564,455 Hz | 8049,952 Hz |
| 2425,394 Hz | 3580.25 Hz | 8283,329 Hz |
| 2656,339 Hz | 3584,291 Hz | 10351,323 Hz |
| 2741,261 Hz | 3674,292 Hz | |

### Example 16. AM Frequencies employed for treatment of Hodgkin disease (lymphoma) (19 frequencies so far included):

| | | |
|---|---|---|
| 752,5 Hz | 3371,216 Hz | 5724,231 Hz |
| 976,3 Hz | 3605,432 Hz | 6358,194 Hz |
| 1558,223 Hz | 3623,198 Hz | 7472,211 Hz |
| 2310,912 Hz | 3838,281 Hz | 8062,121 Hz |
| 2477,919 Hz | 3838,48 Hz | 8222,222 Hz |
| 2560,843 Hz | 5102 Hz | |
| 3348,783 Hz | 5696,932 Hz | |

### Example 17. AM Frequencies employed for treatment of brain cancer (57 frequencies so far included):

| | | |
|---|---|---|
| 1372,934 Hz | 4318,222 Hz | 6943,386 Hz |
| 2318,182 Hz | 4334,33 Hz | 7151,264 Hz |
| 2381,443 Hz | 4358,333 Hz | 7182,922 Hz |
| 2425,394 Hz | 4393,419 Hz | 7194,897 Hz |
| 2442,423 Hz | 4454,194 Hz | 7323,209 Hz |
| 2478,973 Hz | 4515,789 Hz | 7390,343 Hz |
| 2654,513 Hz | 4619,324 Hz | 7796,221 Hz |
| 2661,324 Hz | 4723,937 Hz | 7961,122 Hz |
| 2686,105 Hz | 4853,286 Hz | 8128,942 Hz |
| 2690,179 Hz | 5289,231 Hz | 8245,109 Hz |
| 3249,332 Hz | 5378,099 Hz | 8272,281 Hz |
| 3277,509 Hz | 5426,323 Hz | 8358,154 Hz |
| 3335,279 Hz | 5640,981 Hz | 8408,121 Hz |
| 3348,783 Hz | 6316,211 Hz | 9138,82 Hz |
| 3436,211 Hz | 6459,203 Hz | 10719,318 Hz |
| 3916,321 Hz | 6474,332 Hz | 11556,241 Hz |
| 4031,933 Hz | 6626,572 Hz | 12828,633 Hz |
| 4086,091 Hz | 6855,286 Hz | 14515,962 Hz |
| 4241,321 Hz | 6915,886 Hz | 14586,765 Hz |

The above Examples reflect AM frequencies determined by a biofeedback procedure involving very substantial observations and measurements of physiological responses (at certain well defined AM frequencies) by subjects exposed to low energy electromagnetic emission excitation. In general, it is recommended that all of the listed frequencies be applied in the treatment of subjects suffering from the indicated form of cancer. However, a limited number of the listed frequencies also lead to beneficial effects.

Of note in respect of the above listed frequencies, in particular those Examples including a large number of frequencies, it has earlier on been determined that beneficial therapeutic effects are achieved by application of some but not all of the frequencies listed. However, following on more extended trials, it has been determined that application to subjects of further frequencies enhance the efficacy of treatment and yields therapeutic effects in patients whose tumours have become resistant to therapy. It is accordingly preferred that all of the determined listed frequencies be applied to the subject. The mechanism of including additional frequencies is attributed to either or both of inter-active synergism between applied frequencies or between cells which have been influenced by the treatment and additive effects of the additional frequencies.

Of further note is the fact that different patients suffering from the same type of tumour cell growth practically invariably exhibit the above-mentioned physiological responses at the same well defined AM frequencies. Furthermore, AM frequencies which differ only very slightly (less than 0.0001 % at higher frequencies) from the frequencies listed, in general elicit no physiological response by subjects exposed to excitation at such very slightly different frequency. In view of these determinations, the electronic system of the present invention may be adapted to screen a subject for physiological responses over a broad range of frequencies to determine the presence or absence tumour cells and, if positive, then to note at which defined frequencies physiological responses are elicited. These frequencies will in general match with the defined frequencies listed in one or other of the Examples above or such further examples as may be developed and hence the nature of the tumour will be known. The electronic system of the invention is therefore a valuable diagnostic tool for diagnosing the presence or absence and identities of types of tumour cell growths or cancers. Furthermore, the electronic system of the invention is of value for predicting whether a patient will benefit from the application of a given series of modulation frequencies. The system therefore possesses a capability of predicting responses to treatment, thereby enhancing the possibility to select optimal modes of treatment.

The sequence of well defined frequencies are preferably applied sequentially for determined periods of time, e.g. 3 seconds for each frequency, but several frequencies may also be applied simultaneously. This means that a cycle of application involving 180 frequencies would take nearly 10 minutes time. Advantageous effects may however also arise from applying individual well defined frequencies for differing time periods, e.g. some for 3 seconds, some for 6 seconds, etc...

Therapeutic dosages to be applied to a subject suffering from the presence of tumour cell growth or cancer are determined by the time of application of the low energy electromagnetic emissions to the subject and will depend on the nature of the cancer and the overall condition of the subject. In general, however, greatest experience has been gained in treating terminally ill subjects expected to survive no longer than about three months and who have agreed to discontinue alternative forms of cancer treatments such as chemo-therapy or radioactive treatment. In these severe cases, lengthy times of treatment are recommended, e.g. 3 times 1 hour daily treatment. However, with the development of alternative forms of application, i.e. other than by means of a mouth probe, continuous application is possible and is likely to enhance compliance and the efficacy of the treatment.

## Claims

1. An electronic system (11) activatable by electrical power for use in inhibiting cancerous cell growth or proliferation thereof in a warm-blooded mammalian subject, comprising:
one or more controllable low energy electromagnetic energy generator circuits (29) for generating one or more high frequency carrier signals,
one or more data processors or integrated circuits (21) comprising or communicating with the one or more generator circuits (29) and being for receiving control information from a source of control information being an application storage device or computer (52),
said one or more generator circuits (29) including one or more amplitude modulation control signal generators (34) for controlling amplitude modulated variations of the one or more high frequency carrier signals,
said one or more generator circuits (29) furthermore including one or more programmable amplitude modulation frequency control signal generators (31) for controlling the frequency at which the amplitude modulations are generated,
the system (11) furthermore comprising a connection or coupling position (12a) for connection to an electrically conductive applicator (12,13),
said one or more programmable amplitude modulation frequency control generators (31) are adapted to accurately control the frequency of amplitude modulations to within an accuracy of at least 1000 ppm relative to one or more determined or predetermined reference amplitude modulation frequencies selected from within a range of 0.01 Hz to 150 kHz
**and characterised in that**
said source of control information (52) includes reference amplitude modulation frequency control information which comprises at least a 50% of the accurately defined reference amplitude modulation frequencies listed in any of the items 1 to 17 below,
the applicator (12, 13) being adapted to apply by contact or close-by induction to the warm-blooded mammalian subject one or more amplitude-modulated low energy emissions at a program-controlled frequency resulting in low levels of absorption, substantially less than 1.6 milliW/g weight of living tissue,
and wherein the amplitude is modulated by sequentially or simultaneously applying the at least 50% of the reference amplitude modulation frequencies.
**1. AM Frequencies employed for treatment of breast cancer**
| | | |
|---|---|---|
| 78,76 Hz | 3115,188 Hz | 4969,224 Hz |
| 181,821 Hz | 3249,529 Hz | 4979,321 Hz |
| 414,817 Hz | 3405,182 Hz | 5027,231 Hz |
| 440,933 Hz | 3432,274 Hz | 5059,792 Hz |
| 628,431 Hz | 3434,693 Hz | 5118,094 Hz |
| 721,313 Hz | 3594,231 Hz | 5176,287 Hz |
| 813,205 Hz | 3647,619 Hz | 5365,222 Hz |
| 818,342 Hz | 3742,957 Hz | 5376,392 Hz |
| 891,901 Hz | 3753,382 Hz | 5426,323 Hz |
| 929,095 Hz | 3830,732 Hz | 5431,542 Hz |
| 929,1 Hz | 3855,823 Hz | 5521,621 Hz |
| 1021 Hz | 3916,321 Hz | 5739,422 Hz |
| 1372,207 Hz | 3935,218 Hz | 5745,218 Hz |
| 1372,934 Hz | 3975,383 Hz | 5821,975 Hz |
| 1588,721 Hz | 3993,437 Hz | 6037,432 Hz |
| 1670,699 Hz | 4153,192 Hz | 6044,333 Hz |
| 1821,729 Hz | 4194,968 Hz | 6086,256 Hz |
| 1836,219 Hz | 4241,321 Hz | 6208,932 Hz |
| 2193,937 Hz | 4243,393 Hz | 6212,808 Hz |
| 2221,323 Hz | 4253,432 Hz | 6231,031 Hz |
| 2278,312 Hz | 4314,444 Hz | 6280,321 Hz |
| 2357,832 Hz | 4318,222 Hz | 6329,391 Hz |
| 2381,443 Hz | 4375,962 Hz | 6476,896 Hz |
| 2417,323 Hz | 4393,419 Hz | 6497,319 Hz |
| 2431,334 Hz | 4417,243 Hz | 6504,983 Hz |
| 2450,332 Hz | 4481,463 Hz | 6651,276 Hz |
| 2551,313 Hz | 4482,223 Hz | 6757,901 Hz |
| 2556,221 Hz | 4495,138 Hz | 6758,321 Hz |
| 2598,853 Hz | 4549,808 Hz | 6855,286 Hz |
| 2621,322 Hz | 4558,306 Hz | 6858,121 Hz |
| 2740,191 Hz | 4779,451 Hz | 6898,489 Hz |
| 2851,347 Hz | 4838,674 Hz | 7092,219 Hz |
| 2885,322 Hz | 4871,513 Hz | 7120,218 Hz |
| 2919,273 Hz | 4895,296 Hz | 7127,311 Hz |
| 3074,333 Hz | 4962,213 Hz | 7156,489 Hz |
| 7208,821 Hz | 8548,324 Hz | 11525,121 Hz |
| 7282,169 Hz | 8749,383 Hz | 11541,915 Hz |
| 7376,329 Hz | 8782,421 Hz | 11812,328 Hz |
| 7488,742 Hz | 8784,424 Hz | 11812,419 Hz |
| 7541,319 Hz | 8923,1 Hz | 11840,323 Hz |
| 7577,421 Hz | 8923,361 Hz | 11925,089 Hz |
| 7621,085 Hz | 8935,752 Hz | 12123,281 Hz |
| 7627,207 Hz | 8936,1 Hz | 12267,281 Hz |
| 7650,939 Hz | 9012,282 Hz | 12294,283 Hz |
| 7691,212 Hz | 9012,896 Hz | 12611,288 Hz |
| 7842,184 Hz | 9060,323 Hz | 12629,222 Hz |
| 7849,231 Hz | 9072,409 Hz | 12633,372 Hz |
| 7915,423 Hz | 9131,419 Hz | 12648,221 Hz |
| 7932,482 Hz | 9199,232 Hz | 13315,335 Hz |
| 7949,196 Hz | 9245,927 Hz | 13331,358 Hz |
| 7967,311 Hz | 9270,322 Hz | 13735,241 Hz |
| 8021,229 Hz | 9279,193 Hz | 13826,325 Hz |
| 8070,181 Hz | 9393,946 Hz | 13853,232 Hz |
| 8114,032 Hz | 10227,242 Hz | 13990,123 Hz |
| 8149,922 Hz | 10340,509 Hz | 14122,942 Hz |
| 8194,19 Hz | 10363,313 Hz | 14162,332 Hz |
| 8245,801 Hz | 10449,323 Hz | 14519,232 Hz |
| 8328,322 Hz | 10456,383 Hz | 14543,128 Hz |
| 8330,534 Hz | 10468,231 Hz | 15651,323 Hz |
| 8355,987 Hz | 10470,456 Hz | 17352,085 Hz |
| 8408,121 Hz | 10472,291 Hz | 18785,463 Hz |
| 8431,184 Hz | 10689,339 Hz | 30182,932 Hz |
| 8452,119 Hz | 10832,222 Hz | |
**2. AM Frequencies employed for treatment of liver cancer**
| | | |
|---|---|---|
| 423,321 Hz | 1250,504 Hz | 2353,478 Hz |
| 427,062 Hz | 1755,402 Hz | 2362,309 Hz |
| 470,181 Hz | 1873,477 Hz | 2419,309 Hz |
| 560,32 Hz | 1924,702 Hz | 2425,222 Hz |
| 642,932 Hz | 1975,196 Hz | 2430,219 Hz |
| 668,209 Hz | 2017,962 Hz | 2431,094 Hz |
| 677,972 Hz | 2083,419 Hz | 2471,328 Hz |
| 811,924 Hz | 2190,731 Hz | 2478,331 Hz |
| 842,311 Hz | 2221,323 Hz | 2743,995 Hz |
| 843,22 Hz | 2324,393 Hz | 2744,211 Hz |
| 2831,951 Hz | 5520,218 Hz | 8542,311 Hz |
| 2843,283 Hz | 5882,292 Hz | 8818,104 Hz |
| 2859,891 Hz | 5926,512 Hz | 8852,329 Hz |
| 2873,542 Hz | 6037,311 Hz | 8853,444 Hz |
| 2886,232 Hz | 6180,334 Hz | 8858,179 Hz |
| 3042,012 Hz | 6329,195 Hz | 8939,212 Hz |
| 3078,983 Hz | 6350,333 Hz | 9332,397 Hz |
| 3086,443 Hz | 6361,321 Hz | 9381,221 Hz |
| 3127,232 Hz | 6364,928 Hz | 9740,219 Hz |
| 3160,942 Hz | 6383,321 Hz | 9768,331 Hz |
| 3206,315 Hz | 6461,175 Hz | 9797,294 Hz |
| 3267,433 Hz | 6733,331 Hz | 10317,499 Hz |
| 3269,321 Hz | 6758,232 Hz | 10443,311 Hz |
| 3457,291 Hz | 6779,482 Hz | 10456,383 Hz |
| 3505,229 Hz | 6856,222 Hz | 10579,425 Hz |
| 3516,296 Hz | 6877,183 Hz | 10863,209 Hz |
| 3531,296 Hz | 6980,525 Hz | 10866,382 Hz |
| 3546,323 Hz | 7019,235 Hz | 11067,418 Hz |
| 3572,106 Hz | 7043,209 Hz | 11149,935 Hz |
| 3576,189 Hz | 7130,323 Hz | 11163,895 Hz |
| 3669,513 Hz | 7144,142 Hz | 11802,821 Hz |
| 3923,221 Hz | 7210,223 Hz | 11953,424 Hz |
| 4013,932 Hz | 7291,21 Hz | 12223,329 Hz |
| 4071,121 Hz | 7510,92 Hz | 12265,295 Hz |
| 4079,951 Hz | 7529,233 Hz | 12267,233 Hz |
| 4222,821 Hz | 7549,212 Hz | 12623,191 Hz |
| 4238,402 Hz | 7650,028 Hz | 12685,231 Hz |
| 4256,321 Hz | 7680,518 Hz | 12721,423 Hz |
| 4289,296 Hz | 7692,522 Hz | 12785,342 Hz |
| 4312,947 Hz | 7829,231 Hz | 14085,222 Hz |
| 4435,219 Hz | 7862,209 Hz | 14333,209 Hz |
| 4471,188 Hz | 7947,392 Hz | 14537,331 Hz |
| 4483,889 Hz | 7979,308 Hz | 14542,432 Hz |
| 4486,384 Hz | 8028,339 Hz | 14655,03 Hz |
| 4629,941 Hz | 8055,942 Hz | 14828,234 Hz |
| 4732,211 Hz | 8072,134 Hz | 15149,213 Hz |
| 4876,218 Hz | 8141,174 Hz | 15237,489 Hz |
| 5086,281 Hz | 8336,383 Hz | 16110,932 Hz |
| 5124,084 Hz | 8432,181 Hz | 16144,343 Hz |
| 5133,121 Hz | 8452,119 Hz | 18265,238 Hz |
| 5247,142 Hz | 8460,944 Hz | 18283,323 Hz |
| 5270,834 Hz | 8475,221 Hz | 18863,292 Hz |
| 5340,497 Hz | 8492,193 Hz | 18930,995 Hz |
| 19970,311 Hz | 20330,294 Hz | 20365,284 Hz |
**3. AM Frequencies employed for treatment of ovarian cancer**
| | | |
|---|---|---|
| 78,76 Hz | 1372,207 Hz | 2812,321 Hz |
| 181,821 Hz | 1396,498 Hz | 2831,386 Hz |
| 410,245 Hz | 1502,181 Hz | 2835,332 Hz |
| 414,817 Hz | 1518,208 Hz | 2851,347 Hz |
| 436,332 Hz | 1552,123 Hz | 2877,192 Hz |
| 447,942 Hz | 1579,212 Hz | 2885,322 Hz |
| 481,191 Hz | 1624,802 Hz | 2887,385 Hz |
| 489,292 Hz | 1670,699 Hz | 2894,972 Hz |
| 559,292 Hz | 1696,403 Hz | 2973,771 Hz |
| 608,321 Hz | 1762,938 Hz | 3080,592 Hz |
| 655,435 Hz | 1771,402 Hz | 3157,483 Hz |
| 657,397 Hz | 1775,313 Hz | 3161,465 Hz |
| 657,483 Hz | 1821,729 Hz | 3223,232 Hz |
| 664,211 Hz | 2016,323 Hz | 3238,148 Hz |
| 708,8 Hz | 2034,231 Hz | 3249,529 Hz |
| 708,822 Hz | 2050,282 Hz | 3262,145 Hz |
| 734,921 Hz | 2053,396 Hz | 3314,321 Hz |
| 749,221 Hz | 2082,234 Hz | 3361,671 Hz |
| 764,232 Hz | 2089,092 Hz | 3366,311 Hz |
| 778,295 Hz | 2221,323 Hz | 3523,215 Hz |
| 779,403 Hz | 2228,832 Hz | 3527,233 Hz |
| 806,021 Hz | 2253,704 Hz | 3542,213 Hz |
| 806,389 Hz | 2254,329 Hz | 3590,376 Hz |
| 809,313 Hz | 2278,312 Hz | 3629,232 Hz |
| 824,327 Hz | 2332,949 Hz | 3632,793 Hz |
| 825,145 Hz | 2348,233 Hz | 3636,289 Hz |
| 835,129 Hz | 2381,443 Hz | 3637,085 Hz |
| 839,521 Hz | 2413,193 Hz | 3669,513 Hz |
| 841,208 Hz | 2425,222 Hz | 3770,189 Hz |
| 843,312 Hz | 2433,321 Hz | 3858,916 Hz |
| 956,984 Hz | 2439,253 Hz | 3919,232 Hz |
| 958,929 Hz | 2465,23 Hz | 3957,185 Hz |
| 985,313 Hz | 2477,919 Hz | 3975,228 Hz |
| 1024,208 Hz | 2669,177 Hz | 4061,131 Hz |
| 1102,635 Hz | 2715,232 Hz | 4072,322 Hz |
| 1121,329 Hz | 2733,843 Hz | 4169,451 Hz |
| 1159,738 Hz | 2802,339 Hz | 4174,259 Hz |
| 4241,321 Hz | 6350,333 Hz | 8428,313 Hz |
| 4243,393 Hz | 6406,891 Hz | 8435,451 Hz |
| 4261,228 Hz | 6407,207 Hz | 8486,421 Hz |
| 4279,113 Hz | 6450,787 Hz | 8492,797 Hz |
| 4309,335 Hz | 6477,098 Hz | 8548,324 Hz |
| 4314,188 Hz | 6477,929 Hz | 8554,361 Hz |
| 4318,222 Hz | 6478,338 Hz | 8562,965 Hz |
| 4328,928 Hz | 6543,421 Hz | 8579,323 Hz |
| 4380,321 Hz | 6552,24 Hz | 8579,333 Hz |
| 4394,134 Hz | 6663,955 Hz | 8642,181 Hz |
| 4412,252 Hz | 6753,338 Hz | 8655,818 Hz |
| 4424,236 Hz | 6851,323 Hz | 8758,341 Hz |
| 4439,341 Hz | 6855,286 Hz | 8779,323 Hz |
| 4442,161 Hz | 6875,232 Hz | 8792,231 Hz |
| 4447,221 Hz | 6882,949 Hz | 8819,127 Hz |
| 4458,339 Hz | 7206,403 Hz | 8831,132 Hz |
| 4556,322 Hz | 7232,214 Hz | 9028,031 Hz |
| 4566,009 Hz | 7257,489 Hz | 9173,264 Hz |
| 4682,643 Hz | 7276,209 Hz | 9184,338 Hz |
| 4718,331 Hz | 7281,219 Hz | 9186,919 Hz |
| 4749,302 Hz | 7285,693 Hz | 9393,946 Hz |
| 4765,331 Hz | 7429,212 Hz | 9482,409 Hz |
| 4917,202 Hz | 7460,932 Hz | 9737,211 Hz |
| 5011,325 Hz | 7480,228 Hz | 9746,232 Hz |
| 5149,331 Hz | 7495,763 Hz | 9922,231 Hz |
| 5228,172 Hz | 7539,432 Hz | 10032,684 Hz |
| 5237,132 Hz | 7564,185 Hz | 10446,028 Hz |
| 5313,353 Hz | 7650,028 Hz | 10478,221 Hz |
| 5745,218 Hz | 7689,728 Hz | 10545,313 Hz |
| 5757,897 Hz | 7780,294 Hz | 10639,345 Hz |
| 5762,386 Hz | 8021,921 Hz | 10743,118 Hz |
| 5812,322 Hz | 8038,961 Hz | 10813,981 Hz |
| 5869,321 Hz | 8040,322 Hz | 10832,421 Hz |
| 5882,292 Hz | 8044,233 Hz | 10838,243 Hz |
| 5921,249 Hz | 8095,313 Hz | 10862,429 Hz |
| 5991,932 Hz | 8143,491 Hz | 10865,127 Hz |
| 6069,458 Hz | 8164,332 Hz | 10917,229 Hz |
| 6071,319 Hz | 8261,121 Hz | 10977,188 Hz |
| 6083,214 Hz | 8302,285 Hz | 11120,209 Hz |
| 6161,782 Hz | 8309,752 Hz | 11177,289 Hz |
| 6169,341 Hz | 8372,532 Hz | 11177,409 Hz |
| 6275,232 Hz | 8408,121 Hz | 11321,491 Hz |
| 6294,929 Hz | 8424,229 Hz | 11359,093 Hz |
| 11673,031 Hz | 12755,333 Hz | 15450,183 Hz |
| 11793,886 Hz | 12947,311 Hz | 16144,343 Hz |
| 11895,229 Hz | 13717,221 Hz | 17932,432 Hz |
| 12074,531 Hz | 13825,295 Hz | 17951,395 Hz |
| 12216,212 Hz | 13829,195 Hz | 17970,122 Hz |
| 12253,329 Hz | 14410,949 Hz | 18337,222 Hz |
| 12260,933 Hz | 14436,201 Hz | 18378,321 Hz |
| 12262,853 Hz | 14537,218 Hz | 18921,415 Hz |
| 12292,222 Hz | 14947,184 Hz | 18926,951 Hz |
| 12357,353 Hz | 15429,139 Hz | 18931,327 Hz |
| 12527,032 Hz | 15443,309 Hz | 114508,332 Hz |
**4. AM Frequencies employed for treatment of prostate cancer**
| | | |
|---|---|---|
| 331,3 Hz | 809,205 Hz | 2628,324 Hz |
| 331,358 Hz | 819,322 Hz | 2669,328 Hz |
| 403,218 Hz | 844,8 Hz | 2824,832 Hz |
| 461,233 Hz | 844,822 Hz | 2887,829 Hz |
| 522,2 Hz | 847,332 Hz | 2891,331 Hz |
| 522,213 Hz | 1083,309 Hz | 3081,523 Hz |
| 618,4 Hz | 1102,635 Hz | 3249,529 Hz |
| 618,407 Hz | 1102,71 Hz | 3250,125 Hz |
| 618,8 Hz | 1240,336 Hz | 3251,815 Hz |
| 656,295 Hz | 1372,934 Hz | 3264,827 Hz |
| 657,394 Hz | 1444,288 Hz | 3278,329 Hz |
| 657,397 Hz | 1486,322 Hz | 3281,432 Hz |
| 657,4 Hz | 1563,332 Hz | 3348,783 Hz |
| 657,483 Hz | 1591,322 Hz | 3519,118 Hz |
| 659,033 Hz | 1670,699 Hz | 3539,962 Hz |
| 694,4 Hz | 1697,321 Hz | 3551,318 Hz |
| 694,689 Hz | 1743,521 Hz | 3556,439 Hz |
| 694,7 Hz | 2031,448 Hz | 3572,321 Hz |
| 741,4 Hz | 2050,282 Hz | 3670,129 Hz |
| 741,421 Hz | 2076,519 Hz | 3681,341 Hz |
| 749,221 Hz | 2156,332 Hz | 3686,021 Hz |
| 752,9 Hz | 2229,515 Hz | 3753,382 Hz |
| 752,933 Hz | 2243,121 Hz | 3774,923 Hz |
| 776,194 Hz | 2381,443 Hz | 3867,692 Hz |
| 785,219 Hz | 2440,489 Hz | 3909,333 Hz |
| 786,332 Hz | 2475,912 Hz | 3916,321 Hz |
| 793,331 Hz | 2477,919 Hz | 4031,233 Hz |
| 4031,933 Hz | 6590,328 Hz | 9018,233 Hz |
| 4038,203 Hz | 6651,276 Hz | 9068,231 Hz |
| 4081,743 Hz | 6706,431 Hz | 9137,232 Hz |
| 4084,319 Hz | 6743,322 Hz | 9156,321 Hz |
| 4139,322 Hz | 6783,282 Hz | 9351,931 Hz |
| 4153,192 Hz | 6850,197 Hz | 9393,946 Hz |
| 4223,795 Hz | 6855,286 Hz | 9694,179 Hz |
| 4231,221 Hz | 6864,896 Hz | 9984,405 Hz |
| 4241,321 Hz | 6871,943 Hz | 10226,223 Hz |
| 4320,513 Hz | 6973,393 Hz | 10390,232 Hz |
| 4329,152 Hz | 7120,932 Hz | 10514,768 Hz |
| 4380,321 Hz | 7146,509 Hz | 10689,339 Hz |
| 4417,312 Hz | 7192,505 Hz | 10772,419 Hz |
| 4489,452 Hz | 7251,309 Hz | 10818,452 Hz |
| 4549,808 Hz | 7251,322 Hz | 11165,239 Hz |
| 4558,306 Hz | 7278,124 Hz | 11985,353 Hz |
| 4638,293 Hz | 7279,335 Hz | 12209,329 Hz |
| 4740,322 Hz | 7299,119 Hz | 12308,321 Hz |
| 4854,318 Hz | 7527,229 Hz | 12583,339 Hz |
| 4882,322 Hz | 7589,925 Hz | 13820,329 Hz |
| 4978,822 Hz | 7699,193 Hz | 14013,123 Hz |
| 5237,152 Hz | 7842,184 Hz | 14171,434 Hz |
| 5264,222 Hz | 8023,32 Hz | 14681,329 Hz |
| 5289,195 Hz | 8096,939 Hz | 14759,131 Hz |
| 5426,323 Hz | 8245,801 Hz | 14986,794 Hz |
| 5431,542 Hz | 8315,291 Hz | 15930,249 Hz |
| 5455,593 Hz | 8357,305 Hz | 16026,623 Hz |
| 6345,332 Hz | 8408,121 Hz | 17880,954 Hz |
| 6347,433 Hz | 8432,209 Hz | 18247,532 Hz |
| 6363,284 Hz | 8535,238 Hz | 18282,211 Hz |
| 6418,331 Hz | 8552,431 Hz | 18629,328 Hz |
| 6496,231 Hz | 8585,224 Hz | 19469,318 Hz |
| 6538,295 Hz | 8935,752 Hz | 19766,218 Hz |
| 6577,421 Hz | 9015,253 Hz | 60317,352 Hz |
**5. AM Frequencies employed for treatment of kidney cancer**
| | | |
|---|---|---|
| 628,321 Hz | 826,321 Hz | 2254,329 Hz |
| 631,141 Hz | 1372,934 Hz | 3555,209 Hz |
| 643,312 Hz | 2082,241 Hz | 3928,343 Hz |
| 812,512 Hz | 2156,931 Hz | 4420,932 Hz |
| 4819,228 Hz | 8727,224 Hz | 11421,933 Hz |
| 4828,321 Hz | 8760,983 Hz | 11523,212 Hz |
| 5314,322 Hz | 8831,132 Hz | 11561,221 Hz |
| 6007,332 Hz | 8870,228 Hz | 11846,212 Hz |
| 7054,279 Hz | 10565,321 Hz | 12631,331 Hz |
| 7074,429 Hz | 10586,229 Hz | 12693,272 Hz |
| 7254,343 Hz | 10634,293 Hz | 14411,321 Hz |
| 8041,289 Hz | 10687,949 Hz | 20178,941 Hz |
**6. AM Frequencies employed for treatment of thyroid cancer**
| | | |
|---|---|---|
| 493,442 Hz | 3284,192 Hz | 6745,333 Hz |
| 517,202 Hz | 3335,332 Hz | 6766,281 Hz |
| 618,927 Hz | 3434,911 Hz | 6884,432 Hz |
| 621,321 Hz | 3440,212 Hz | 7036,122 Hz |
| 648,252 Hz | 3475,216 Hz | 7230,838 Hz |
| 663,407 Hz | 3509,522 Hz | 7323,209 Hz |
| 821,202 Hz | 3533,328 Hz | 7355,378 Hz |
| 874,341 Hz | 3637,085 Hz | 7432,143 Hz |
| 914,429 Hz | 3682,489 Hz | 7534,221 Hz |
| 941,311 Hz | 4154,301 Hz | 7623,184 Hz |
| 983,429 Hz | 4243,393 Hz | 7725,339 Hz |
| 1587,811 Hz | 4261,228 Hz | 7920,879 Hz |
| 1723,389 Hz | 4330,289 Hz | 8013,953 Hz |
| 2179,231 Hz | 4340,833 Hz | 8019,912 Hz |
| 2315,888 Hz | 4358,333 Hz | 8040,231 Hz |
| 2341,312 Hz | 4366,294 Hz | 8078,955 Hz |
| 2445,123 Hz | 4426,387 Hz | 8082,173 Hz |
| 2454,232 Hz | 4458,339 Hz | 8147,1 Hz |
| 2723,302 Hz | 4479,113 Hz | 8281,259 Hz |
| 2740,384 Hz | 4744,424 Hz | 8309,752 Hz |
| 2749,323 Hz | 4865,421 Hz | 8311,371 Hz |
| 2856,253 Hz | 5323,192 Hz | 8435,094 Hz |
| 2859,495 Hz | 5324,123 Hz | 8525,789 Hz |
| 2886,232 Hz | 5548,879 Hz | 8744,527 Hz |
| 3021,122 Hz | 5711,283 Hz | 9009,329 Hz |
| 3078,275 Hz | 5754,332 Hz | 9070,809 Hz |
| 3080,592 Hz | 6455,131 Hz | 10020,521 Hz |
| 3198,323 Hz | 6620,132 Hz | 10039,109 Hz |
| 3248,321 Hz | 6666,839 Hz | 10127,279 Hz |
| 3271,329 Hz | 6714,189 Hz | 10134,161 Hz |
| 10257,324 Hz | 12120,049 Hz | 16023,119 Hz |
| 10498,339 Hz | 12139,222 Hz | 16048,391 Hz |
| 11537,292 Hz | 13636,082 Hz | 17323,196 Hz |
| 11559,292 Hz | 13654,272 Hz | 17577,221 Hz |
| 11913,222 Hz | 13677,211 Hz | 17881,709 Hz |
| 11927,934 Hz | 14014,941 Hz | 17911,323 Hz |
| 11955,949 Hz | 14445,214 Hz | |
**7. AM Frequencies employed for treatment of bladder cancer**
| | | |
|---|---|---|
| 623,243 Hz | 3438,109 Hz | 8235,21 Hz |
| 757,084 Hz | 3692,319 Hz | 8749,232 Hz |
| 870,4 Hz | 3952,308 Hz | 9354,812 Hz |
| 2454,423 Hz | 5230,227 Hz | 12532,729 Hz |
| 2480,191 Hz | 6022,942 Hz | 13467,209 Hz |
| 2581,101 Hz | 6061,711 Hz | 13777,9 Hz |
| 2715,232 Hz | 6710,899 Hz | 14015,241 Hz |
| 3042,012 Hz | 6721,912 Hz | 18524,419 Hz |
| 3196,194 Hz | 7181,784 Hz | |
| 3265,323 Hz | 7458,209 Hz | |
**8. AM Frequencies employed for treatment of colon cancer**
| | | |
|---|---|---|
| 78,76 Hz | 3131,123 Hz | 4318,222 Hz |
| 796,562 Hz | 3161,465 Hz | 4344,082 Hz |
| 841,541 Hz | 3175,313 Hz | 4416,221 Hz |
| 842,783 Hz | 3249,529 Hz | 4481,242 Hz |
| 914,429 Hz | 3363,229 Hz | 4724,263 Hz |
| 1162,117 Hz | 3373,892 Hz | 4751,319 Hz |
| 1372,207 Hz | 3390,925 Hz | 4755,323 Hz |
| 1372,934 Hz | 3409,179 Hz | 4788,485 Hz |
| 1718,532 Hz | 3432,274 Hz | 5149,331 Hz |
| 2243,169 Hz | 3509,522 Hz | 5217,402 Hz |
| 2278,312 Hz | 3531,422 Hz | 5386,212 Hz |
| 2286,5 Hz | 3533,328 Hz | 5407,192 Hz |
| 2286,519 Hz | 3766,296 Hz | 5426,323 Hz |
| 2334,178 Hz | 4040,839 Hz | 5496,434 Hz |
| 2423,292 Hz | 4081,022 Hz | 5555,212 Hz |
| 2454,423 Hz | 4123,953 Hz | 5572,032 Hz |
| 2464,229 Hz | 4146,274 Hz | 5634,933 Hz |
| 2598,853 Hz | 4233,822 Hz | 5724,231 Hz |
| 2623,048 Hz | 4282,332 Hz | 5758,378 Hz |
| 5787,342 Hz | 7368,222 Hz | 9744,193 Hz |
| 5948,897 Hz | 7645,859 Hz | 9942,321 Hz |
| 5967,448 Hz | 7829,234 Hz | 10301,371 Hz |
| 5976,825 Hz | 7866,229 Hz | 10401,515 Hz |
| 6182,322 Hz | 7877,334 Hz | 10872,693 Hz |
| 6292,379 Hz | 8013,314 Hz | 11220,222 Hz |
| 6324,493 Hz | 8374,942 Hz | 11283,378 Hz |
| 6341,248 Hz | 8384,228 Hz | 12256,432 Hz |
| 6471,322 Hz | 8408,121 Hz | 13749,858 Hz |
| 6477,218 Hz | 8534,111 Hz | 15231,548 Hz |
| 6558,342 Hz | 8568,033 Hz | 15248,324 Hz |
| 6855,286 Hz | 8573,122 Hz | 58191,928 Hz |
| 7129,843 Hz | 9226,222 Hz | 60317,352 Hz |
| 7140,187 Hz | 9351,9 Hz | |
| 7162,422 Hz | 9737,211 Hz | |
**9. AM Frequencies employed for treatment of pancreas cancer**
| | | |
|---|---|---|
| 331,3 Hz | 2315,921 Hz | 3564,419 Hz |
| 331,365 Hz | 2320,315 Hz | 3619,412 Hz |
| 436,3 Hz | 2334,178 Hz | 3622,312 Hz |
| 436,332 Hz | 2381,443 Hz | 3638,432 Hz |
| 447,942 Hz | 2469 Hz | 3696,424 Hz |
| 476,127 Hz | 2477,919 Hz | 3943,214 Hz |
| 559,292 Hz | 2542,221 Hz | 3976,929 Hz |
| 589,187 Hz | 2598,853 Hz | 4014,889 Hz |
| 624,218 Hz | 2647,938 Hz | 4041,219 Hz |
| 727 Hz | 2685,081 Hz | 4044,195 Hz |
| 734,921 Hz | 2716,095 Hz | 4056,384 Hz |
| 809,313 Hz | 2721,331 Hz | 4085,971 Hz |
| 845,309 Hz | 2732,231 Hz | 4144,592 Hz |
| 870,4 Hz | 2809,849 Hz | 4153,192 Hz |
| 963,221 Hz | 2823,428 Hz | 4161,889 Hz |
| 1156,79 Hz | 2835,332 Hz | 4243,393 Hz |
| 1157 Hz | 3134,313 Hz | 4332,498 Hz |
| 1179 Hz | 3241,461 Hz | 4341,423 Hz |
| 1360,133 Hz | 3255,219 Hz | 4355,327 Hz |
| 1372,207 Hz | 3263,432 Hz | 4417,885 Hz |
| 1372,934 Hz | 3286,255 Hz | 4422,322 Hz |
| 1804,126 Hz | 3330,935 Hz | 4451,297 Hz |
| 1816,221 Hz | 3373,892 Hz | 4486,384 Hz |
| 1873,477 Hz | 3438,109 Hz | 4558,306 Hz |
| 1967,211 Hz | 3449,219 Hz | 4580 Hz |
| 1990,482 Hz | 3535,219 Hz | 4685,082 Hz |
| 2278,312 Hz | 3549,215 Hz | 4839,589 Hz |
| 5151,402 Hz | 7320,494 Hz | 9942,321 Hz |
| 5209,911 Hz | 7366,412 Hz | 10279,122 Hz |
| 5262,282 Hz | 7534,221 Hz | 10388,49 Hz |
| 5271,312 Hz | 7548,713 Hz | 10438,495 Hz |
| 5387,73 Hz | 7567,127 Hz | 10518,311 Hz |
| 5494,928 Hz | 7620,851 Hz | 10528,239 Hz |
| 5521,221 Hz | 7663,209 Hz | 10582,095 Hz |
| 5573,209 Hz | 7725,203 Hz | 10926,111 Hz |
| 5609,382 Hz | 7852,233 Hz | 10948,411 Hz |
| 5929,616 Hz | 7920,879 Hz | 10955,558 Hz |
| 5948,897 Hz | 7985,122 Hz | 11538,193 Hz |
| 5966,112 Hz | 8008,323 Hz | 11904,741 Hz |
| 5976,825 Hz | 8013,312 Hz | 12255,229 Hz |
| 6064,197 Hz | 8045,484 Hz | 12613,341 Hz |
| 6086,256 Hz | 8242,332 Hz | 12819,942 Hz |
| 6157,253 Hz | 8351,622 Hz | 13674,482 Hz |
| 6215,298 Hz | 8408,121 Hz | 13731,322 Hz |
| 6333,917 Hz | 8455,894 Hz | 14525,312 Hz |
| 6365,242 Hz | 8551,231 Hz | 14537,218 Hz |
| 6558,342 Hz | 8743,321 Hz | 14549,331 Hz |
| 6568,278 Hz | 8789,631 Hz | 14845,453 Hz |
| 6823,194 Hz | 8868,809 Hz | 14944,989 Hz |
| 6853,391 Hz | 9012,241 Hz | 15246,315 Hz |
| 6855,286 Hz | 9028,994 Hz | 18668,239 Hz |
| 7213,204 Hz | 9131,232 Hz | 19321,231 Hz |
| 7228,528 Hz | 9658,296 Hz | 19347,208 Hz |
| 7238,232 Hz | 9663,495 Hz | 30182,932 Hz |
| 7277,921 Hz | 9680,737 Hz | |
| 7280,422 Hz | 9824,442 Hz | |
**10. AM Frequencies employed for treatment of lung cancer**
| | | |
|---|---|---|
| 304,148 Hz | 2761,312 Hz | 3461,322 Hz |
| 694,7 Hz | 2784,491 Hz | 3682,489 Hz |
| 694,727 Hz | 2812,443 Hz | 3727,231 Hz |
| 708,8 Hz | 2855,218 Hz | 3749,882 Hz |
| 708,841 Hz | 2859,495 Hz | 3769,942 Hz |
| 1587,811 Hz | 3128,822 Hz | 4131,235 Hz |
| 1759,318 Hz | 3139,297 Hz | 4158,393 Hz |
| 1873,477 Hz | 3193,212 Hz | 4243,393 Hz |
| 2253,704 Hz | 3348,783 Hz | 4347,733 Hz |
| 2391,312 Hz | 3360,971 Hz | 4373,411 Hz |
| 2454,232 Hz | 3366,311 Hz | 4378,321 Hz |
| 2729,929 Hz | 3373,892 Hz | 4416,221 Hz |
| 2741,261 Hz | 3440,212 Hz | 4481,242 Hz |
| 4777,521 Hz | 6838,434 Hz | 9181,434 Hz |
| 4798,422 Hz | 6870,955 Hz | 9317,913 Hz |
| 4837,241 Hz | 6879,216 Hz | 9363,896 Hz |
| 4959,842 Hz | 7079,411 Hz | 9736,919 Hz |
| 5013,321 Hz | 7216,288 Hz | 9753,321 Hz |
| 5047,523 Hz | 7376,089 Hz | 10424,908 Hz |
| 5068,322 Hz | 7761,289 Hz | 10452,913 Hz |
| 5371,922 Hz | 8082,173 Hz | 10824,609 Hz |
| 5538,432 Hz | 8281,259 Hz | 11656,329 Hz |
| 5548,879 Hz | 8352,189 Hz | 12748,919 Hz |
| 5679,309 Hz | 8442,473 Hz | 15774,291 Hz |
| 5734,143 Hz | 8773,916 Hz | 15798,333 Hz |
| 5787,342 Hz | 8935,752 Hz | 16510,321 Hz |
| 6445,309 Hz | 9121,223 Hz | |
**11. AM Frequencies employed for treatment of leiomyosarcoma**
| | | |
|---|---|---|
| 836,923 Hz | 4241,321 Hz | 6651,276 Hz |
| 843,181 Hz | 4266,591 Hz | 7168,892 Hz |
| 1411,241 Hz | 4337,322 Hz | 7406,309 Hz |
| 2073,721 Hz | 4424,112 Hz | 7452,528 Hz |
| 2381,443 Hz | 4436,111 Hz | 7649,209 Hz |
| 2711,019 Hz | 4485,22 Hz | 7808,352 Hz |
| 2911,329 Hz | 5545,521 Hz | 9040,313 Hz |
| 3232,185 Hz | 5577,841 Hz | 9074,294 Hz |
| 3518,321 Hz | 5631,422 Hz | 9189,092 Hz |
| 3544,209 Hz | 5696,184 Hz | 9484,512 Hz |
| 3569,219 Hz | 6472,098 Hz | 9943,972 Hz |
| 4233,822 Hz | 6558,342 Hz | 12086,394 Hz |
**12. AM Frequencies employed for treatment of mesothelioma**
| | | |
|---|---|---|
| 958,929 Hz | 3319,945 Hz | 6516,793 Hz |
| 1713,913 Hz | 3449,219 Hz | 7224,197 Hz |
| 1736,782 Hz | 3622,312 Hz | 9471,152 Hz |
| 2334,178 Hz | 5151,402 Hz | 14617,393 Hz |
| 2607,193 Hz | 5887,022 Hz | |
| 3112,974 Hz | 5965,922 Hz | |
**13. AM Frequencies employed for treatment of neuro-endocrine**
| | | |
|---|---|---|
| 1766,335 Hz | 3440,212 Hz | 6291,631 Hz |
| 2408,225 Hz | 3533,328 Hz | 6406,891 Hz |
| 2441,502 Hz | 3666,283 Hz | 6780,679 Hz |
| 2647,938 Hz | 4079,282 Hz | 7151,264 Hz |
| 2741,261 Hz | 4243,393 Hz | 7482,245 Hz |
| 3020,212 Hz | 4426,387 Hz | 7575,393 Hz |
| 3128,822 Hz | 5245,818 Hz | 8359,932 Hz |
| 3238,742 Hz | 5536,242 Hz | 9073,418 Hz |
| 3296,431 Hz | 5548,879 Hz | |
| 3348,783 Hz | 5739,422 Hz | |
| 3360,971 Hz | 5849,241 Hz | |
**14. AM Frequencies employed for treatment of leukemia and chronic lymphoid cancer**
| | | |
|---|---|---|
| 814,413 Hz | 3361,671 Hz | 7629,318 Hz |
| 825,145 Hz | 5245,452 Hz | 8172,405 Hz |
| 2415,243 Hz | 5557,333 Hz | 8272,338 Hz |
| 2436,316 Hz | 6850,197 Hz | 8438,453 Hz |
| 2874,432 Hz | 6919,322 Hz | 12950,331 Hz |
| 2891,029 Hz | 7587,224 Hz | |
**15. AM Frequencies employed for treatment of myeloma, multiple cancer**
| | | |
|---|---|---|
| 765,196 Hz | 2883,618 Hz | 5249,331 Hz |
| 2336,238 Hz | 2919,273 Hz | 7967,311 Hz |
| 2372,122 Hz | 3265,323 Hz | 7973,125 Hz |
| 2381,443 Hz | 3564,455 Hz | 8049,952 Hz |
| 2425,394 Hz | 3580,25 Hz | 8283,329 Hz |
| 2656,339 Hz | 3584,291 Hz | 10351,323 Hz |
| 2741,261 Hz | 3674,292 Hz | |
**16. AM Frequencies employed for treatment of Hodgkin disease**
| | | |
|---|---|---|
| 752,5 Hz | 3371,216 Hz | 5724,231 Hz |
| 976,3 Hz | 3605,432 Hz | 6358,194 Hz |
| 1558,223 Hz | 3623,198 Hz | 7472,211 Hz |
| 2310,912 Hz | 3838,281 Hz | 8062,121 Hz |
| 2477,919 Hz | 3838,48 Hz | 8222,222 Hz |
| 2560,843 Hz | 5102 Hz | |
| 3348,783 Hz | 5696,932 Hz | |
**17. AM Frequencies employed for treatment of brain cancer**
| | | |
|---|---|---|
| 1372,934 Hz | 4318,222 Hz | 6943,386 Hz |
| 2318,182 Hz | 4334,33 Hz | 7151,264 Hz |
| 2381,443 Hz | 4358,333 Hz | 7182,922 Hz |
| 2425,394 Hz | 4393,419 Hz | 7194,897 Hz |
| 2442,423 Hz | 4454,194 Hz | 7323,209 Hz |
| 2478,973 Hz | 4515,789 Hz | 7390,343 Hz |
| 2654,513 Hz | 4619,324 Hz | 7796,221 Hz |
| 2661,324 Hz | 4723,937 Hz | 7961,122 Hz |
| 2686,105 Hz | 4853,286 Hz | 8128,942 Hz |
| 2690,179 Hz | 5289,231 Hz | 8245,109 Hz |
| 3249,332 Hz | 5378,099 Hz | 8272,281 Hz |
| 3277,509 Hz | 5426,323 Hz | 8358,154 Hz |
| 3335,279 Hz | 5640,981 Hz | 8408,121 Hz |
| 3348,783 Hz | 6316,211 Hz | 9138,82 Hz |
| 3436,211 Hz | 6459,203 Hz | 10719,318 Hz |
| 3916,321 Hz | 6474,332 Hz | 11556,241 Hz |
| 4031,933 Hz | 6626,572 Hz | 12828,633 Hz |
| 4086,091 Hz | 6855,286 Hz | 14515,962 Hz |
| 4241,321 Hz | 6915,886 Hz | 14586,765 Hz |

2. A system (11) according to claim 1, in which the frequency of the one or more amplitude modulations generated are controllable to within an accuracy of 100 ppm relative to the one or more determined or predetermined reference amplitude modulation frequencies.

3. A system (11) according to claim 2, in which the frequency of the one or more amplitude modulations generated are controllable to within an accuracy of 10 ppm relative to the one or more determined or predetermined reference amplitude modulation frequencies.

4. A system (11) according to claim 3, in which the frequency of the one or more amplitude modulations generated are controllable to within an accuracy of about 1 ppm relative to the one or more determined or predetermined reference amplitude modulation frequencies.

5. A system (11) according to any one of the preceding claims, in which the amplitude modulated low energy electromagnetic emissions generated are maintained at a stability during emission of at least 10⁻⁵.

6. A system (11) according to claim 5, in which a stability of at least 10⁻⁶ is maintained.

7. A system according to claim 6, in which a stability of at least 10⁻⁷ is maintained.

8. A system (11) according to any one of the preceding claims, in which the one or more controllable generator circuits (29) are controllable by amplitude modulation control signals which lead to various forms of amplitude modulation wave forms being generated.

9. A system (11) according to claim 8, in which the amplitude modulation wave forms are selected from sinusoidal, square, triangular or multiple combinations thereof.

10. A system (11) according to claim 8 or claim 9, in which the one or more generator circuits (29) are controllable by amplitude modulation control signals which generate a plurality of amplitude modulation wave forms, either sequentially or simultaneously.

11. A system (11) according to any one of the preceding claims, in which the high frequency carrier signals to be generated by the one or more generator circuits (29) are selected from one or more high frequencies selected from about 27 MHz, 433 MHz and 900 MHz

12. A system (11) according to any one of the preceding claims, in which the system (11) comprises one or more interfaces or receiver means (16) communicating with the one or more data processors or integrated circuits (21), and in which the control information is transferable to the one or more interfaces or receiver means (16) and hence to the one or more data processors or integrated circuits (21) to enable command signals responsive to received control information to be communicated to the one or more generator circuits (29) by the one or more data processors or integrated circuits (21).

13. A system (11) according to claim 12, in which the control information is transferable or transmittable over the telephone, internet, radio or other means, to the one or more data processors or integrated circuits (21) via the one or more interfaces or receiver means (16) communicating with the one or more data processors or integrated circuits (21).

14. A system (11) according to claim 12 or claim 13, in which the control information is stored in an information storage device (52) and in which the control information is transferable to the one or more data processors or integrated circuits (21) via one or more interfaces (16) communicating with the one or more data processors or integrated circuits (21).

15. A system (11) according to any one of the preceding claims, in which the system comprises a user identification device (21a) communicating with at least one of the one or more data processors or integrated circuits (21) enabling the system (11) to be activated or employed for use only by the user.

16. A system (11) according to any one of the preceding claims, which includes a monitor (54) comprising monitoring software for monitoring the amplitude and amplitude modulation frequency of the amplitude modulated low energy electromagnetic emissions generated by the one or more generator circuits (29).

17. A system (11) according to any one of the preceding claims, in which the determined or predetermined amplitude modulation frequency control information is determined or predetermined by means of a bio-feedback process involving observations or measurements of physiological reactions by the subject when or during the time that cellular functions of the subject are excited by exposing the subject to emissions of high frequency carrier signals amplitude modulated at a series of amplitude modulation frequencies:

18. A system (11) according to claim 17, in which the determined or predetermined frequencies are employed as a mode to identify the nature of a tumour or cancer harboured by a warm-blooded mammalian subject.

## Patentansprüche

1. Durch elektrische Energie aktivierbares elektronisches System (11) zur Verwendung beim Inhibieren von kanzerogenem Zellwachstum oder dessen Proliferation in einem warmblütigen Säugetierlebewesen, Folgendes umfassend:
eine oder mehrere kontrollierbare Generatorschaltung/en (29) für niedrigenergetische elektromagnetische Energie, um ein oder mehrere Hochfrequenzträgersignal/e zu generieren,
eine/n oder mehrere Datenprozessor/en oder integrierte Schaltung/en (21), der bzw. die die eine oder die mehreren Generatorschaltungen (29) umfasst bzw. umfassen oder mit dieser/diesen kommuniziert bzw. kommunizieren und dazu bestimmt ist bzw. sind,
Kontrollinformation aus einer Kontrollinformationsquelle zu empfangen, bei der es sich um eine Anwendungsspeichervonichtung oder einen Computer (52) handelt,
wobei die eine oder die mehreren Generatorschaltung/en (29) einen oder mehrere Amplitudenmodulationskontrollsignalgenerator/en (34) umfasst bzw. umfassen, um amplitudenmodulierte Varianten des einen Hochfrequenzträgersignals oder der mehreren Hochfrequenzträgersignale zu kontrollieren,
wobei die eine oder die mehreren Generatorschaltung/en (29) darüber hinaus einen oder
mehrere programmierbare/n Amplitudenmodulationsfrequenzkontrollsignalgenerator/en (31) umfasst bzw. umfassen, um die Frequenz zu kontrollieren, mit der die Amplitudenmodulationen generiert werden,
wobei das System (11) darüber hinaus eine Verbindungs- oder Anschlussstelle (12a) zum Anschluss an einen elektrisch leitfähigen Applikator (12, 13) umfasst,
wobei der eine oder die mehreren programmierbare/n Amplitudenmodulationsfrequenzkontrollsignalgenerator/en (31) dazu angepasst ist bzw. sind, die Frequenz von Amplitudenmodulationen auf innerhalb einer Genauigkeit von mindestens 1000 ppm in Bezug auf eine oder mehrere bestimmte oder vorbestimmte
Referenzamplitudenmodulationsfrequenzen genau zu kontrollieren, die aus innerhalb eines Bereichs von 0,01 Hz bis 150 kHz ausgewählt sind,
und **dadurch gekennzeichnet, dass**
die Kontrollinformationsquelle (52) Referenzamplitudenmodulationsfrequenzkontrollinformation beinhaltet, die mindestens 50% der genau definierten Referenzamplitudenmodulationsfrequenzen umfasst, die in einem der nachstehenden Punkte 1 bis 17 aufgelistet sind,
wobei der Applikator (12, 13) dazu angepasst ist, durch Kontakt oder
Nahbereichsinduktion an das warmblütige Säugetierlebewesen eine oder mehrere amplitudenmodulierte niedrigenergetische Emissionen mit einer programmkontrollierten Frequenz anzulegen, die zu geringen Absorptionsgraden, im Wesentlichen von weniger als 1,6 milliW/g Gewicht Lebendgewebe führen,
und wobei die Amplitude moduliert wird, indem die mindestens 50% der Referenzamplitudenmodulationsfrequenzen sequentiell oder gleichzeitig angewendet werden.
1. AM-Frequenzen, die zur Behandlung von Brustkrebs verwendet werden
| | | |
|---|---|---|
| **78,76 Hz** | **3115,188 Hz** | **4969,224 Hz** |
| **181,821 Hz** | **3249,529 Hz** | **4979,321 Hz** |
| **414,817 Hz** | **3405,182 Hz** | **5027,231 Hz** |
| **440,933 Hz** | **3432,274 Hz** | **5059,792 Hz** |
| **628,431 Hz** | **3434,693 Hz** | **5118,094 Hz** |
| **721,313 Hz** | **3594,231 Hz** | **5176,287 Hz** |
| **813,205 Hz** | **3647,619 Hz** | **5365,222 Hz** |
| **818,342 Hz** | **3742,957 Hz** | **5376,392 Hz** |
| **891,901 Hz** | **3753,382 Hz** | **5426,323 Hz** |
| **929,095 Hz** | **3830,732 Hz** | **5431,542 Hz** |
| **929,1 Hz** | **3855,823 Hz** | **5521,621 Hz** |
| **1021 Hz** | **3916,321 Hz** | **5739,422 Hz** |
| **1372,207 Hz** | **3935,218 Hz** | **5745,218 Hz** |
| **1372,934 Hz** | **3975,383 Hz** | **5821,975 Hz** |
| **1588,721 Hz** | **3993,437 Hz** | **6037,432 Hz** |
| **1670,699 Hz** | **4153,192 Hz** | **6044,333 Hz** |
| **1821,729 Hz** | **4194,968 Hz** | **6086,256 Hz** |
| **1836,219 Hz** | **4241,321 Hz** | **6208,932 Hz** |
| **2193,937 Hz** | **4243,393 Hz** | **6212,808 Hz** |
| **2221,323 Hz** | **4253,432 Hz** | **6231,031 Hz** |
| **2278,312 Hz** | **4314,444 Hz** | **6280,321 Hz** |
| **2357,832 Hz** | **4318,222 Hz** | **6329,391 Hz** |
| **2381,443 Hz** | **4375,962 Hz** | **6476,896 Hz** |
| **2417,323 Hz** | **4393,419 Hz** | **6497,319 Hz** |
| **2431,334 Hz** | **4417,243 Hz** | **6504,983 Hz** |
| **2450,332 Hz** | **4461,463 Hz** | **6651,276 Hz** |
| **2551,313 Hz** | **4482,223 Hz** | **6757,901 Hz** |
| **2556,221 Hz** | **4495,138 Hz** | **6758,321 Hz** |
| **2598,853 Hz** | **4549,808 Hz** | **6655,286 Hz** |
| **2621,322 Hz** | **4558,306 Hz** | **6858,121 Hz** |
| **2740,191 Hz** | **4779,451 Hz** | **6898,489 Hz** |
| **2851,347 Hz** | **4838,674 Hz** | **7092,219 Hz** |
| **2885,322 Hz** | **4871,513 Hz** | **7120,218 Hz** |
| **2919,213 Hz** | **4895,296 Hz** | **7127,311 Hz** |
| **3074,333 Hz** | **4962,213 Hz** | **7156,489 Hz** |
| **7205,821 Hz** | **8548,324 Hz** | **11525,121 Hz** |
| **7282,169 Hz** | **8749,383 Hz** | **11541,915 Hz** |
| **7378,329 Hz** | **8782,421 Hz** | **11812,328 Hz** |
| **7488,742 Hz** | **8784,424 Hz** | **11812,419 Hz** |
| **7541,319 Hz** | **8923,1 Hz** | **11840,323 Hz** |
| **7577,421 Hz** | **8923,361 Hz** | **11925,089 Hz** |
| **7621,085 Hz** | **8935,752 Hz** | **12123,281 Hz** |
| **7027,207 Hz** | **8936,1 Hz** | **12267,281 Hz** |
| **7650,939 Hz** | **9012,282 Hz** | **12294,283 Hz** |
| **7691,212 Hz** | **9012,896 Hz** | **12611,288 Hz** |
| **7842,184 Hz** | **9060,323 Hz** | **12629,222 Hz** |
| **7649,231 Hz** | **9072,409 Hz** | **12633,372 Hz** |
| **7915,423 Hz** | **9131,419 Hz** | **12648,221 Hz** |
| **7932,482 Hz** | **9199,232 Hz** | **13315,335 Hz** |
| **7949,196 Hz** | **9245,927 Hz** | **13331,358 Hz** |
| **7967,311 Hz** | **9270,322 Hz** | **13735,241 Hz** |
| **8021,229 Hz** | **9279,193 Hz** | **13826,325 Hz** |
| **8070,181 Hz** | **9393,946 Hz** | **13853,232 Hz** |
| **8114,032 Hz** | **10227,242 Hz** | **13990,123 Hz** |
| **8149,922 Hz** | **10340,509 Hz** | **14122,942 Hz** |
| **8194.19 Hz** | **10363,313 Hz** | **14162,332 Hz** |
| **8245,801 Hz** | **10449,323 Hz** | **14519,232 Hz** |
| **8328,322 Hz** | **10456,383 Hz** | **14543,128 Hz** |
| **8330,534 Hz** | **10468,231 Hz** | **15651,323 Hz** |
| **8355,987 Hz** | **10470,456 Hz** | **17352,085 Hz** |
| **8408,121 Hz** | **10472,291 Hz** | **18785,463 Hz** |
| **8431,184 Hz** | **10689,339 Hz** | **30182,932 Hz** |
| **8452,119 Hz** | **10832,222 Hz** | |
2. AM-Frequenzen, die zur Behandlung von Leberkrebs verwendet werden
| | | |
|---|---|---|
| **423.321 Hz** | **1250,504 Hz** | **2353,478 Hz** |
| **427,052 Hz** | **1755,402 Hz** | **2362,309 Hz** |
| **470,181 Hz** | **1873.477 Hz** | **2419,309 Hz** |
| **560,32 Hz** | **1924,792 Hz** | **2425,222 Hz** |
| **642,932 Hz** | **1975,196 Hz** | **2430,219 Hz** |
| **668,209 Hz** | **2017,962 Hz** | **2431,094 Hz** |
| **677,972 Hz** | **2083,419 Hz** | **2471,328 Hz** |
| **811,924 Hz** | **2190,731 Hz** | **2478,331 Hz** |
| **842,311 Hz** | **2221,323 Hz** | **2743,995 Hz** |
| **843,22 Hz** | **2324,393 Hz** | **2744,211 Hz** |
| **2831,951 Hz** | **5520,218 Hz** | **8542,311 Hz** |
| **2843,283 Hz** | **5882,292 Hz** | **8818,104 Hz** |
| **2859,891 Hz** | **5926,512 Hz** | **8852,329 Hz** |
| **2873,542 Hz** | **6037,311 Hz** | **8853,444 Hz** |
| **2886,232 Hz** | **6180,334 Hz** | **8858,179 Hz** |
| **3042,012 Hz** | **6329,195 Hz** | **8939,212 Hz** |
| **3078,983 Hz** | **6350,333 Hz** | **9332,397 Hz** |
| **3086,443 HZ** | **6361,321 Hz** | **9381,221 Hz** |
| **3127,232 Hz** | **6364,928 Hz** | **9740,219 Hz** |
| **3160,942 Hz** | **6383,321 Hz** | **9768,331 Hz** |
| **3206,315 Hz** | **6461,175 Hz** | **9797,294 Hz** |
| **3267,433 Hz** | **6733,331 Hz** | **10317,499 Hz** |
| **3269,321 Hz** | **6758,232 Hz** | **10443,311 Hz** |
| **3457,291 Hz** | **6779,482 Hz** | **10456,383 Hz** |
| **3505,229 Hz** | **6856,222 Hz** | **10579,425 Hz** |
| **3516,296 Hz** | **6877,183 Hz** | **10863,209 Hz** |
| **3531,296 Hz** | **6980,525 Hz** | **10866,382 Hz** |
| **3546,323 Hz** | **7019,235 Hz** | **11067,418 Hz** |
| **3572,106 Hz** | **7043,209 Hz** | **11149,935 Hz** |
| **3576,189 Hz** | **7130,323 Hz** | **11163,895 Hz** |
| **3669,513 Hz** | **7144,142 Hz** | **11802,821 Hz** |
| **3923,221 Hz** | **7210.223 Hz** | **11953,424 Hz** |
| **4013,932 Hz** | **7291,21 Hz** | **12223,329 Hz** |
| **4071,121 Hz** | **7510,92 Hz** | **12265,295 Hz** |
| **4079,951 Hz** | **7529,233 Hz** | **12267,233 Hz** |
| **4222,821 Hz** | **7549,212 Hz** | **12623,191** |
| **4238,402 Hz** | **7650,028 Hz** | **12685,231 Hz** |
| **4256,321 Hz** | **7680,518 Hz** | **12721,423 Hz** |
| **4289,296 Hz** | **7692,522 Hz** | **12785,342 Hz** |
| **4312,947 Hz** | **7829,231 Hz** | **14085,222 Hz** |
| **4435,219 Hz** | **7862,209 Hz** | **14333,209 Hz** |
| **4471,188 Hz** | **7947,392 Hz** | **14537,331 Hz** |
| **4463,889 Hz** | **7979,308 Hz** | **14542,432 Hz** |
| **4486,384 Hz** | **8028,339 Hz** | **14655,03 Hz** |
| **4629,941 Hz** | **8055,942 Hz** | **14828,234 Hz** |
| **4732,211 Hz** | **8072,134 Hz** | **15149,213 Hz** |
| **4876,218 Hz** | **8141,174 Hz** | **15237,489 Hz** |
| **5086,281 Hz** | **8336,383 Hz** | **16110,932 Hz** |
| **5124,084 Hz** | **8432,181 Hz** | **16144,343 Hz** |
| **5133,121 Hz** | **8452,119 Hz** | **18265,238 Hz** |
| **5247,142 Hz** | **8460,944 Hz** | **18283,323 Hz** |
| **5270,834 Hz** | **8475,221 Hz** | **18863,292 Hz** |
| **5340,497 Hz** | **8492,193 Hz** | **18930,995 Hz** |
| **19970,311 Hz** | **20330,294 Hz** | **20365,284 Hz** |
3. AM-Frequenzen, die zur Behandlung von Eierstockkrebs verwendet werden
| | | |
|---|---|---|
| **78,76 Hz** | **1372,207 Hz** | **2812,321 Hz** |
| **181,821 Hz** | **1396,498 Hz** | **2631,386 Hz** |
| **410,245 Hz** | **1502,181 Hz** | **2835,332 Hz** |
| **414,817 Hz** | **1518,208 Hz** | **2851,347 Hz** |
| **436,332 Hz** | **1552,123 Hz** | **2877,192 Hz** |
| **447,942 Hz** | **1579,212 Hz** | **2885,322 Hz** |
| **481,191 Hz** | **1624,802 Hz** | **2887,385 Hz** |
| **489,292 Hz** | **1670,699 Hz** | **2894,972 Hz** |
| **559,292 Hz** | **1696,403 Hz** | **2973,771 Hz** |
| **608,321 Hz** | **1762,938 Hz** | **3080,592 Hz** |
| **655,435 Hz** | **1771,402 Hz** | **3157,483 Hz** |
| **657,397 Hz** | **1775,313 Hz** | **3161,485 Hz** |
| **657,483 Hz** | **1821,729 Hz** | **3223,232 Hz** |
| **664,211 Hz** | **2016,323 Hz** | **3238,148 Hz** |
| **708,8 Hz** | **2034,231 Hz** | **3249,529 Hz** |
| **708,822 Hz** | **2050,282 Hz** | **3262,145 Hz** |
| **734,921 Hz** | **2053,396 Hz** | **3314,321 Hz** |
| **749,221 Hz** | **2082,234 Hz** | **3361,671 Hz** |
| **764,232 Hz** | **2089,092 Hz** | **3366,311 Hz** |
| **778,295 Hz** | **2221,323 Hz** | **3523,215 Hz** |
| **779,403 Hz** | **2228,832 Hz** | **3527,233 Hz** |
| **806,021 Hz** | **2253,704 Hz** | **3542,213 Hz** |
| **806,389 Hz** | **2254,329 Hz** | **3590,376 Hz** |
| **809,313 Hz** | **2278,312 Hz** | **3629,232 Hz** |
| **824,327 Hz** | **2332,949 Hz** | **3632,793 Hz** |
| **825,146 Hz** | **2348,233 Hz** | **3636,289 Hz** |
| **835,129 Hz** | **2381,443 Hz** | **3637,085 Hz** |
| **839,521 Hz** | **2413,193 Hz** | **3669,513 Hz** |
| **841,208 Hz** | **2425,222 Hz** | **3770.189 Hz** |
| **843,312 Hz** | **2433,321 Hz** | **3858,918 Hz** |
| **956,984 Hz** | **2439,253 Hz** | **3919,232 Hz** |
| **958,929 Hz** | **2465,23 Hz** | **3957,185 Hz** |
| **985,313 Hz** | **2477,919 Hz** | **3975,228 Hz** |
| **1024,208 Hz** | **2669,177 Hz** | **4061,131 Hz** |
| **1102,635 Hz** | **2715,232 Hz** | **4072,322 Hz** |
| **1121,329 Hz** | **2733,843 Hz** | **4169,451 Hz** |
| **1159,738 Hz** | **2802,339 Hz** | **4174,259 Hz** |
| **4241,321 Hz** | **6350,333 Hz** | **8428,313 Hz** |
| **4243,393 Hz** | **6406,891 Hz** | **8435,451 Hz** |
| **4261,228 Hz** | **6407,207 Hz** | **8486,421 Hz** |
| **4279,113 Hz** | **6450,787 Hz** | **8492,797 Hz** |
| **4309,335 Hz** | **6477,098 Hz** | **8548,324 Hz** |
| **4314,188 Hz** | **8477,929 Hz** | **8554,361 Hz** |
| **4318,222 Hz** | **6478.338 Hz** | **8562,965 Hz** |
| **4328,928 Hz** | **6543,421 Hz** | **8579,323 Hz** |
| **4380,321 Hz** | **6552,24 Hz** | **8579,333 Hz** |
| **4394,134 Hz** | **6663,955 Hz** | **8642,181 Hz** |
| **4412,252 Hz** | **6753,330 Hz** | **8655,818 Hz** |
| **4424,236 Hz** | **6851,323 Hz** | **8758,341 Hz** |
| **4439,341 Hz** | **6855,286 Hz** | **8779,323 Hz** |
| **4442,161 Hz** | **6875,232 Hz** | **8792,231 Hz** |
| **4447,221 Hz** | **6882,949 Hz** | **8819,127 Hz** |
| **4458,339 Hz** | **7206,403 Hz** | **8831,132 Hz** |
| **4556,322 Hz** | **7232,214 Hz** | **9028,031 Hz** |
| **4566,009 Hz** | **7257,489 Hz** | **9173,264 Hz** |
| **4662,643 Hz** | **7276,209 Hz** | **9184,338 Hz** |
| **4718,331 Hz** | **7281,219 Hz** | **9186,919 Hz** |
| **4749,302 Hz** | **7285,693 Hz** | **9393,946 Hz** |
| **4765,331 Hz** | **7429,212 Hz** | **9482,409 Hz** |
| **4917,202 Hz** | **7460,932 Hz** | **9737,211 Hz** |
| **5011,325 Hz** | **7480,228 Hz** | **9746,232 Hz** |
| **5149,331 Hz** | **7495,763 MHz** | **9922,231 Hz** |
| **5228,172 Hz** | **7539,432 Hz** | **10032,684 Hz** |
| **5237,132 Hz** | **7584,185 Hz** | **10446,028 Hz** |
| **5313,353 Hz** | **7650,028 Hz** | **10478.221 Hz** |
| **5745,218 Hz** | **7689,728 Hz** | **10545,313 Hz** |
| **5757,897 Hz** | **7780,294 Hz** | **10639,345 Hz** |
| **5762,386 Hz** | **8021,921 Hz** | **10743,118 Hz** |
| **5812,322 Hz** | **8038,961 Hz** | **10813,981 Hz** |
| **5869,321 Hz** | **8040,322 Hz** | **10832,421 Hz** |
| **5882,292 Hz** | **8044,233 Hz** | **10838,243 Hz** |
| **5921,249 Hz** | **8095,313 Hz** | **10862,429 Hz** |
| **5991,932 Hz** | **8143,491 Hz** | **10865,127 Hz** |
| **6069,458 Hz** | **8164,332 Hz** | **10917,229 Hz** |
| **6071,319 Hz** | **8261,121 Hz** | **10977,188 Hz** |
| **6083,214 Hz** | **8302,285 Hz** | **11120,209 Hz** |
| **6161,782 Hz** | **8309,752 Hz** | **11177,289 Hz** |
| **6169,341 Hz** | **8372,532 Hz** | **11177,409 Hz** |
| **6275,232 Hz** | **8408,121 Hz** | **11321,491 Hz** |
| **6294,929 Hz** | **8424,229 Hz** | **11359,093 Hz** |
| **11673,031 Hz** | **12755,333 Hz** | **15450,183 Hz** |
| **11793,060 Hz** | **12947,311 Hz** | **16144,343 Hz** |
| **11895,229 Hz** | **13717,221 Hz** | **17932,432 Hz** |
| **12074,531 Hz** | **13825,295 Hz** | **17951,395 Hz** |
| **12210,212 Hz** | **13829,195 Hz** | **17970,122 Hz** |
| **12253,329 Hz** | **14410,949 Hz** | **18337,222 Hz** |
| **12260,933 Hz** | **14436,201 Hz** | **18376,321 Hz** |
| **12262,853 Hz** | **14537,218 Hz** | **18921,415 Hz** |
| **12292,222 Hz** | **14941,184 Hz** | **18926,951 Hz** |
| **12351,353 Hz** | **15429,139 Hz** | **18931,327 Hz** |
| **12527,032 Hz** | **15443,309 Hz** | **114508,332 Hz** |
4. AM-Frequenzen, die zur Behandlung von Prostatakrebs verwendet werden
| | | |
|---|---|---|
| **331.3 Hz** | **809,205 Hz** | **2628,324 Hz** |
| **331,358 Hz** | **819,322 Hz** | **2669,328 Hz** |
| **403,218 Hz** | **844,8 Hz** | **2824,832 Hz** |
| **461,233 Hz** | **844,822 Hz** | **2887,829 Hz** |
| **522,2 Hz** | **847,332 Hz** | **2891,331 Hz** |
| **522,213 Hz** | **1083,309 Hz** | **3081,523 Hz** |
| **618,4 Hz** | **1102,635 Hz** | **3249,529 Hz** |
| **618,407 Hz** | **1102,71 Hz** | **3250,125 Hz** |
| **618,8 Hz** | **1240,336 Hz** | **3251,815 Hz** |
| **656,295 Hz** | **1372,934 Hz** | **3264,827 Hz** |
| **657,394 Hz** | **1444,288 Hz** | **3278,329 Hz** |
| **657,397 Hz** | **1486,322 Hz** | **3281.432 Hz** |
| **657,4 Hz** | **1563,332 Hz** | **3348,783 Hz** |
| **657,483 Hz** | **1591,322 Hz** | **3519,118 Hz** |
| **659,033 Hz** | **1670,699 Hz** | **3539,962 Hz** |
| **694,4 Hz** | **1697,321 Hz** | **3551,318 Hz** |
| **694,689 Hz** | **1743,521 Hz** | **3556,439 Hz** |
| **694,7 Hz** | **2031,448 Hz** | **3572,321 Hz** |
| **741,4 Hz** | **2050,282 Hz** | **3670,129 Hz** |
| **741,421 Hz** | **2076,519 Hz** | **3681,341 Hz** |
| **749,221 Hz** | **2156,332 Hz** | **3686,021 Hz** |
| **752,9 Hz** | **2229,515 Hz** | **3753,382 Hz** |
| **752,933 Hz** | **2243,121 Hz** | **3774,923 Hz** |
| **776,194 Hz** | **2381,443 Hz** | **3867,692 Hz** |
| **785,219 Hz** | **2440,489 Hz** | **3909,333 Hz** |
| **786,332 Hz** | **2475,912 Hz** | **3916,321 Hz** |
| **793,331 Hz** | **2477,919 Hz** | **4031,233 Hz** |
| **4031,933 Hz** | **6590,328 Hz** | **9018,233 Hz** |
| **4038,203 Hz** | **6651,276 Hz** | **9068,231 Hz** |
| **4081,743 Hz** | **6706,431 Hz** | **9137,232 Hz** |
| **4084,319 Hz** | **6743,322 Hz** | **9156.321 Hz** |
| **4139,322 Hz** | **6783,282 Hz** | **9351.931 Hz** |
| **4153,192 Hz** | **6950,197 Hz** | **9393,948 Hz** |
| **4223,795 Hz** | **6855,286 Hz** | **9694,179 Hz** |
| **4231,221 Hz** | **6864,896 Hz** | **9984,405 Hz** |
| **4241,321 Hz** | **6871,943 Hz** | **10226.223 Hz** |
| **4320,513 Hz** | **6973,393 Hz** | **10390.232 Hz** |
| **4329,152 Hz** | **7120,932 Hz** | **10514,768 Hz** |
| **4380,321 Hz** | **7146,509 Hz** | **10689.339 Hz** |
| **4417,312 Hz** | **7192,505 Hz** | **10772,419 Hz** |
| **4489,452 Hz** | **7251,309 Hz** | **10818,452 Hz** |
| **4549,808 Hz** | **7251,322 Hz** | **11165,239 Hz** |
| **4558,306 Hz** | **7218,124 Hz** | **11985,353 Hz** |
| **4638,293 Hz** | **7279,335 Hz** | **12209,329 Hz** |
| **4740,322 Hz** | **7299,119 Hz** | **12308,321 Hz** |
| **4854,318 Hz** | **7527,229 Hz** | **12583,339 Hz** |
| **4882,322 Hz** | **7589,925 Hz** | **13820,329 Hz** |
| **4978,822 Hz** | **7699,193 Hz** | **14013,123 Hz** |
| **5237,152 Hz** | **7842,184 Hz** | **14171,434 Hz** |
| **5264,222 Hz** | **8023,32 Hz** | **14681,329 Hz** |
| **5289,195 Hz** | **8096,939 Hz** | **14759,131 Hz** |
| **5426,323 Hz** | **8245,801 Hz** | **14986,794 Hz** |
| **5431,542 Hz** | **8315,291 Hz** | **15930,249 Hz** |
| **5455,593 Hz** | **8357,305 Hz** | **16026,623 Hz** |
| **6345,332 Hz** | **8408,121 Hz** | **17880,954 Hz** |
| **6347,433 Hz** | **8432,209 Hz** | **18247,532 Hz** |
| **6363,284 Hz** | **8535,238 Hz** | **18282,211 Hz** |
| **6418,331 Hz** | **8552,431 Hz** | **18629,328 Hz** |
| **6496,231 Hz** | **8585,224 Hz** | **19469,318 Hz** |
| **6538,295 Hz** | **8935,752 Hz** | **19766,218 Hz** |
| **6577,421 Hz** | **9015,253 Hz** | **60317,352 Hz** |
5. AM-Frequenzen, die zur Behandlung von Nierenkrebs verwendet werden
| | | |
|---|---|---|
| **628,321 Hz** | **826,321 Hz** | **2254,329 Hz** |
| **631,141 Hz** | **1372,934 Hz** | **3555,209 Hz** |
| **643,312 Hz** | **2082,241 Hz** | **3928,343 Hz** |
| **812,512 Hz** | **2156,931 Hz** | **4420,932 Hz** |
| **4819,228 Hz** | **8727,224 Hz** | **11421,933 Hz** |
| **4828,321 Hz** | **8760,963 Hz** | **11523,212 Hz** |
| **5314,322 Hz** | **8831,132 Hz** | **11561,221 Hz** |
| **6007,332 Hz** | **8870,228 Hz** | **11846,212 Hz** |
| **7054,279 Hz** | **10565,321 Hz** | **12631,331 Hz** |
| **7074,429 Hz** | **10586,229 Hz** | **12693,272 Hz** |
| **7254,343 Hz** | **10634,293 Hz** | **14411,321 Hz** |
| **8041,289 Hz** | **10687,949 Hz** | **20178,941 Hz** |
6. AM-Frequenzen, die zur Behandlung von Schilddrüsenkrebs verwendet werden
| | | |
|---|---|---|
| **493,442 Hz** | **3284,192 Hz** | **6746,333 Hz** |
| **511,202 Hz** | **3335,332 Hz** | **6766,261 Hz** |
| **618,927 Hz** | **3434,911 Hz** | **6864,432 Hz** |
| **621,321 Hz** | **3440,212 Hz** | **7036,122 Hz** |
| **648,252 Hz** | **3475,210 Hz** | **7230,838 Hz** |
| **663,401 Hz** | **3509,522 Hz** | **7323,209 Hz** |
| **821,202 Hz** | **3533,326 Hz** | **7355,378 Hz** |
| **874,341 Hz** | **3637,085 Hz** | **7432,143 Hz** |
| **914,429 Hz** | **3682,489 Hz** | **7534,221 Hz** |
| **941,311 Hz** | **4154,301 Hz** | **7623,184 Hz** |
| **983.429 Hz** | **4243,393 Hz** | **7725,339 Hz** |
| **1587,811 Hz** | **4261,228 Hz** | **7920,879 Hz** |
| **1723,389 Hz** | **4330,289 Hz** | **8013,953 Hz** |
| **2179,231 Hz** | **4340,833 Hz** | **8019,912 Hz** |
| **2315.888 Hz** | **4358,333 Hz** | **8040,231 Hz** |
| **2341,312 Hz** | **4366,294 Hz** | **8078,955 Hz** |
| **2445,123 Hz** | **4426,387 Hz** | **8092,173 Hz** |
| **2454,232 Hz** | **4458,339 Hz** | **8147,1 Hz** |
| **2723,302 Hz** | **4479,113 Hz** | **8281,259 Hz** |
| **2740,384 Hz** | **4744,424 Hz** | **8309,752 Hz** |
| **2749,323 Hz** | **4865,421 Hz** | **8311,371 Hz** |
| **2856,253 Hz** | **5323,192 Hz** | **8435,094 Hz** |
| **2859,495 Hz** | **5324,123 Hz** | **8525,789 Hz** |
| **2886,232 Hz** | **5548,879 Hz** | **8744,527 Hz** |
| **3021,122 Hz** | **5711,283 Hz** | **9009,329 Hz** |
| **3078,275 Hz** | **5754,332 Hz** | **9070,809 Hz** |
| **3080,592 Hz** | **6455,131 Hz** | **10020,521 Hz** |
| **3198,323 Hz** | **6620,132 Hz** | **10039,109 Hz** |
| **3248,321 Hz** | **6666,839 Hz** | **10127,279 Hz** |
| **3271,329 Hz** | **6714,189 Hz** | **10134,161 Hz** |
| **10257,324 Hz** | **12120,049 Hz** | **16023,119 Hz** |
| **10498,339 Hz** | **12139,222 Hz** | **16048,391 Hz** |
| **11537.292 Hz** | **13636,082 Hz** | **17323,196 Hz** |
| **11559,292 Hz** | **13654,272 Hz** | **17577,221 Hz** |
| **11913,222 Hz** | **13677,211 Hz** | **17881,709 Hz** |
| **11927,934 Hz** | **14014,941 Hz** | **17911,323 Hz** |
| **11955,949 Hz** | **14445,214 Hz** | |
7. AM-Frequenzen, die zur Behandlung von Blasenkrebs verwendet werden
| | | |
|---|---|---|
| **623,243 Hz** | **3438,109 Hz** | **8235,21 Hz** |
| **151,084 Hz** | **3692,319 Hz** | **8749,232 Hz** |
| **870,4 Hz** | **3952,308 Hz** | **9354,812 Hz** |
| **2454,423 Hz** | **5230,227 Hz** | **12532,129 Hz** |
| **2480,191 Hz** | **6022,942 Hz** | **13467,209 Hz** |
| **2581,101 Hz** | **6061,711 Hz** | **13777,9 Hz** |
| **2715,232 Hz** | **6710,899 Hz** | **14015,241 Hz** |
| **3042,012 Hz** | **6721,912 Hz** | **18524,419 Hz** |
| **3196,194 Hz** | **7181,784 Hz** | |
| **3265,323 Hz** | **7458,209 Hz** | |
8. AM-Frequenzen, die zur Behandlung von Dickdarmkrebs verwendet werden
| | | |
|---|---|---|
| **78,76 Hz** | **3131,123 Hz** | **4318,222 Hz** |
| **796,562 Hz** | **3161,465 Hz** | **4344,082 Hz** |
| **841,541 Hz** | **3175,313 Hz** | **4416,221 Hz** |
| **842,783 Hz** | **3249,529 Hz** | **4481,242 Hz** |
| **914.429 Hz** | **3363,229 Hz** | **4724,263 Hz** |
| **1162,117 Hz** | **3373,892 Hz** | **4751,319 Hz** |
| **1372,207 Hz** | **3390,925 Hz** | **4755,323 Hz** |
| **1372,934 Hz** | **3409,179 Hz** | **4788.485 Hz** |
| **1718,532 Hz** | **3432.274 Hz** | **5149.331 Hz** |
| **2243,169 Hz** | **3500,522 Hz** | **5217.402 Hz** |
| **2278,312 Hz** | **3531,422 Hz** | **5386,212 Hz** |
| **2286,5 Hz** | **3533,328 Hz** | **5407,192 Hz** |
| **2268,510 Hz** | **3766,298 Hz** | **5426,323 Hz** |
| **2334.178 Hz** | **4040,839 Hz** | **5496,434 Hz** |
| **2423,292 Hz** | **4081,022 Hz** | **5555,212 Hz** |
| **2454,423 Hz** | **4123,953 Hz** | **5572,032 Hz** |
| **24454,229 Hz** | **4146,274 Hz** | **5634,933 Hz** |
| **2588.853 Hz** | **4233,822 Hz** | **5724,231 Hz** |
| **2623.048 Hz** | **4262,332 Hz** | **5758,378 Hz** |
| **5787.342 Hz** | **7368,222 Hz** | **9744,193 Hz** |
| **5948,897 Hz** | **7645,859 Hz** | **9942,321 Hz** |
| **5967,448 Hz** | **7829,234 Hz** | **10301.371 Hz** |
| **5976.825 Hz** | **7866,229 Hz** | **10401,515 Hz** |
| **6182,322 Hz** | **7877,334 Hz** | **10872,593 Hz** |
| **8292,319 Hz** | **8013,314 Hz** | **11220,222 Hz** |
| **6324,493 Hz** | **6374,942 Hz** | **11283,378 Hz** |
| **6341,248 Hz** | **8384,228 Hz** | **12256,432 Hz** |
| **6471,322 Hz** | **8408,121 Hz** | **13749,858 Hz** |
| **5477,218 Hz** | **8534,111 Hz** | **15231,548 Hz** |
| **6558,342 Hz** | **8566,033 Hz** | **15248,324 Hz** |
| **6855,286 Hz** | **8573,122 Hz** | **58191,928 Hz** |
| **7129,843 Hz** | **9226,222 Hz** | **60317,352 Hz** |
| **7140,167 Hz** | **9351,9 Hz** | |
| **7162,422 Hz** | **9737,211 Hz** | |
9. AM-Frequenzen, die zur Behandlung von Pankreaskrebs verwendet werden
| | | |
|---|---|---|
| **331,3 Hz** | **2315,921 Hz** | **3564,419 Hz** |
| **331,365 Hz** | **2320,315 Hz** | **3619,412 MHz** |
| **436,3 Hz** | **2334,178 Hz** | **3622,312 Hz** |
| **436,332 Hz** | **2381,443 Hz** | **3638,432 Hz** |
| **447.942 Hz** | **2469 Hz** | **3696,424 Hz** |
| **476,127 Hz** | **2477,919 Hz** | **3943,214 Hz** |
| **559,292 Hz** | **2542,221 Hz** | **3976,929 Hz** |
| **589,187 Hz** | **2598,853 Hz** | **4014,889 Hz** |
| **824,218 Hz** | **2647,908 Hz** | **4041,219 Hz** |
| **727 Hz** | **2685,081 Hz** | **4044,195 Hz** |
| **734,921 Hz** | **2710,095 Hz** | **4056,384 Hz** |
| **809,313 Hz** | **2721,331 Hz** | **4085,971 Hz** |
| **845,309 Hz** | **2132,231 Hz** | **4144,592 Hz** |
| **870,4 Hz** | **2809,849 Hz** | **4163,192 Hz** |
| **963,221 Hz** | **2823,428 Hz** | **4161,889 Hz** |
| **1156,79 Hz** | **2835,332 Hz** | **4243,393 Hz** |
| **1157 Hz** | **3134,313 Hz** | **4332,498 Hz** |
| **1179 Hz** | **3241,461 Hz** | **4341,423 Hz** |
| **1380,133 Hz** | **3255,219 Hz** | **4355,327 Hz** |
| **1372,207 Hz** | **3283,432 Hz** | **4417,885 Hz** |
| **1372,934 Hz** | **3286,255 Hz** | **4422,322 Hz** |
| **1804,126 Hz** | **3330,935 Hz** | **4451,297 Hz** |
| **1816,221 Hz** | **3373,892 Hz** | **4486,384 Hz** |
| **1873,477 Hz** | **3438,109 Hz** | **4558,306 Hz** |
| **1967,211 Hz** | **3449,219 Hz** | **4580 Hz** |
| **1990,462 Hz** | **3535,219 Hz** | **4685,082 Hz** |
| **2278,312 Hz** | **3549,215 Hz** | **4839,589 Hz** |
| **5151,402 Hz** | **7320,494 Hz** | **9942,321 Hz** |
| **5209,911 Hz** | **7366,412 Hz** | **10279,122 Hz** |
| **5262,262 Hz** | **7534,221 Hz** | **10388,49 Hz** |
| **5271,312 Hz** | **7548,713 Hz** | **10438,495 Hz** |
| **5387,73 Hz** | **1567,127 Hz** | **10518,311 Hz** |
| **5494,928 Hz** | **7620,851 Hz** | **10528,239 Hz** |
| **5521,221 Hz** | **7663,209 Hz** | **10582,095 Hz** |
| **5573,209 Hz** | **7725,203 Hz** | **10926,111 Hz** |
| **5609,382 Hz** | **7852,233 Hz** | **10948,411 Hz** |
| **5929,616 Hz** | **7920,879 Hz** | **10955,558 Hz** |
| **5948,897 Hz** | **7985,122 Hz** | **11538,193 Hz** |
| **5966,112 Hz** | **8008,323 Hz** | **11904,741 Hz** |
| **5976,825 Hz** | **8013,312 Hz** | **12255,229 Hz** |
| **6064,197 Hz** | **8045,484 Hz** | **12613,341 Hz** |
| **6066,256 Hz** | **8242,332 Hz** | **12819,942 Hz** |
| **6157,253 Hz** | **8351,622 Hz** | **13674,482 Hz** |
| **6215,298 Hz** | **8408,121 Hz** | **13731,322 Hz** |
| **6333,917 Hz** | **8455,894 Hz** | **14525,312 Hz** |
| **6365,242 Hz** | **8551,231 Hz** | **14537,218 Hz** |
| **6558,342 Hz** | **8743,321 Hz** | **14549,331 Hz** |
| **6568,278 Hz** | **8789,631 Hz** | **14845,453 Hz** |
| **6823,194 Hz** | **8868,609 Hz** | **14944,989 Hz** |
| **6853,391 Hz** | **9012,241 Hz** | **15246,315 Hz** |
| **6855,286 Hz** | **9028,994 Hz** | **18668,239 Hz** |
| **7213,204 Hz** | **9131,232 Hz** | **19321,231 Hz** |
| **7228,528 Hz** | **9658,296 Hz** | **19347,208 Hz** |
| **7238,232 Hz** | **9663,495 Hz** | **30182,932 Hz** |
| **7277,921 Hz** | **9680,737 Hz** | |
| **7280,422 Hz** | **9824,442 Hz** | |
10. AM-Frequenzen, die zur Behandlung von Lungenkrebs verwendet werden
| | | |
|---|---|---|
| **304,148 Hz** | **2761,312 Hz** | **3461.322 Hz** |
| **694,7 Hz** | **2784,491 Hz** | **3682,489 Hz** |
| **694,727 Hz** | **2812,443 Hz** | **3727,231 Hz** |
| **708,8 Hz** | **2855,218 Hz** | **3749,882 Hz** |
| **708,841 Hz** | **2859,495 Hz** | **3769,942 Hz** |
| **1587,811 Hz** | **3126,822 Hz** | **4131,235 Hz** |
| **1759,318 Hz** | **3139,297 Hz** | **4158,393 Hz** |
| **1873.477 Hz** | **3193,212 Hz** | **4243,393 Hz** |
| **2263,104 Hz** | **3348,783 Hz** | **4347,733 Hz** |
| **2391,312 Hz** | **3360,971 Hz** | **4373,411 Hz** |
| **2454,232 Hz** | **3368,311 Hz** | **4378,321 Hz** |
| **2729,929 Hz** | **3373,892 Hz** | **4416,221 Hz** |
| **2741,261 Hz** | **3440,212 Hz** | **4481,242 Hz** |
| **4777,521 Hz** | **6838,434 Hz** | **9181,434 Hz** |
| **4798,422 Hz** | **6870,955 Hz** | **9317,913 Hz** |
| **4837,241 Hz** | **6879,216 Hz** | **9363,896 Hz** |
| **4959,842 Hz** | **7079,411 Hz** | **9736,919 Hz** |
| **5013,321 Hz** | **7216,288 Hz** | **9753,321 Hz** |
| **5047,523 Hz** | **7376,089 Hz** | **10424,908 Hz** |
| **5068,322 Hz** | **7761,289 Hz** | **10452,913 Hz** |
| **6371,922 Hz** | **8082,173 Hz** | **10824,609 Hz** |
| **5538,432 Hz** | **8281,259 Hz** | **11656,329 Hz** |
| **5548,879 Hz** | **8352,189 Hz** | **12748,919 Hz** |
| **5679,309 Hz** | **8442,473 Hz** | **15774,291 Hz** |
| **5734,143 Hz** | **8773,916 Hz** | **15798,333 Hz** |
| **5787,342 Hz** | **8935,752 Hz** | **16510,321 Hz** |
| **6445,309 Hz** | **9121,223 Hz** | |
11. AM-Frequenzen, die zur Behandlung von Leiomyosarkom verwendet werden
| | | |
|---|---|---|
| **836,923 Hz** | **4241,321 Hz** | **6651,276 Hz** |
| **843,181 Hz** | **4266,591 Hz** | **7168,892 Hz** |
| **1411,241 Hz** | **4337,322 Hz** | **7406,309 Hz** |
| **2073,721 Hz** | **4424,112 Hz** | **7452,528 Hz** |
| **2381,443 Hz** | **4436,111 Hz** | **7649,209 Hz** |
| **2711,019 Hz** | **4485,22 Hz** | **7808,352 Hz** |
| **2911,329 Hz** | **5545,521 Hz** | **9040,313 Hz** |
| **3232,185 Hz** | **5577,841 Hz** | **9074.294 Hz** |
| **3618,321 Hz** | **5631,422 Hz** | **9189,092 Hz** |
| **3544,209 Hz** | **5696,184 Hz** | **9484,512 Hz** |
| **3569,219 Hz** | **6472,098 Hz** | **9943,972 Hz** |
| **4233,822 Hz** | **6558,342 Hz** | **12088,394 Hz** |
12. AM-Frequenzen, die zur Behandlung von Mesotheliom verwendet werden
| | | |
|---|---|---|
| **958,929 Hz** | **3319,945 Hz** | **6516,793 Hz** |
| **1713,913 Hz** | **3449,219 Hz** | **7224,197 Hz** |
| **1736,782 Hz** | **3622,312 Hz** | **9471,152 Hz** |
| **2334,178 Hz** | **5151,402 Hz** | **14617,393 Hz** |
| **2607,193 Hz** | **5887,022 Hz** | |
| **3112,974 Hz** | **5965,922 Hz** | |
13. AM-Frequenzen, die zur Behandlung von Neuroendokiinium verwendet werden
| | | |
|---|---|---|
| **1766,335 Hz** | **3440,212 Hz** | **6291,631 Hz** |
| **2408,22 Hz** | **3533,328 Hz** | **6406,891 Hz** |
| **2441,502 Hz** | **3666,283 Hz** | **6780,679 Hz** |
| **2647,938 Hz** | **4079.282 Hz** | **7151,264 Hz** |
| **2741,261 Hz** | **4243,393 Hz** | **7482,245 Hz** |
| **3020,212 Hz** | **4426,387 Hz** | **1575,393 Hz** |
| **3128,822 Hz** | **5245,818 Hz** | **8359,932 Hz** |
| **3238,742 Hz** | **5536,242 Hz** | **9073,418 Hz** |
| **3296,431 Hz** | **5548,879 Hz** | |
| **3348,793 Hz** | **5739,422 Hz** | |
| **3360,971 Hz** | **5849,241 Hz** | |
14. AM-Frequenzen, die zur Behandlung von Leukämie und chronischem Lymphsystemkrebs verwendet werden
| | | |
|---|---|---|
| **814,413 Hz** | **3361,671 Hz** | **7629,318 Hz** |
| **825,145 Hz** | **5245,452 MHz** | **8172,405 Hz** |
| **2415,243 Hz** | **5557,333 Hz** | **8272,338 Hz** |
| **2436,316 Hz** | **6850,197 Hz** | **8438,483 Hz** |
| **2874,432 Hz** | **6919,322 MHz** | **12950,331 Hz** |
| **2891,029 Hz** | **7587,224 Hz** | |
15. AM-Frequenzen, die zur Behandlung von Myelom, multiplem Krebs verwendet werden
| | | |
|---|---|---|
| **765,196 Hz** | **2883,618 Hz** | **5249,331 Hz** |
| **2336,238 Hz** | **2919,273 Hz** | **7967,311 Hz** |
| **2372,122 Hz** | **3265,323 Hz** | **7973.125 Hz** |
| **2381,443 Hz** | **3564,455 Hz** | **8049,952 Hz** |
| **2425,394 Hz** | **3580,25 Hz** | **8283,329 Hz** |
| **2656,339 Hz** | **3584.291 Hz** | **10351,323 Hz** |
| **2741,261 Hz** | **3674,292 Hz** | |
16. AM-Frequenzen, die zur Behandlung der Hodgkin-Krankheit verwendet werden
| | | |
|---|---|---|
| **752,5 Hz** | **3371,216 Hz** | **5724,231 Hz** |
| **976,3 Hz** | **3605,432 Hz** | **6358,194 Hz** |
| **1558,223 Hz** | **3623,198 Hz** | **7472,211 Hz** |
| **2310,912 Hz** | **3838,281 Hz** | **8062,121 Hz** |
| **2477,919 Hz** | **3838,48 Hz** | **8222,222 Hz** |
| **2560,843 Hz** | **5102 Hz** | |
| **3348,783 Hz** | **5696,932 Hz** | |
17. AM-Frequenzen, die zur Behandlung von Gehirntumoren verwendet werden
| | | |
|---|---|---|
| **1372,934 Hz** | **4318,222 Hz** | **6943,386 Hz** |
| **2318,182 Hz** | **4334,33 Hz** | **7151,264 Hz** |
| **2381,443 Hz** | **4358,333 Hz** | **7182,922 Hz** |
| **2425,394 Hz** | **4393,419 Hz** | **7194,897 Hz** |
| **2442,423 Hz** | **4454,194 Hz** | **7323,209 Hz** |
| **2478,973 Hz** | **4515,789 Hz** | **7390,343 Hz** |
| **2654,513 Hz** | **4619,324 Hz** | **7790,221 Hz** |
| **2661,324 Hz** | **4723,937 Hz** | **7961,122 Hz** |
| **2686,105 Hz** | **4853,286 Hz** | **8128,942 Hz** |
| **2690,179 Hz** | **5289,231 Hz** | **8245,109 Hz** |
| **3249,332 Hz** | **5378,099 Hz** | **8272,281 Hz** |
| **3277,509 Hz** | **5426,323 Hz** | **8358,154 Hz** |
| **3335,279 Hz** | **5640,981 Hz** | **8408,121 Hz** |
| **3348,783 Hz** | **6316,211 Hz** | **9138,82 Hz** |
| **3436,211 Hz** | **6459,203 Hz** | **10719,318 Hz** |
| **3916,321 Hz** | **6474,332 Hz** | **11558,241 Hz** |
| **4031,933 Hz** | **6626,572 Hz** | **12828,633 Hz** |
| **4086,091 Hz** | **6855,286 Hz** | **14515,962 Hz** |
| **4241,321 Hz** | **6915,886 Hz** | **14586,765 Hz** |

2. System (11) nach Anspruch 1, wobei die Frequenz der generierten einen oder mehreren Amplitudenmodulation/en auf innerhalb eine Genauigkeit von 100 ppm in Bezug auf die eine oder mehreren bestimmte/n oder vorbestimmte/n Referenzamplitudenmodulationsfrequenz/en kontrollierbar ist.

3. System (11) nach Anspruch 2, wobei die Frequenz der generierten einen oder mehreren Amplitudenmodulation/en auf innerhalb eine Genauigkeit von 10 ppm in Bezug auf die eine oder mehreren bestimmte/n oder vorbestimmte/n Referenzamplitudenmodulationsfrequenz/en kontrollierbar ist.

4. System (11) nach Anspruch 3, wobei die Frequenz der generierten einen oder mehreren Amplitudenmodulation/en auf innerhalb eine Genauigkeit von ca. 1 ppm in Bezug auf die eine oder mehreren bestimmte/n oder vorbestimmte/n Referenzamplitudenmodulationsfrequenz/en kontrollierbar ist.

5. System (11) nach einem der vorhergehenden Ansprüche, wobei die generierten, amplitudenmodulierten, niedrigenergetischen elektromagnetischen Emissionen während der Emission auf einer Stabilität von mindestens 10⁻⁵ gehalten werden.

6. System (11) nach Anspruch 5, wobei eine Stabilität von mindestens 10⁻⁶ aufrechterhalten wird.

7. System (11) nach Anspruch 6, wobei eine Stabilität von mindestens 10⁻⁷ aufrechterhalten wird.

8. System (11) nach einem der vorhergehenden Ansprüche, wobei die eine oder die mehreren Generatorschaltung/en (29) durch Amplitudenmodulationskontrollsignale kontrollierbar sind, die dazu führen, dass verschiedene Amplitudenmodulationswellenformen generiert werden.

9. System (11) nach Anspruch 8, wobei die Amplitudenmodulationswellenformen aus Sinus-, Quadrat-, Dreieckswellenformen oder Mehlfachkombinationen von diesen ausgewählt sind.

10. System (11) nach Anspruch 8 oder 9, wobei die eine oder die mehreren Generatorschaltung/en (29) durch Amplitudenmodulationskontrollsignale kontrollierbar ist bzw. sind, die mehrere Amplitudenmodulationswellenformen generieren, entweder sequentiell oder gleichzeitig.

11. System (11) nach einem der vorhergehenden Ansprüche, wobei die durch die eine oder die mehreren Generatorschaltungen (29) zu generierenden Hochfrequenzträgersignale aus einer oder mehreren Frequenz/en ausgewählt sind, die aus ca. 27 MHz, 433 MHz und 900 MHz ausgewählt sind.

12. System (11) nach einem der vorhergehenden Ansprüche, wobei das System (11) eine oder mehrere Schnittstelle/n oder Empfängereinrichtung/en (16) umfasst, die mit dem einen oder den mehreren Datenprozessor/en oder der einen oder den mehreren integrierten Schaltung/en (21) kommuniziert bzw. kommunizieren, und wobei die Kontrollinformation auf die eine oder die mehreren Schnittstelle/n oder Empfängereinrichtung/en (16) und damit auf den einen oder die mehreren Datenprozessor/en oder die eine oder die mehreren integrierte/n Schaltung/en (21) übertragbar ist, um Befehls signale in Reaktion auf empfangene Kontrollinformation zu der einen oder den mehreren Generatorschaltung/en (29) durch den einen oder die mehreren Datenprozessor/en oder die eine oder die mehreren integrierte/n Schaltung/en (21) zu übermitteln.

13. System (11) nach Anspruch 12, wobei die Kontrollinformation übertragbar oder übermittelbar ist über das Telefon, Internet, Funk oder andere Mittel an den einen oder die mehreren Datenprozessor/en oder die eine oder die mehreren integrierte/n Schaltung/en (21) über die eine oder die mehreren Schnittstelle/n oder Empfängereinrichtung/en (16), die mit dem einen oder den mehreren Datenprozessor/en oder der einen oder den mehreren integrierten Schaltung/en (21) kommuniziert bzw. kommunizieren.

14. System (11) nach Anspruch 12 oder 13, wobei die Kontrollinformation in einer Informationsspeichervorrichtung (52) gespeichert ist, und wobei die Kontrollinformation an den einen oder die mehreren Datenprozessor/en oder die eine oder die mehreren integrierte/n Schaltung/en (21) über die eine oder die mehreren Schnittstelle/n (16) übertragbar ist, die mit dem einen oder den mehreren Datenprozessor/en oder der einen oder den mehreren integrierten Schaltung/en (21) kommuniziert bzw. kommunizieren.

15. System (11) nach einem der vorhergehenden Ansprüche, wobei das System eine Nutzeridentifikationsvorrichtung (21a) umfasst, die mit mindestens einem/einer der einen oder mehreren Datenprozessor/en oder integrierten Schaltung/en (21) kommuniziert, wodurch es ermöglicht wird, dass das System (11) nur zum Gebrauch durch den Nutzer aktiviert oder verwendet wird.

16. System (11) nach einem der vorhergehenden Ansprüche, das einen Überwachungssoftware umfassenden Monitor (54) zum Überwachen der Amplitude und Amplitudenmodulationsfrequenz der amplitudenmodulierten, niedrigenergetischen, elektromagnetischen Emissionen umfasst, die durch die eine oder die mehreren Generatorschaltung/en (29) generiert werden.

17. System (11) nach einem der vorhergehenden Ansprüche, wobei die bestimmte oder vorbestimmte Amplitudenmodulationsfrequenzkontrollinformation mittels eines Biofeedback-Prozesses bestimmt oder vorbestimmt ist, der Beobachtungen oder Messungen physiologischer Reaktionen des Lebewesens in oder während der Zeit einschließt, in der Zellfunktionen des Lebewesens angeregt werden, indem das Lebewesen Emissionen von Hochfrequenzträgersignalen ausgesetzt wird, die mit einer Reihe von Amplitudenmodulationsfrequenzen amplitudenmoduliert sind.

18. System (11) nach Anspruch 17, wobei die bestimmten oder vorbestimmten Frequenzen als ein Modus zum Identifizieren der Art eines Tumors oder Krebses verwendet wird, den ein warmblütiges Säugetierlebewesen hat.

## Revendications

1. Système électronique (11) activable par énergie électrique, destiné à être utilisé dans l'inhibition de la croissance de cellules cancéreuses ou de la prolifération de celles-ci chez un sujet mammifère à sang chaud, comprenant:
un ou plusieurs circuits générateurs d'énergie électromagnétique à basse énergie contrôlables (29) destinés à générer un ou plusieurs signaux porteurs à haute fréquence,
un ou plusieurs processeurs de données ou circuits intégrés (21) comprenant ou communiquant avec l'un ou les plusieurs circuits générateurs (29) et destinés à recevoir des informations de contrôle depuis une source d'informations de contrôle étant un dispositif de stockage d'application ou un ordinateur (52),
ledit un ou lesdits plusieurs circuits générateurs (29) incluant un ou plusieurs générateurs de signal de contrôle de modulation d'amplitude (34) destinés à contrôler des variations modulées en amplitude de l'un ou de plusieurs signaux porteurs à haute fréquence,
ledit un ou lesdits plusieurs circuits générateurs (29) incluant en outre un ou plusieurs générateurs de signal de contrôle de fréquence de modulation d'amplitude programmables (31) destinés à contrôler la fréquence à laquelle les modulations d'amplitude sont générées, le système (11) comprenant en outre une position de connexion ou de couplage (12a) pour la connexion à un applicateur électriquement conducteur (12, 13),
ledit un ou lesdits plusieurs générateurs de contrôle de fréquence de modulation d'amplitude programmables (31) sont aptes à contrôler précisément la fréquence de modulations d'amplitude avec une précision maximum d'au moins 1000 ppm par rapport à une ou plusieurs fréquences de modulation d'amplitude de référence déterminées ou prédéterminées sélectionnées dans une plage de 0,01 Hz à 150 kHz,
et **caractérisé en ce que**
ladite source d'informations de contrôle (52) inclut des informations de contrôle de fréquence de modulation d'amplitude de référence qui comprennent au moins 50 % des fréquences de modulation d'amplitude de référence définies précisément, répertoriées dans l'un quelconque des points 1 à 17 ci-dessous,
l'applicateur (12, 13) étant apte à appliquer par contact ou par induction de proximité au sujet mammifère à sang chaud une ou plusieurs émissions à basse énergie modulées en amplitude à une fréquence contrôlée par programme entraînant de bas niveaux d'absorption, sensiblement inférieurs à 1,6 milliW/g en poids de tissu vivant,
et dans lequel l'amplitude est modulée par application séquentielle ou simultanée d'au moins 50 % des fréquences de modulation d'amplitude de référence.
1. Fréquences AM utilisées pour le traitement du cancer du sein c
| | | |
|---|---|---|
| **78,76 Hz** | **3115,188 Hz** | **4969,224 Hz** |
| **181,821 Hz** | **3249,529 Hz** | **4979,321 Hz** |
| **414,817 Hz** | **3405,182 Hz** | **5027,231 Hz** |
| **440,933 Hz** | **3432,274 Hz** | **5059,762 Hz** |
| **628,431 Hz** | **3434,693 Hz** | **5118,094 Hz** |
| **721,313 Hz** | **3594,231 Hz** | **5176,287 Hz** |
| **813,205 Hz** | **3647,619 Hz** | **5365,222 Hz** |
| **818,342 Hz** | **3742,957 Hz** | **5376,392 Hz** |
| **891,901 Hz** | **3753,382 Hz** | **5426,323 Hz** |
| **929,095 Hz** | **3830,732 Hz** | **5431,542 Hz** |
| **929,1 Hz** | **3855,823 Hz** | **5521,621 Hz** |
| **1021 Hz** | **3916.321 Hz** | **5739,422 Hz** |
| **1372,207 Hz** | **3935,218 Hz** | **5745,218 Hz** |
| **1372,934 Hz** | **3975,383 Hz** | **5821,975 Hz** |
| **1588,721 Hz** | **3993,437 Hz** | **6037,432 Hz** |
| **1670,699 Hz** | **4153,192 Hz** | **6044,333 Hz** |
| **1821,729 Hz** | **4194,968 Hz** | **6086,256 Hz** |
| **1836,219 Hz** | **4241,321 Hz** | **6208,932 Hz** |
| **2193,937 Hz** | **4243,393 Hz** | **6212,808 Hz** |
| **2221,323 Hz** | **4253,432 Hz** | **6231,031 Hz** |
| **2278,312 Hz** | **4314,444 Hz** | **6280,321 Hz** |
| **2357,832 Hz** | **4318,222 Hz** | **6329.391 Hz** |
| **2381,443 Hz** | **4375,962 Hz** | **6476.896 Hz** |
| **2417,323 Hz** | **4393,419 Hz** | **6497,319 Hz** |
| **2431,334 Hz** | **4417,243 Hz** | **6504,983 Hz** |
| **2450,332 Hz** | **4481,463 Hz** | **6651,276 Hz** |
| **2551,313 Hz** | **4482,223 Hz** | **6757,901 Hz** |
| **2556,221 Hz** | **4495,138 Hz** | **6758,321 Hz** |
| **2598.853 Hz** | **4549,808 Hz** | **6855,286 Hz** |
| **2621,322 Hz** | **4558,306 Hz** | **6858,121 Hz** |
| **2740,191 Hz** | **4779,451 Hz** | **6898,489 Hz** |
| **2851,347 Hz** | **4838,674 Hz** | **7092,219 Hz** |
| **2885,322 Hz** | **4871,513 Hz** | **7120,218 Hz** |
| **2919,273 Hz** | **4895,296 Hz** | **7127,311 Hz** |
| **3074,333 Hz** | **4962,213 Hz** | **7156,489 Hz** |
| **7208,821 Hz** | **8548,324 Hz** | **11525,121 Hz** |
| **7282,169 Hz** | **8749,383 Hz** | **11541,915 Hz** |
| **7376,329 Hz** | **8782,421 Hz** | **11812,328 Hz** |
| **7488,742 Hz** | **8784,424 Hz** | **11812,419 Hz** |
| **7541,319 Hz** | **8923,1 Hz** | **11840,323 Hz** |
| **7577,421 Hz** | **8923,361 Hz** | **11925,089 Hz** |
| **7621,085 Hz** | **8935,752 Hz** | **12123,281 Hz** |
| **7627,207 Hz** | **8936,1 Hz** | **12267,281 Hz** |
| **7650,939 Hz** | **9012,282 Hz** | **12294,283 Hz** |
| **7691,212 Hz** | **9012,896 Hz** | **12611,288 Hz** |
| **7842,184 Hz** | **9060,323 Hz** | **12629,222 Hz** |
| **7849,231 Hz** | **9072,409 Hz** | **12633,372 Hz** |
| **7915,423 Hz** | **9131,419 Hz** | **12648,221 Hz** |
| **7932,482 Hz** | **9199,232 Hz** | **13315,335 Hz** |
| **7949,196 Hz** | **9245,927 Hz** | **13331,358 Hz** |
| **7967,311 Hz** | **9270,322 Hz** | **13735,241 Hz** |
| **8021,229 Hz** | **9279,193 Hz** | **13826,325 Hz** |
| **8070,181 Hz** | **9393,946 Hz** | **13853,232 Hz** |
| **8114,032 Hz** | **10227,242 Hz** | **13990,123 Hz** |
| **8149,922 Hz** | **10340,509 Hz** | **14122,942 Hz** |
| **8194.19 Hz** | **10363,313 Hz** | **14162,332 Hz** |
| **8245,801 Hz** | **10449,323 Hz** | **14519,232 Hz** |
| **8328,322 Hz** | **10456,383 Hz** | **14543,128 Hz** |
| **8330,534 Hz** | **10468,231 Hz** | **15651,323 Hz** |
| **8355,987 Hz** | **10470,456 Hz** | **17352,085 Hz** |
| **8408,121 Hz** | **10472,291 Hz** | **18785,463 Hz** |
| **8431,184 Hz** | **10689,339 Hz** | **30182,932 Hz** |
| **8452,119 Hz** | **10832,222 Hz** | |
2. Fréquences AM utilisées pour le traitement du cancer du foie (carcinome hépatocellulaire)
| | | |
|---|---|---|
| **423,321 Hz** | **1250,504 Hz** | **2353.478 Hz** |
| **427,062 Hz** | **1755,402 Hz** | **2362,309 Hz** |
| **470,181 Hz** | **1873,477 Hz** | **2419,309 Hz** |
| **560,32 Hz** | **1924,702 Hz** | **2425,222 Hz** |
| **642,932 Hz** | **1975,196 Hz** | **2430,219 Hz** |
| **668,209 Hz** | **2017,982 Hz** | **2431,094 Hz** |
| **677,972 Hz** | **2083,419 Hz** | **2471,328 Hz** |
| **811,924 Hz** | **2190,731 Hz** | **2478,331 Hz** |
| **842,311 Hz** | **2221,323 Hz** | **2743,995 Hz** |
| **843,22 Hz** | **2324,393 Hz** | **2744,211 Hz** |
| **2831,951 Hz** | **5520,218 Hz** | **8542,311 Hz** |
| **2843,283 Hz** | **5882,292 Hz** | **8818,104 Hz** |
| **2859,891 Hz** | **5926,512 Hz** | **8852,329 Hz** |
| **2873,542 Hz** | **6037,311 Hz** | **8853,444 Hz** |
| **2886,232 Hz** | **6180,334 Hz** | **8858,179 Hz** |
| **3042,012 Hz** | **6329,195 Hz** | **8939,212 Hz** |
| **3078,983 Hz** | **6350,333 Hz** | **9332,397 Hz** |
| **3086,443 Hz** | **6361,321 Hz** | **9381,221 Hz** |
| **3127,232 Hz** | **6364,928 Hz** | **9740,219 Hz** |
| **3160,942 Hz** | **8383,321 Hz** | **9768,331 Hz** |
| **3206,315 Hz** | **6461,175 Hz** | **9797,294 Hz** |
| **3267,433 Hz** | **6733,331 Hz** | **10317,499 Hz** |
| **3269,321 Hz** | **6758,232 Hz** | **10443,311 Hz** |
| **3457,291 Hz** | **6779,482 Hz** | **10456,383 Hz** |
| **3505,229 Hz** | **6856,222 Hz** | **10579,425 Hz** |
| **3516,296 Hz** | **6877,183 Hz** | **10863,209 Hz** |
| **3531,296 Hz** | **6980,525 Hz** | **10866,362 Hz** |
| **3546,323 Hz** | **7019,235 Hz** | **11067,418 Hz** |
| **3572,106 Hz** | **7043,209 Hz** | **11149,935 Hz** |
| **3516,189 Hz** | **7130,323 Hz** | **11163,895 Hz** |
| **3669,513 Hz** | **7144,142 Hz** | **11802,821 Hz** |
| **3923,221 Hz** | **7210,223 Hz** | **11953,424 Hz** |
| **4013,932 Hz** | **7291,21 Hz** | **12223,329 Hz** |
| **4071,121 Hz** | **7510,92 Hz** | **12265,295 Hz** |
| **4079,951 Hz** | **7529,233 Hz** | **12267,233 Hz** |
| **4222,821 Hz** | **7549,212 Hz** | **12623,191 Hz** |
| **4238,402 Hz** | **7650,028 Hz** | **12685,231 Hz** |
| **4256,321 Hz** | **7680,518 Hz** | **12721,423 Hz** |
| **4289,296 Hz** | **7692.522 Hz** | **12785,342 Hz** |
| **4312,947 Hz** | **7829,231 Hz** | **14085,222 Hz** |
| **4435,219 Hz** | **7862,209 Hz** | **14333,209 Hz** |
| **4471,188 Hz** | **7947,392 Hz** | **14537,331 Hz** |
| **4483,889 Hz** | **7979,308 Hz** | **14542,432 Hz** |
| **4486,384 Hz** | **8028,339 Hz** | **14655,03 Hz** |
| **4629,941 Hz** | **8055,942 Hz** | **14828,234 Hz** |
| **4732,211 Hz** | **8072,134 Hz** | **15149,213 Hz** |
| **4876,218 Hz** | **8141,174 Hz** | **15237,489 Hz** |
| **5086,281 Hz** | **8336,383 Hz** | **16110,932 Hz** |
| **5124,084 Hz** | **8432,181 Hz** | **16144,343 Hz** |
| **5133,121 Hz** | **8452,119 Hz** | **18265,238 Hz** |
| **5247,142 Hz** | **8460,944 Hz** | **18283,323 Hz** |
| **5270,834 Hz** | **8475,221 Hz** | **18863,292 Hz** |
| **5340,497 Hz** | **8492,193 Hz** | **18930,995 Hz** |
| **19970,311 Hz** | **20330,294 Hz** | **20365,284 Hz** |
3. Fréquences AM utilisées pour le traitement du cancer de l'ovaire
| | | |
|---|---|---|
| **78,76 Hz** | **1372,207 Hz** | **2812,321 Hz** |
| **181,821 Hz** | **1396,498 Hz** | **2831,386 Hz** |
| **410,245 Hz** | **1502,181 Hz** | **2835,332 Hz** |
| **414,817 Hz** | **1518,208 Hz** | **2851,347 Hz** |
| **436,332 Hz** | **1552,123 Hz** | **2877, 192 Hz** |
| **447,942 Hz** | **1579,212 Hz** | **2885,322 Hz** |
| **481,191 Hz** | **1624,802 Hz** | **2687,385 Hz** |
| **489,292 Hz** | **1670,699 Hz** | **2894.972 Hz** |
| **559,292 Hz** | **1696,403 Hz** | **2973,771 Hz** |
| **606,321 Hz** | **1762,938 Hz** | **3080,592 Hz** |
| **655,435 Hz** | **1771,402 Hz** | **3157,483 Hz** |
| **657,397 Hz** | **1775,313 Hz** | **3161,465 Hz** |
| **657,483 Hz** | **1821,729 Hz** | **3223,232 Hz** |
| **664,211 Hz** | **2016,323 Hz** | **3238,148 Hz** |
| **708,8 Hz** | **2034,231 Hz** | **3249,529 Hz** |
| **708,822 Hz** | **2050,282 Hz** | **3262,145 Hz** |
| **734,921 Hz** | **2053,396 Hz** | **3314.321 Hz** |
| **749,221 Hz** | **2082,234 Hz** | **3361,671 Hz** |
| **764,232 Hz** | **2089,092 Hz** | **3366,311 Hz** |
| **778,295 Hz** | **2221,323 Hz** | **3523,215 Hz** |
| **779,403 Hz** | **2228,832 Hz** | **3527,233 Hz** |
| **806,021 Hz** | **2253,704 Hz** | **3542,213 Hz** |
| **806,389 Hz** | **2254,329 Hz** | **3590,376 Hz** |
| **809,313 Hz** | **2278,312 Hz** | **3629,232 Hz** |
| **824,327 Hz** | **2332,949 Hz** | **3632,793 Hz** |
| **825,145 Hz** | **2348,233 Hz** | **3636,289 Hz** |
| **835,129 Hz** | **2381,443 Hz** | **3637,085 Hz** |
| **839,521 Hz** | **2413,193 Hz** | **3669.513 Hz** |
| **841,208 Hz** | **2425,222 Hz** | **3770,189 Hz** |
| **843,312 Hz** | **2433,321 Hz** | **3858,916 Hz** |
| **956,984 Hz** | **2439,253 Hz** | **3919,232 Hz** |
| **958,929 Hz** | **2465,23 Hz** | **3957,185 Hz** |
| **985.313 Hz** | **2477,919 Hz** | **3975,228 Hz** |
| **1024,208 Hz** | **2669,177 Hz** | **4061,131 Hz** |
| **1102.635 Hz** | **2715,232 Hz** | **4072,322 Hz** |
| **1121,329 Hz** | **2733,843 Hz** | **4169,451 Hz** |
| **1159,738 Hz** | **2802,339 Hz** | **4174,259 Hz** |
| **4241,321 Hz** | **6350,333 Hz** | **8428,313 Hz** |
| **4243,393 Hz** | **6406,891 Hz** | **8435,451 Hz** |
| **4261,228 Hz** | **6407,207 Hz** | **8486,421 Hz** |
| **4279,113 Hz** | **6450,787 Hz** | **8492,797 Hz** |
| **4309,335 Hz** | **6477,098 Hz** | **8548,324 Hz** |
| **4314,188 Hz** | **6477,929 Hz** | **8554,361 Hz** |
| **4318,222 Hz** | **6478,338 Hz** | **8562,965 Hz** |
| **4328,928 Hz** | **6543,421 Hz** | **8579,323 Hz** |
| **4380,321 Hz** | **6552,24 Hz** | **8579,333 Hz** |
| **4394,134 Hz** | **6663,955 Hz** | **8642,181 Hz** |
| **4412,252 Hz** | **6753,338 Hz** | **8655,818 Hz** |
| **4424,236 Hz** | **6851,323 Hz** | **8758,341 Hz** |
| **4439,341 Hz** | **6855,286 Hz** | **8779,323 Hz** |
| **4442,161 Hz** | **6875,232 Hz** | **8792,231 Hz** |
| **4447,221 Hz** | **6882,949 Hz** | **8819,127 Hz** |
| **4458,339 Hz** | **7206,403 Hz** | **8831,132 Hz** |
| **4556,322 Hz** | **7232,214 Hz** | **9028,031 Hz** |
| **4566,009 Hz** | **7257,489 Hz** | **9173,264 Hz** |
| **4682,643 Hz** | **7276,209 Hz** | **9184,338 Hz** |
| **4718,331 Hz** | **7281,219 Hz** | **9186,919 Hz** |
| **4749,302 Hz** | **7285,693 Hz** | **9393,946 Hz** |
| **4765,331 Hz** | **7429,212 Hz** | **9482,409 Hz** |
| **4917,202 Hz** | **7480,932 Hz** | **9737,211 Hz** |
| **5011,325 Hz** | **7480,228 Hz** | **9746,232 Hz** |
| **5149,331 Hz** | **7495,763 Hz** | **9922,231 Hz** |
| **5228,172 Hz** | **7539,432 Hz** | **10032,684 Hz** |
| **5237,132 Hz** | **7564,185 Hz** | **10446,028 Hz** |
| **5313,353 Hz** | **7650,028 Hz** | **10478.221 Hz** |
| **5745,218 Hz** | **7689,728 Hz** | **10545,313 Hz** |
| **5757,897 Hz** | **7780,294 Hz** | **10639,345 Hz** |
| **5762,386 Hz** | **8021,921 Hz** | **10743,118 Hz** |
| **5812,322 Hz** | **8038,961 Hz** | **10813,981 Hz** |
| **5869,321 Hz** | **8040,322 Hz** | **10832,421 Hz** |
| **5882,292 Hz** | **8044,233 Hz** | **10838,243 Hz** |
| **5921,249 Hz** | **8095,313 Hz** | **10862,429 Hz** |
| **5991,932 Hz** | **8143,491 Hz** | **10865,127 Hz** |
| **6069,458 Hz** | **8164.332 Hz** | **10917,229 Hz** |
| **6071,319 Hz** | **8261,121 Hz** | **10977,188 Hz** |
| **6083,214 Hz** | **8302,285 Hz** | **11120,209 Hz** |
| **6161,782 Hz** | **8309,752 Hz** | **11177,289 Hz** |
| **6169,341 Hz** | **8372,532 Hz** | **11177,409 Hz** |
| **6275,232 Hz** | **8408,121 Hz** | **11321,491 Hz** |
| **6294.929 Hz** | **8424,229 Hz** | **11359.093 Hz** |
| **11673,031 Hz** | **12755,333 Hz** | **15450,183 Hz** |
| **11793,886 Hz** | **12947,311 Hz** | **16144,343 Hz** |
| **11895,229 Hz** | **13717,221 Hz** | **17932,432 Hz** |
| **12074,531 Hz** | **13825,295 Hz** | **17951,395 Hz** |
| **12216,212 Hz** | **13829,195 Hz** | **17970,122 Hz** |
| **12263,329 Hz** | **14410,949 Hz** | **18337,222 Hz** |
| **12260,933 Hz** | **14436,201 Hz** | **18378,321 Hz** |
| **12262,853 Hz** | **14537,218 Hz** | **18921,415 Hz** |
| **12292,222 Hz** | **14947,184 Hz** | **18926,951 Hz** |
| **12357,353 Hz** | **15429,139 Hz** | **18931,327 Hz** |
| **12527,032 Hz** | **15443,309 Hz** | **114508,332 Hz** |
4. Fréquences AM utilisées pour le traitement du cancer de la prostate
| | | |
|---|---|---|
| **331.3 Hz** | **809,205 Hz** | **2628,324 Hz** |
| **331,358 Hz** | **819,322 Hz** | **2668,328 Hz** |
| **403,218 Hz** | **844,8 Hz** | **2824,832 Hz** |
| **461,233 Hz** | **844,822 Hz** | **2887,829 Hz** |
| **522,2 Hz** | **847,332 Hz** | **2891,331 Hz** |
| **522,213 Hz** | **1083,309 Hz** | **3081,523 Hz** |
| **618,4 Hz** | **1102,635 Hz** | **3249,529 Hz** |
| **618,407 Hz** | **1102,71 Hz** | **3250,125 Hz** |
| **618,8 Hz** | **1240,336 Hz** | **3251,815 Hz** |
| **656,295 Hz** | **1372,934 Hz** | **3264,827 Hz** |
| **657,394 Hz** | **1444,288 Hz** | **3278,329 Hz** |
| **657,397 Hz** | **1486,322 Hz** | **3281,432 Hz** |
| **657,4 Hz** | **1563,332 Hz** | **3348,783 Hz** |
| **657,483 Hz** | **1591,322 Hz** | **3519,118 Hz** |
| **659,033 Hz** | **1670,699 Hz** | **3539,962 Hz** |
| **694,4 Hz** | **1697,321 Hz** | **3551.318 Hz** |
| **694,689 Hz** | **1743,521 Hz** | **3556,439 Hz** |
| **694.7 Hz** | **2031,448 Hz** | **3572,321 Hz** |
| **741,4 Hz** | **2050,282 Hz** | **3670,129 Hz** |
| **741,421 Hz** | **2076,519 Hz** | **3681,341 Hz** |
| **749,221 Hz** | **2156,332 Hz** | **3686.021 Hz** |
| **752,9 Hz** | **2229,515 Hz** | **3753,382 Hz** |
| **752,933 Hz** | **2243,121 Hz** | **3774,923 Hz** |
| **776,194 Hz** | **2381,443 Hz** | **3867,692 Hz** |
| **785,219 Hz** | **2440,489 Hz** | **3909,333 Hz** |
| **786,332 Hz** | **2475,912 Hz** | **3916,321 Hz** |
| **793,331 Hz** | **2477,919 Hz** | **4031,233 Hz** |
| **4031,933 Hz** | **6590,328 Hz** | **9018,233 Hz** |
| **4038,203 Hz** | **6651,276 Hz** | **9068,231 Hz** |
| **4081,743 Hz** | **6706,431 Hz** | **9137,232 Hz** |
| **4084,319 Hz** | **6743,322 Hz** | **9156.321 Hz** |
| **4139,322 Hz** | **6783,282 Hz** | **9351.931 Hz** |
| **4153,192 Hz** | **6850,197 Hz** | **9393,946 Hz** |
| **4223,795 Hz** | **6855,286 Hz** | **9694,179 Hz** |
| **4231,221 Hz** | **6864,896 Hz** | **9984,405 Hz** |
| **4241,321 Hz** | **6871,943 Hz** | **10226,223 Hz** |
| **4320,513 Hz** | **6973,393 Hz** | **10390,232 Hz** |
| **4329,152 Hz** | **7120,932 Hz** | **10514,168 Hz** |
| **4380,321 Hz** | **7146,509 Hz** | **10689,339 Hz** |
| **4417.312 Hz** | **7192.505 Hz** | **10772,419 Hz** |
| **4489,452 Hz** | **7251,309 Hz** | **10818,452 Hz** |
| **4549,808 Hz** | **7251,322 Hz** | **11165,239 Hz** |
| **4558,306 Hz** | **7278,124 Hz** | **11985,353 Hz** |
| **4638,293 Hz** | **7279,335 Hz** | **12209,329 Hz** |
| **4740,322 Hz** | **7299,119 Hz** | **12308,321 Hz** |
| **4854,318 Hz** | **7527,229 Hz** | **12583,339 Hz** |
| **4882,322 Hz** | **7589,925 Hz** | **13620,329 Hz** |
| **4978,822 Hz** | **7699,193 Hz** | **14013,123 Hz** |
| **5237,152 Hz** | **7842,184 Hz** | **14171,434 Hz** |
| **5264,222 Hz** | **8023,32 Hz** | **14681.329 Hz** |
| **5289,195 Hz** | **8096,939 Hz** | **14759,131 Hz** |
| **5426,323 Hz** | **8245,801 Hz** | **14986,794 Hz** |
| **5431,542 Hz** | **8315,291 Hz** | **15930,249 Hz** |
| **5455,593 Hz** | **8357,305 Hz** | **16026,623 Hz** |
| **6345,332 Hz** | **8408,121 Hz** | **17880,954 Hz** |
| **6347,433 Hz** | **8432,209 Hz** | **18247,532 Hz** |
| **6363,284 Hz** | **8535,238 Hz** | **18282,211 Hz** |
| **6418,331 Hz** | **8552,431 Hz** | **18629,328 Hz** |
| **6496,231 Hz** | **8585,224 Hz** | **19469,318 Hz** |
| **6538,295 Hz** | **8935,752 Hz** | **19766,218 Hz** |
| **6577,421 Hz** | **9015,253 Hz** | **60317,352 Hz** |
5. Fréquences AM utilisées pour le traitement du cancer du rein
| | | |
|---|---|---|
| **628,321 Hz** | **826,321 Hz** | **2254,329 Hz** |
| **631,141 Hz** | **1372,934 Hz** | **3555,209 Hz** |
| **643,312 Hz** | **2082,241 Hz** | **3928,343 Hz** |
| **812,512 Hz** | **2156,931 Hz** | **4420,932 Hz** |
| **4819,228 Hz** | **8727,224 Hz** | **11421,933 Hz** |
| **4828,321 Hz** | **8760,983 Hz** | **11523,212 Hz** |
| **5314,322 Hz** | **8831,132 Hz** | **11561,221 Hz** |
| **6007,332 Hz** | **8870,228 Hz** | **11846,212 Mz** |
| **7054,279 Hz** | **10565,321 Hz** | **12631,331 Hz** |
| **7074,429 Hz** | **10386,229 Hz** | **12693,272 Hz** |
| **7254,343 Hz** | **10634,293 Hz** | **14411,321 Hz** |
| **8041,289 Hz** | **10687,949 Hz** | **20178,941 Hz** |
6. Fréquences AM utilisées pour le traitement du cancer de la thyroïde
| | | |
|---|---|---|
| **493.442 Hz** | **3284,192 Hz** | **6145,333 Hz** |
| **517,202 Hz** | **3335,332 Hz** | **6766,281 Hz** |
| **618,927 Hz** | **3434,911 Hz** | **6884,432 Hz** |
| **621,321 Hz** | **3440,212 Hz** | **7036,122 Hz** |
| **648,252 Hz** | **3475,216 Hz** | **7230,838 Hz** |
| **663,407 Hz** | **3509,522 Hz** | **7323,209 Hz** |
| **821,202 Hz** | **3533,328 Hz** | **7355,378 Hz** |
| **874,341 Hz** | **3637,085 Hz** | **7432,143 Hz** |
| **914,429 Hz** | **3682,489 Hz** | **7534,221 Hz** |
| **941,311 Hz** | **4154,301 Hz** | **7623,184 Hz** |
| **983,429 Hz** | **4243,393 Hz** | **7725,339 Hz** |
| **1587,811 Hz** | **4261,228 Hz** | **7920,879 Hz** |
| **1723.389 Hz** | **4330,289 Hz** | **8013,953 Hz** |
| **2179.231 Hz** | **4340,833 Hz** | **8019.912 Hz** |
| **2315,888 Hz** | **4358,333 Hz** | **8040,231 Hz** |
| **2341,312 Hz** | **4366,294 Hz** | **8078,955 Hz** |
| **2445,123 Hz** | **4426,387 Hz** | **8082,173 Hz** |
| **2454,232 Hz** | **4458,339 Hz** | **8147,1 Hz** |
| **2723,302 Hz** | **4479,113 Hz** | **8281.259 Hz** |
| **2740,384 Hz** | **4744,424 Hz** | **8309,752 Hz** |
| **2749,323 Hz** | **4865,421 Hz** | **8311,371 Hz** |
| **2856,253 Hz** | **5323,192 Hz** | **8435,094 Hz** |
| **2859,495 Hz** | **5324,123 Hz** | **8525.789 Hz** |
| **2886,232 Hz** | **5648,879 Hz** | **8744,527 Hz** |
| **3021,122 Hz** | **5711,283 Hz** | **9009.329 Hz** |
| **3078,275 Hz** | **5754,332 Hz** | **9070,809 Hz** |
| **3080.592 Hz** | **6455,131 Hz** | **10020.521 Hz** |
| **3198,323 Hz** | **6620,132 Hz** | **10039.109 Hz** |
| **3248,321 Hz** | **6666.839 Hz** | **10127,279 Hz** |
| **3271,329 Hz** | **6714,189 Hz** | **10134,161 Hz** |
| **10257,324 Hz** | **12120,049 Hz** | **16023,119 Hz** |
| **10498,339 Hz** | **12139.222 Hz** | **16048,391 Hz** |
| **11537.292 Hz** | **13636,082 Hz** | **17323,196 Hz** |
| **11559,292 Hz** | **13654,272 Hz** | **17577,221 Hz** |
| **11913,222 Hz** | **13677,211 Hz** | **17881,709 Hz** |
| **11927,934 Hz** | **14014,941 Hz** | **17911,323 Hz** |
| **11955,949 Hz** | **14445,214 Hz** | |
7. Fréquences AM utilisées pour le traitement du cancer de la vessie
| | | |
|---|---|---|
| **623,243 Hz** | **3438.109 Hz** | **8235,21 Hz** |
| **757.084 Hz** | **3692,319 Hz** | **8749,232 Hz** |
| **870,4 Hz** | **3952,308 Hz** | **9354,812 Hz** |
| **2454,423 Hz** | **5230,227 Hz** | **12532,729 Hz** |
| **2480,191 Hz** | **6022,942 Hz** | **13467,209 Hz** |
| **2581,101 Hz** | **6061,711 Hz** | **13777,9 Hz** |
| **2715,232 Hz** | **6710,899 Hz** | **14015,241 Hz** |
| **3042,012 Hz** | **6721,912 Hz** | **18524,419 Hz** |
| **3196,194 Hz** | **7181,784 Hz** | |
| **3265,323 Hz** | **7458,209 Hz** | |
8. Fréquences AM utilisées pour le traitement du cancer du colon
| | | |
|---|---|---|
| **78,76 Hz** | **3131,123 Hz** | **4318,222 Hz** |
| **796.562 Hz** | **3161,465 Hz** | **4344,082 Hz** |
| **841,541 Hz** | **3175,313 Hz** | **4416,221 Hz** |
| **842.783 Hz** | **3249,529 Hz** | **4481.242 Hz** |
| **914.429 Hz** | **3363,229 Hz** | **4724,263 Hz** |
| **1162,117 Hz** | **3373,892 Hz** | **4751,319 Hz** |
| **1372,207 Hz** | **3390,925 Hz** | **4755,323 Hz** |
| **1372,934 Hz** | **3409,179 Hz** | **4788,485 Hz** |
| **1716,532 Hz** | **3432.274 Hz** | **5149,331 Hz** |
| **2243,169 Hz** | **3509,522 Hz** | **5217,402 Hz** |
| **2278,312 Hz** | **3531.422 Hz** | **5386,212 Hz** |
| **2286,5 Hz** | **3533,328 Hz** | **5407,192 Hz** |
| **2286,519 Hz** | **3766,296 Hz** | **5426,323 Hz** |
| **2334,178 Hz** | **4040,839 Hz** | **5496,434 Hz** |
| **2423,292 Hz** | **4081,022 Hz** | **5555,212 Hz** |
| **2454,423 Hz** | **4123,953 Hz** | **5572,032 Hz** |
| **2464,229 Hz** | **4146.274 Hz** | **5634,933 Hz** |
| **2598,853 Hz** | **4233,822 Hz** | **5724,231 Hz** |
| **2623,048 Hz** | **4282,332 Hz** | **5758,378 Hz** |
| **5787,342 Hz** | **7368,222 Hz** | **9744,193 Hz** |
| **5948,897 Hz** | **7645,859 Hz** | **9942,321 Hz** |
| **5967,448 Hz** | **7829,234 Hz** | **10301.371 Hz** |
| **5976,825 Hz** | **7866,229 Hz** | **10401,515 Hz** |
| **6182,322 Hz** | **7877,334 Hz** | **10872,693 Hz** |
| **6292,379 Hz** | **8013,314 Hz** | **11220,222 Hz** |
| **6324,493 Hz** | **6374,942 Hz** | **11283,378 Hz** |
| **6341,248 Hz** | **8384,228 Hz** | **12256,432 Hz** |
| **6471,322 Hz** | **8408,121 Hz** | **13749,858 Hz** |
| **6477,218 Hz** | **8534,111 Hz** | **15231,548 Hz** |
| **6558,342 Hz** | **8568,033 Hz** | **15248,324 Hz** |
| **6855,286 Hz** | **8573,122 Hz** | **58191,928 Hz** |
| **7129.843 Hz** | **9226,222 Hz** | **60317,352 Hz** |
| **7140,187 Hz** | **9351,9 Hz** | |
| **7162,422 Hz** | **9737,211 Hz** | |
9. Fréquences AM utilisées pour le traitement du cancer du pancréas
| | | |
|---|---|---|
| **331,3 Hz** | **2315,921 Hz** | **3564,419 Hz** |
| **331,365 Hz** | **2320,315 Hz** | **3619,412 Hz** |
| **436,3 Hz** | **2334,178 Hz** | **3622,312 Hz** |
| **436,332 Hz** | **2381,443 Hz** | **3638,432 Hz** |
| **447.942 Hz** | **2469 Hz** | **36196,424 Hz** |
| **476,127 Hz** | **2477,919 Hz** | **3943,214 Hz** |
| **559,292 Hz** | **2542,221 Hz** | **3976,929 Hz** |
| **589,187 Hz** | **2598,853 Hz** | **4014,889 Hz** |
| **624,218 Hz** | **2647,938 Hz** | **4041,219 Hz** |
| **727 Hz** | **2685,081 Hz** | **4044,195 Hz** |
| **734.921 Hz** | **2716,095 Hz** | **4056,384 Hz** |
| **809,313 Hz** | **2721,331 Hz** | **4085,971 Hz** |
| **845,309 Hz** | **2732,231 Hz** | **4144,592 Hz** |
| **870,4 Hz** | **2809,849 Hz** | **4153,192 Hz** |
| **963.221 Hz** | **2823,428 Hz** | **4161,889 Hz** |
| **1156,79 Hz** | **2835,332 Hz** | **4243,393 Hz** |
| **1157 Hz** | **3134,313 Hz** | **4332,498 Hz** |
| **1179 Hz** | **3241,461 Hz** | **4341,423 Hz** |
| **1360,133 Hz** | **3255,219 Hz** | **4355,327 Hz** |
| **1372,207 Hz** | **3283,432 Hz** | **4417,885 Hz** |
| **1372,934 Hz** | **3286,255 Hz** | **4422,322 Hz** |
| **1804,126 Hz** | **3330,935 Hz** | **4451,297 Hz** |
| **1816,221 Hz** | **3373,892 Hz** | **4486,384 Hz** |
| **1873,477 Hz** | **3438,109 Hz** | **4558,306 Hz** |
| **1967,211 Hz** | **3449,219 Hz** | **4580 Hz** |
| **1990,482 Hz** | **3535,219 Hz** | **4685,082 Hz** |
| **2278,312 Hz** | **3549,215 Hz** | **4839,589 Hz** |
| **5151,402 Hz** | **7320,494 Hz** | **9942,321 Hz** |
| **5209,911 Hz** | **7366,412 Hz** | **10279,122 Hz** |
| **5262,282 Hz** | **7534,221 Hz** | **10386,49 Hz** |
| **5271,312 Hz** | **7548,713 Hz** | **10438,496 Hz** |
| **5387,73 Hz** | **7567,127 Hz** | **10518,311 Hz** |
| **5494,928 Hz** | **7620,851 Hz** | **10528,239 Hz** |
| **5521,221 Hz** | **7663,209 Hz** | **10582,095 Hz** |
| **5573,209 Hz** | **7725,203 Hz** | **10926,111 Hz** |
| **5609,362 Hz** | **7852,233 Hz** | **10948,411 Hz** |
| **5929,616 Hz** | **7920,879 Hz** | **10955,558 Hz** |
| **5948,897 Hz** | **7985,122 Hz** | **11538,193 Hz** |
| **5966,112 Hz** | **8008,323 Hz** | **11904,741 Hz** |
| **5976,825 Hz** | **8013,312 Hz** | **12255,229 Hz** |
| **6064,197 Hz** | **8045,484 Hz** | **12613,341 Hz** |
| **6086,256 Hz** | **8242,332 Hz** | **12819,942 Hz** |
| **6157,253 Hz** | **8351,622 Hz** | **13674,482 Hz** |
| **6215,298 Hz** | **8408,121 Hz** | **13731,322 Hz** |
| **6333,917 Hz** | **8455,894 Hz** | **14525,312 Hz** |
| **6365,242 Hz** | **8551,231 Hz** | **14537,218 Hz** |
| **6558,342 Hz** | **8743,321 Hz** | **14549,331 Hz** |
| **6568,278 Hz** | **8789,631 Hz** | **14845,453 Hz** |
| **6823,194 Hz** | **8868,809 Hz** | **14944,989 MHz** |
| **6853,391 Hz** | **9012,241 Hz** | **15246,315 Hz** |
| **6855,286 Hz** | **9028,994 Hz** | **18668,239 Hz** |
| **7213,204 Hz** | **9131,232 Hz** | **19321,231 Hz** |
| **7228,526 Hz** | **9658,296 Hz** | **19347,208 Hz** |
| **7238,232 Hz** | **9663,495 Hz** | **30182,932 Hz** |
| **7277,921 Hz** | **9680,737 Hz** | |
| **7280,422 Hz** | **9824,442 Hz** | |
10. Fréquences AM utilisées pour le traitement du cancer du poumon
| | | |
|---|---|---|
| **304,148 Hz** | **2761,312 Hz** | **3461,322 Hz** |
| **694,7 Hz** | **2784,491 Hz** | **3682,489 Hz** |
| **694,727 Hz** | **2812,443 Hz** | **3727,231 Hz** |
| **708,8 Hz** | **2855,218 Hz** | **3749,882 Hz** |
| **78,841 Hz** | **2859,495 Hz** | **3769,942 Hz** |
| **1587,811 Hz** | **3128,822 Hz** | **4131,235 Hz** |
| **1759,318 Hz** | **3139,297 Hz** | **4158,393 Hz** |
| **1873,477 Hz** | **3193,212 Hz** | **4243,393 Hz** |
| **2263,704 Hz** | **3348,783 Hz** | **4347,733 Hz** |
| **2391,312 Hz** | **3360,971 Hz** | **4373,411 Hz** |
| **2454,232 Hz** | **3366,311 Hz** | **4378,321 Hz** |
| **2729,929 Hz** | **3373,892 Hz** | **4416,221 Hz** |
| **2741,261 Hz** | **3440,212 Hz** | **4481,242 Hz** |
| **4777,521 Hz** | **6838,434 Hz** | **9181,434 Hz** |
| **4798,422 Hz** | **6870,955 Hz** | **9317,913 Hz** |
| **4837,241 Hz** | **6879,216 Hz** | **9363,896 Hz** |
| **4959,842 Hz** | **7079,411 Hz** | **9736,919 Hz** |
| **5013,321 Hz** | **7216,288 Hz** | **9753,321 Hz** |
| **5047,523 Hz** | **7376,089 Hz** | **10424,908 Hz** |
| **5068,322 Hz** | **7761,289 Hz** | **10452,913 Hz** |
| **6371,922 Hz** | **8082,173 Hz** | **10824,609 Hz** |
| **5538,432 Hz** | **8281,259 Hz** | **11656,329 Hz** |
| **5548,879 Hz** | **8352,189 Hz** | **12748,919 Hz** |
| **5679,309 Hz** | **8442,473 Hz** | **15774,291 Hz** |
| **5734,143 Hz** | **8773.916 Hz** | **15798,333 Hz** |
| **5787,342 Hz** | **8935,752 Hz** | **16510,321 Hz** |
| **6445,309 Hz** | **9121,223 Hz** | |
11. Fréquences AM utilisées pour le traitement du léiomyosarcome
| | | |
|---|---|---|
| **836,923 Hz** | **4241,321 Hz** | **6651.276 Hz** |
| **843,181 Hz** | **4266,591 Hz** | **7168,892 Hz** |
| **1411,241 Hz** | **4337,322 Hz** | **7406,309 Hz** |
| **2073,721 Hz** | **4424.112 Hz** | **7452,528 Hz** |
| **2381,443 Hz** | **4436.111 Hz** | **7649,209 Hz** |
| **2711,019 Hz** | **4485,22 Hz** | **7808,352 Hz** |
| **2911,329 Hz** | **5545,521 Hz** | **9040,313 Hz** |
| **3232,185 Hz** | **5577,841 Hz** | **9074.294 Hz** |
| **3518,321 Hz** | **5631,422 Hz** | **9189,092 Hz** |
| **3544.209 Hz** | **5696,184 Hz** | **9484,512 Hz** |
| **3069,219 Hz** | **6472,098 Hz** | **9943,972 Hz** |
| **4233.822 Hz** | **6558,342 Hz** | **12086,394 Hz** |
12. Fréquences AM utilisées pour le traitement du mésothéliome
| | | |
|---|---|---|
| **958,929 Hz** | **3319,945 Hz** | **6516,793 Hz** |
| **1713,913 Hz** | **3449,219 Hz** | **7224,197 Hz** |
| **1736,782 Hz** | **3622,312 Hz** | **9471,152 Hz** |
| **2334,178 Hz** | **5151,402 Hz** | **14617,393 Hz** |
| **2607,193 Hz** | **5887,022 Hz** | |
| **3112,974 MHz** | **5965,922 Hz** | |
13. Fréquences AM utilisées pour le traitement des tumeurs neuro-endocrines
| | | |
|---|---|---|
| **1766,335 Hz** | **3440,212 Hz** | **6291,631 Hz** |
| **2408,225 Hz** | **3533,328 Hz** | **6406,891 Hz** |
| **2441,502 Hz** | **3600,283 Hz** | **6780,679 Hz** |
| **2647,938 Hz** | **4079.282 Hz** | **7151,264 Hz** |
| **2741,261 Hz** | **4243,393 Hz** | **7482,245 Hz** |
| **3020,212 Hz** | **4426,387 Hz** | **1575,393 Hz** |
| **3128,822 Hz** | **5245,818 Hz** | **8359,932 Hz** |
| **3238,742 Hz** | **5536,242 Hz** | **9073,418 Hz** |
| **3296,431 Hz** | **5548,879 Hz** | |
| **3348,783 Hz** | **5739,422 Hz** | |
| **3360,971 Hz** | **5849,241 Hz** | |
14. Fréquences AM utilisées pour le traitement de la leucémie et du cancer lymphoïde chronique
| | | |
|---|---|---|
| **814,413 Hz** | **3361,671 Hz** | **7829,318 Hz** |
| **825,145 Hz** | **5245,452 Hz** | **8172,405 Hz** |
| **2415,243 Hz** | **5557,333 Hz** | **8272,338 Hz** |
| **2436,316 Hz** | **6850,197 Hz** | **8438,453 Hz** |
| **2874,432 Hz** | **6919,322 Hz** | **12950,331 Hz** |
| **2891,029 Hz** | **7587,224 Hz** | |
15. Fréquences AM utilisées pour le traitement du myélome multiple
| | | |
|---|---|---|
| **765,196 Hz** | **2883,618 Hz** | **5249,331 Hz** |
| **2336,238 Hz** | **2919,273 Hz** | **7967,311 Hz** |
| **2372,122 Hz** | **3265,323 Hz** | **7973,125 Hz** |
| **2381,443 Hz** | **3564,455 Hz** | **8049,952 Hz** |
| **2425,394 Hz** | **3580,25 Hz** | **8283,329 Hz** |
| **2656,339 Hz** | **3584.291 Hz** | **10351,323 Hz** |
| **2741,261 Hz** | **3674,292 Hz** | |
16. Fréquences AM utilisées pour le traitement de la maladie de Hodgkin
| | | |
|---|---|---|
| **752,5 Hz** | **3371,216 Hz** | **5724,231 Hz** |
| **976,3 Hz** | **3605,432 Hz** | **6358,194 Hz** |
| **1558,223 Hz** | **3623,193 Hz** | **7472,211 Hz** |
| **2310,912 Hz** | **3838,281 Hz** | **8062,121 Hz** |
| **2477,919 Hz** | **3838,48 Hz** | **8222,222 Hz** |
| **2560,843 Hz** | **5102 Hz** | |
| **3348,783 Hz** | **5698,932 Hz** | |
17. Fréquences AM utilisées pour le traitement du cancer du cerveau
| | | |
|---|---|---|
| **1372,934 Hz** | **4318,222 Hz** | **6943,388 Hz** |
| **2318,182 Hz** | **4334,33 Hz** | **7151,264 Hz** |
| **2381,443 Hz** | **4358,333 Hz** | **7182,922 Hz** |
| **2425,394 Hz** | **4393,419 Hz** | **7194,897 Hz** |
| **2442,423 Hz** | **4454,194 Hz** | **7323,209 Hz** |
| **2478,973 Hz** | **4515,789 Hz** | **7390,343 Hz** |
| **2654,513 Hz** | **4619,324 Hz** | **7796,221 Hz** |
| **2661,324 Hz** | **4723,937 Hz** | **7961,122 Hz** |
| **2686,105 Hz** | **4853,286 Hz** | **8128,942 Hz** |
| **2690,179 Hz** | **5289,231 Hz** | **8245,109 Hz** |
| **3249,332 Hz** | **5378,099 Hz** | **8272,281 Hz** |
| **3277,509 Hz** | **5426,323 Hz** | **8358,154 Hz** |
| **3335,278 Hz** | **5640,981 Hz** | **8408,121 Hz** |
| **3348,783 Hz** | **6316,211 Hz** | **9138,82 Hz** |
| **3436,211 Hz** | **6459,203 Hz** | **10719,318 Hz** |
| **3916,321 Hz** | **6474,332 Hz** | **11556,241 Hz** |
| **4031,933 Hz** | **6626,572 Hz** | **12828,633 Hz** |
| **4086,091 Hz** | **6855,286 Hz** | **14515,962 Hz** |
| **4241,321 Hz** | **6915,886 Hz** | **14586,765 Hz** |

2. Système (11) selon la revendication 1, dans lequel la fréquence de l'une ou des plusieurs modulations d'amplitude générées est contrôlable avec une précision maximum de 100 ppm par rapport à l'une ou aux plusieurs fréquences de modulation d'amplitude de référence déterminées ou prédéterminées.

3. Système (11) selon la revendication 2, dans lequel la fréquence de l'une ou des plusieurs modulations d'amplitude générées est contrôlable avec une précision maximum de 10 ppm par rapport à l'une ou aux plusieurs fréquences de modulation d'amplitude de référence déterminées ou prédéterminées.

4. Système (11) selon la revendication 3, dans lequel la fréquence de l'une ou des plusieurs modulations d'amplitude générées est contrôlable avec une précision maximum d'environ 1 ppm par rapport à l'une ou aux plusieurs fréquences de modulation d'amplitude de référence déterminées ou prédéterminées.

5. Système (11) selon l'une quelconque des revendications précédentes, dans lequel les émissions électromagnétiques à basse énergie modulées en amplitude générées sont maintenues à une stabilité, pendant l'émission, d'au moins 10⁻⁵.

6. Système (11) selon la revendication 5, dans lequel une stabilité d'au moins 10⁻⁶ est maintenue.

7. Système selon la revendication 6, dans lequel une stabilité d'au moins 10⁻⁷ est maintenue.

8. Système (11) selon l'une quelconque des revendications précédentes, dans lequel l'un ou les plusieurs circuits générateurs contrôlables (29) sont contrôlables par des signaux de contrôle de modulation d'amplitude qui mènent à diverses formes de formes d'onde de modulation d'amplitude étant générées.

9. Système (11) selon la revendication 8, dans lequel les formes d'onde de modulation d'amplitude sont sélectionnées parmi les formes sinusoïdales, carrées, triangulaires ou des combinaisons multiples de celles-ci.

10. Système (11) selon la revendication 8 ou la revendication 9, dans lequel l'un ou les plusieurs circuits générateurs (29) sont contrôlables par des signaux de contrôle de modulation d'amplitude qui génèrent une pluralité de formes d'onde de modulation d'amplitude, soit séquentiellement soit simultanément.

11. Système (11) selon l'une quelconque des revendications précédentes, dans lequel les signaux porteurs à haute fréquence devant être générés par l'un ou les plusieurs circuits générateurs (29) sont sélectionnés parmi une ou plusieurs hautes fréquences sélectionnées parmi environ 27 MHz, 433 MHz et 900 MHz.

12. Système (11) selon l'une quelconque des revendications précédentes, dans lequel le système (11) comprend une ou plusieurs interfaces ou moyens récepteurs (16) communiquant avec l'un ou les plusieurs processeurs de données ou circuits intégrés (21), et dans lequel les informations de contrôle sont transférables vers l'une ou les plusieurs interfaces ou moyens récepteurs (16) et dès lors à l'un ou les plusieurs processeurs de données ou circuits intégrés (21) pour permettre à des signaux de consigne réagissant à des informations de contrôle reçues d'être communiqués à l'un ou les plusieurs circuits générateurs (29) par l'un ou les plusieurs processeurs de données ou circuits intégrés (21).

13. Système (11) selon la revendication 12, dans lequel les informations de contrôle sont transférables ou transmissibles via le téléphone, l'internet, la radio ou d'autres moyens, à l'un ou aux plusieurs processeurs de données ou circuits intégrés (21) via l'une ou les plusieurs interfaces ou moyens récepteurs (16) communiquant avec l'un ou les plusieurs processeurs de données ou circuits intégrés (21).

14. Système (11) selon la revendication 12 ou la revendication 13, dans lequel les informations de contrôle sont stockées dans un dispositif de stockage d'informations (52) et dans lequel les informations de contrôle sont transférables à l'un ou aux plusieurs processeurs de données ou circuits intégrés (21) via l'une ou les plusieurs interfaces (16) communiquant avec l'un ou les plusieurs processeurs de données ou circuits intégrés (21).

15. Système (11) selon l'une quelconque des revendications précédentes, dans lequel le système comprend un dispositif d'identification d'utilisateur (21a) communiquant avec au moins un de l'un ou des plusieurs processeurs de données ou circuits intégrés (21), permettant au système (11) d'être activé ou utilisé seulement par l'utilisateur.

16. Système (11) selon l'une quelconque des revendications précédentes, lequel inclut un moniteur (54) comprenant un logiciel de monitorage pour monitorer l'amplitude et la fréquence de modulation d'amplitude des émissions électromagnétiques à basse énergie modulées en amplitude générées par l'un ou les plusieurs circuits générateurs (29).

17. Système (11) selon l'une quelconque des revendications précédentes, dans lequel les informations de contrôle de fréquence de modulation d'amplitude déterminées ou prédéterminées sont déterminées ou prédéterminées au moyen d'un processus de rétroaction biologique impliquant des observations ou des mesures de réactions physiologiques par le sujet lorsque ou pendant le temps où des fonctions cellulaires du sujet sont excitées par exposition du sujet à des émissions de signaux porteurs à haute fréquence modulées en amplitude à une série de fréquences de modulation d'amplitude.

18. Système (11) selon la revendication 17, dans lequel les fréquences déterminées ou prédéterminées sont utilisées comme manière d'identifier la nature d'une tumeur ou d'un cancer présent chez un sujet mammifère à sang chaud.
